# EUROPEAN PATENT SPECIFICATION

(11) **EP 2 049 514 B1**
(45) Date of publication and mention of the grant of the patent: **26.01.2011**
(21) Application number: 07788301.5
(22) Date of filing: 07.08.2007
(51) Int. Cl.: C07D 401/04, C07D 405/04, C07D 405/12, C07D 413/04, A61K 31/445, A61P 9/00

(54) **NITRATE ESTERS OF PIPERIDINES**
NITRATESTER VON PIPERIDINEN
ESTERS DE NITRATE DE PIPÉRIDINES

(30) Priority: 08.08.2006 CH 12792006
(43) Date of publication of application: 22.04.2009
(73) Proprietor: Novartis AG, 4056 Basel (CH)
(72) Inventor: HEROLD, Peter, 4142 Münchenstein (CH); MAH, Robert, 4132 Muttenz (CH); STUTZ, Stefan, 4053 Basel (CH); TSCHINKE, Vincenzo, 4102 Binningen (CH); LYOTHIER, Isabelle, 68330 Huningue (FR); SCHUMACHER, Christoph, 4126 Bettingen (CH); MARTI, Christiane, 4310 Rheinfelden (CH); JOTTERAND, Nathalie, 4053 Basel (CH)
(74) Representative: Warner, James Alexander
(86) International application number: PCT/EP2007/058207
(87) International publication number: WO 2008/017685

(56) References cited:
- WO-A-00/64873
- WO-A-2004/089903
- WO-A-2005/051911
- WO-A-2006/005741
- WO-A-2006/103273
- WO-A-2006/103275
- WO-A-2006/103277
- WO-A-2007/082907

## Description

### FIELD OF THE INVENTION

The present invention relates to novel nitrate ester derivatives of substituted piperidines, to methods for their preparation and to their use in cardiovascular diseases, in particular in high blood pressure and vascular and organ damage accompanying high blood pressure.

### BACKGROUND OF THE INVENTION

Cardiovascular diseases and further organ damage are the most frequent consequences of untreated or inadequately treated high blood pressure. Thus, high blood pressure in the long term leads to overloading of the heart and vessels and, accompanying this, an increased risk of developing cardiovascular disease and organ damage. Typical consequences are arterial occlusive diseases, cardiac infarction, stroke and kidney damage. In the presence of diabetes or risk factors such as smoking and overweight, the risk of suffering one of these high blood pressure sequelae grows.

In many clinical morbidity and mortality studies, the benefit of an intensive medicinal blood pressure treatment was shown. These studies also showed that therapeutic classes over and beyond lowering of blood pressure can differ in their tissue and organ protection. These distinctions were explained by pleiotropic therapeutic effects. Inhibitors of the renin-angiotensin system, such as angiotensin-converting enzyme (ACE) inhibitors or angiotensin receptor blockers (ARB), were, for example, ascribed anti-inflammatory, anti-proliferative and antithrombotic properties. These properties could be particularly well shown with renin inhibitors and were described, for example, in WO 2006/005741.

### OBJECTS OF THE INVENTION

The derivatization of renin inhibitors based on piperidine to the corresponding nitrate esters led to compounds having unexpectedly strong blood pressure-lowering and tissue-protecting properties, which go beyond the inhibition of renin.

One subject of the invention is therefore substituted piperidines of the general formula 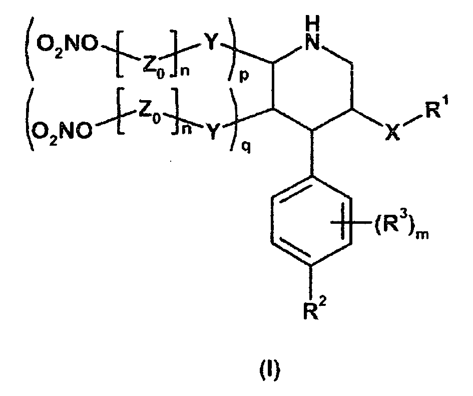 where

R¹ is aryl or heterocyclyl, in particular benzoimidazolyl, benzo[1,3]dioxolyl, benzofuranyl, benzoxazolyl, benzothiazolyl, benzo[b]thienyl, quinazolinyl, quinolyl, quinoxalinyl, 2H-chromenyl, 3,4-dihydro-2H-benzo[1,4]oxazinyl, dihydro-3H-benzo-[1,4]oxazinyl, dihydro-2H-benzo[1,4]thiazinyl, 1a,7b-dihydro-1H-cyclopropa[c]chromenyl, 2,3-dihydro-1H-indolyl, dihydro-1H-pyrido[2,3-b][1,4]oxazinyl, imidazo[1,2-a]pyridyl, imidazo[1,5-a]pyridyl, indazolyl, indolyl, isobenzofuranyl, isoquinolyl, [1,5]naphthyridyl, phenyl, phthalazinyl, pyridyl, pyrimidinyl, 1H-pyrrolo[2,3-b]pyridyl, 1H-pyrrolo[2,3-c]pyridyl, 1H-pyrrolo[3,2-b]pyridyl, tetrahydro-quinolyl, tetrahydroquinoxalinyl, 1,1a,2,7b-tetrahydro-cyclopropa[c]chromenyl, tetrahydroimidazo[1,2-a]-pyridyl, tetrahydroimidazo[1,5-a]pyridyl, tetrahydroisoquinolyl, [1,2,3]triazolo[1,5-a]pyridyl or [1,2,4]triazolo [4,3-a]pyridyl, which are substituted by 1-4acyl-C₁₋₈-alkoxy-C₁₋₈-alkoxy, acyl-C₁₋₈-alkoxy-C₁₋₈-alkyl, (N-acyl)-C₁₋₈-alkoxy-C₁₋₈-alkylamino, C₁₋₈-alkanoyl, C₁₋₈-alkoxy, C₁₋₈-alkoxy-C₁₋₈-alkanoyl, C₁₋₈-alkoxy-C₁₋₈-alkoxy, C₁₋₈-al koxy-C₁₋₈-alkoxy-C₁₋₈-alkyl, C₁₋₈-alkoxy-C₁₋₈-alkyl, (N-C₁₋₈-alkoxy)-C₁₋₈-alkylamino-carbonyl-C₁₋₈-alkoxy, (N-C₁₋₈-alkoxy)-C₁₋₈-alkylaminocarbonyl-C₁₋₈-alkyl, C₁₋₈-alkoxy-C₁₋₈-alkylcarbamoyl, C₁₋₈-alkoxy-C₁₋₈-alkylcarbonyl, C₁₋₈-alkoxy-C₁₋₈-alkyl-carbonylamino, 1-C₁₋₈-alkoxy-C₁₋₈-alkylheterocydyl, C₁₋₈-alkoxyaminocarbonyl-C₁₋₈-alkoxy, C₁₋₈-alkoxyamino-carbonyl-C₁₋₈-alkyl, C-₁₋₈-alkoxycarbonyl, C₁₋₈-alkoxy-carbonyl-C₁₋₈-alkoxy, C₁₋₈-alkoxycarbonyl-C₁₋₈-alkyl, C₁₋₈-alkoxycarbonylamino-C-₁₋₈-alkoxy, C₁₋₈-alkoxycarbonyl-amino-C₁₋₈-alkyl, C₁₋₈-alkyl, (N-C₁₋₈-alkyl)-C₁₋₈-alkoxy-C₁₋₈-alkylcarbamoyl, (N-C₁₋₈-alkyl)-C₁₋₈-alkoxy-C₁₋₈-alkylcarbonylamino, (N-C₁₋₈-alkyl)-C₁₋₈-alkoxycarbonylamino, (N-C₁₋₈-alkyl)-C₁₋₈-alkylcarbonylamino-C₁₋₈-alkoxy, (N-C₁₋₈-alkyl-C₁₋₈-alkylcarbonylamino-C₁₋₈-alkyl, (N-C₁₋ₐ-alkyl)-C₁₋₈-alkylsulphonylamino-C₁₋₈-alkoxy, (N-C₁₋₈-alkyl)-C₁₋₈-alkylsulfonylamino-C₁₋₈-alkyl, C₁₋₈-alkylamidinyl, C₁₋₈-alkylamino-C₁₋₈-alkoxy, di-C₁₋₈-alkylamino-C₁₋₈-alkoxy, C₁₋₈-alkylamino-C₁₋₈-alkyl, di-C₁₋₈-alkylamino-C₁₋₈-alkyl, C₁₋₈-alkylaminocarbonyl-C₁₋₈-alkoxy, di-C₁₋₈-alkylamino-carbonyl-C₁₋₈-alkoxy, C₁₋₈-alkylaminocarbonyl-C₁₋₈-alkoxy-C₁₋₈-alkyl, C₁₋₈-alkylaminocarbonyl-C₁₋₈-alkyl, di-C₁₋₈-alkylaminocarbonyl-C₁₋₈-alkyl, C₁₋₈-alkylamino-carbonylamino-C₁₋₈-alkoxy, C₁₋₈-alkylaminocarbonylamino-C₁₋₈-alkyl, C₁₋₈-alkylcarbonylamino, C₁₋₈-alkylcarbonyl-amino-C₁₋₈-alkoxy, C₁₋₈-alkylcarbonylamino-C₁₋₈-alkyl, C₁₋₈-alkylcarbonyloxy-C₁₋₈-alkoxy, C₁₋₈-alkylcarbonyloxy-C₁₋₈-alkyl, C₁₋₈-alkylsulphonyl, C₁₋₈-alkylsulphonyl-C₁₋₈-alkoxy, C₁₋₈-alkylsulphonyl-C₁₋₈-alkyl, C₁₋₈-alkyl-sulphonylamino-C₁₋₈-alkoxy, C₁₋₈-alkylsulphonylamino-C₁₋₈-alkyl, optionally N-mono- or N,N-di-C₁₋₈-alkylated amino, aryl-C₀₋₈-alkoxy, aryl-C₀₋₈-alkyl, optionally N-mono- or N,N-di-C₁₋₈-alkylated carbamoyl-C₀₋₈-alkoxy, optionally N-mono- or N,N-di-C₁₋₈-alkylated carbamoyl-C₀₋₈-alkyl, carboxy-C₁₋₈-alkoxy, carboxy-C₁₋₈-alkoxy-C₁₋₈-alkyl, carboxy-C₁₋₈-alkyl, cyano, cyano-C₁₋₈-alkoxy, cyano-C₁₋₈-alkyl, C₃₋₈-cycloalkyl-C₁₋₈-alkoxy, C₃₋₈-cycloalkyl-C₁₋₈-alkyl, C₃₋₈-cycloalkylcarbonylamino-C₁₋₈-alkoxy, C₃₋₈-cycloalkylcarbonylamino-C₁₋₈-alkyl, O,N-dimethylhydroxyamino-C₁₋₈-alkyl, halogen, halogeno-C₁₋₈-alkoxy, halogeno-C₁₋₈-alkyl, heterocydyl-C₀₋₈-alkoxy, heterocydyl-C₀₋₈-alkyl, heterocyclylcarbonyl, hydroxy-C₁₋₈-alkoxy-C₁₋₈-alkoxy, hydroxy-C₁₋₈-alkoxy-C₁₋₈-alkyl, hydroxy-C₁₋₈-alkyl, O-methyloximyl-C₁₋₈-alkyl, oxide or oxo;

R² is C₂₋₈-alkenyloxy-C₁₋₈-alkoxy, C₂₋₈-alkenyloxy-C₁₋₈-alkyl, C₁₋₈-alkoxy, C₁₋₈-alkoxy-C₁₋₈-alkoxy, optionally halogen-substituted C₁₋₈-alkoxy-C₁₋₈-alkoxy-C₁₋₈-alkyl, C₁₋₈-alkoxy-C₁₋₈-alkyl, C₁₋₈-alkoxy-C₁₋₈-alkylamino-C₁₋₈-alkoxy, C₁₋₈-alkoxy-C₁₋₈-alkylamino-C₁₋₈-alkyl, C₁₋₈-alkoxy-C₁₋₈-alkysulphanyl-C₁₋₈-alkoxy, C₁₋₈-alkoxy-C₁₋₈-alkylsulphanyl-C₁₋₈-alkyl, C₁₋₈-alkoxy-C₀₋₈-alkyl-C₃₋₈-Cycloalkyl-C₀₋₈-alkoxy-C₁₋₈-alkyl, C₁₋₈-alkyl, C₁₋₈-alkyl-sulphanyl-C₁₋₈-alkoxy, C₁₋₈-alkylsulphanyl-C₁₋₈-alkoxy-C₁₋₈-alkoxy, C₁₋₈-alkylsulphanyl-C₁₋₈-alkoxy-C₁₋₈-alkyl, C₁₋₈-alkylsulphanyl-C₁₋₈-alkyl, C₁₋₈-alkylsulphonyl-C₁₋₈-alkoxy- C₁₋₈-alkoxy, C₁₋₈-alkylsulphonyl-C₁₋₈-alkoxy-C₁₋₈-alkyl, aryl-C₀₋₈-alkoxy-C₁₋₈-alkoxy, aryl-C₀₋₈-alkoxy-C₁₋₈-alkyl, aryloxy, C₃₋₈-cycloalkyl-C₀₋₈-alkoxy-C₁₋₈-alkoxy, C₃₋₈-cycloalkyl-C₀₋₈-alkoxy-C₁₋₈-alkoxy-C₁₋₈-alkyl, C₃₋₈-cyclo-alkyl-C₀₋₈-alkoxy-C₁₋₈-alkyl, heterocyclyl-C₀₋₈-alkoxy-C₁₋₈-alkoxy, heterocydyl-C₀₋₈-alkoxy-C₁₋₈-alkyl or hetero-cyclyloxy;

R³ is halogen;
R⁴ is acyl, C₂₋₈-alkenyl, C₁₋₈-alkyl, aryl-C₁₋₈-alkyl or hydrogen;
X is -Alk-, -O-Alk-, -Alk-O-, -O-Alk-O-, -S-Alk-, -Alk-S-, -Alk-NR⁴-, -NR⁴-Alk-, -C(O)-NR⁴-, -Alk-C(O)-NR⁴-, -O-Alk-C(O)-NR⁴-, -C(O)-NR⁴-Alk-, -Alk-C(O)-NR⁴-Alk-, -NR⁴-C(O)-, -Alk-NR⁴-C(O)-, -NR⁴-C(O)-Alk-, -Alk-NR⁴-C(O)-Alk-, -O-Alk-C(O)-NR⁴-, -O-Alk-NR⁴-C(O)-, -S(O)₂-NR⁴-, -Alk-S(O)₂-NR⁴-, -S(O)₂-NR⁴-Alk-, -Alk-S(O)₂-NR⁴-Alk-, -NR⁴-S(O)₂-, -Alk-NR⁴-S(O)₂-, -NR⁴-S(O)₂-Alk- or -Alk-NR⁴-S(O)₂-Alk-, where Alk designates C₁₋₈-alkylene which can optionally be substituted by halogen;
Y is a) unsubstituted or heterocyclyl- and/or halogen- and/or hydroxy- and/or C₁₋₈-alkoxy-substituted
- Alk-,
- Alk-O-Alk-,
- Alk-NR⁴-Alk-,
- Alk-C(O)-Alk-,
- Alk-C(O)-NR⁴-Alk-,
- Alk-C(O)-Alk-NR⁴-Alk-,
- Alk-NR⁴-C(O)-Alk- or
- Alk-NR⁴-Alk-C(O)-Alk-,
where Alk designates linear or branched C₁₋₈-alkylene; or
b) if p = 0, is additionally
i) a bond
ii) unsubstituted or heterocyclyl- and/or halogen- and/or hydroxy- and/or C₁₋₈-al koxy-substituted
   - Alk-O-,
   - Alk-NR⁴-,
   - Alk-C(O)- or
      -Alk-NR⁴-C(O)-Alk-O-,
   where Alk designates linear or branched C₁₋₈-alkylene;
Z₀ is equal to -U-Z₁-
wherein Z₁ is -C(O)-O-, -O-C(O)O-, -C(O)-NR⁷-C(O)- or -O-C(O)-NR⁷-C(O)-, wherein R⁷ is as defined below;
U is a bivalent radical with the following meaning:
a)
   - C₁₋₈-alkylene, preferably C₁₋₈-alkylene, being optionally substituted with one or more of the substituents selected from the group consisting of: halogen, hydroxy, - ONO₂ or Tₒ, wherein Tₒ is -OC(O)-(C₁₋₈-alkyl)-ONO₂ or-O-(C₁₋₈-alkyl)-ONO₂;
   - C₃₋₈-cycloalkylene, the ring being optionally substituted with side chains T, wherein T is C₁₋₈-alkyl;
b) 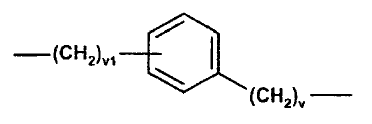 wherein v is an integer from 0 to 20, and v1 is an integer from 1 to 20;
c) 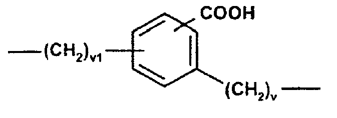 wherein v is an integer from 0 to 20, and v1 is an integer from 1 to 20;
d) 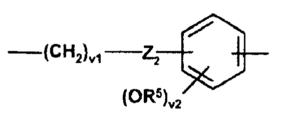 wherein:
   v1 is as defined above and v2 is an integer from 0 to 2;
   Z₂ = -O-C(O)- or -C(O)-O- and R⁵ is H or CH₃;
e) 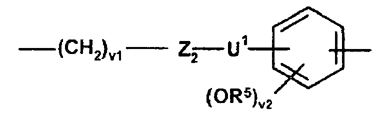 wherein:
   v1, v2, R⁵ and Z₂ are as defined above;
   U¹ is -CH₂-CH₂- or -CH=CH-(CH₂)ᵥ₂-;
f) 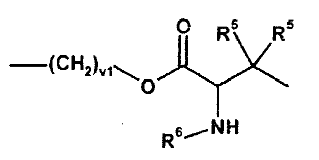 wherein:
   v1 and R⁵ are as defined above, R⁶ is H or -C(O)CH₃;
g) 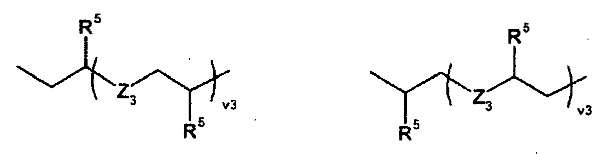 wherein Z₃ is -O- or -S-, v3 is an integer from 1 to 6, preferably from 1 to 4, R⁵ is as defined above; or
h) 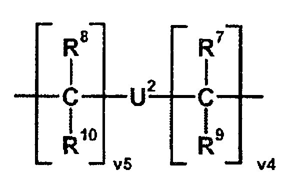 wherein:
   v4 is an integer from 0 to 10;
   v5 is an integer from 1 to 10;
   R⁷, R⁸, R⁹, R¹⁰ are the same or different, and are H or C₁₋₄ alkyl;
   U² is a heterocyclic saturated, unsaturated or aromatic 5 or 6 membered ring, containing one or more heteroatoms selected from nitrogen, oxygen and sulphur, and is preferably selected from 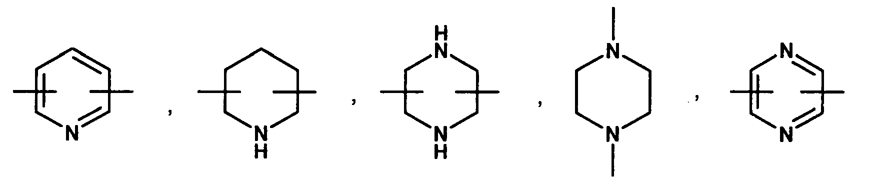 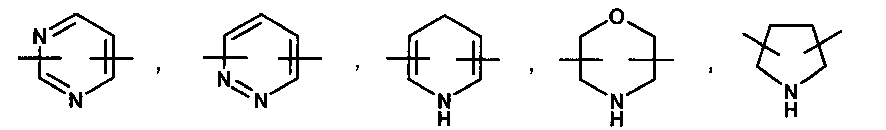 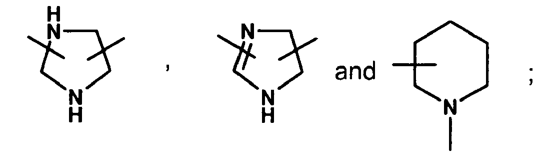
   m is 0, 1 or 2;
   n is 0 or 1;
   p is 0 or 1;
   q is 0 or 1;
   where if p is = 0, q is = 1 and if p is = 1, q is = 0,
   and their salts, preferably their pharmaceutically usable salts.

The linkage of the above (and hereinafter) mentioned substituent -X- within the compound of the formula (I) starts from the piperidine ring with the substituent -X-being arranged from left to right when written as indicated above. For example, the fragment "-X-R¹" of the compound of the formula (I) with X meaning "-NR⁴-S(O)₂-" is: "-NR₄S(O)₂-R¹",

The linkage of the above (and hereinafter) mentioned substituent -Y- within the compound of the formula (I) starts from O₂NO-[Z₀]ₙ- with the substituent -Y- being arranged from left to right when written as indicated above. For example, the fragment "O₂NO-[Z₀]ₙ-Y-" of the compound of the formula (I) with Y meaning "-Alk-NR⁴-C(O)-Alk-" is: "O₂NO-[Z₀]ₙ-Alk-NR⁴-C(O)-Alk-".

In the case n = 1, the linkage of the above (and hereinafter) mentioned substituent - U-Z₁- within the compound of the formula (I) starts from O₂NO- with the substituent - U-Z₁- being arranged from left to right when written as indicated above. For example, the fragment "O₂NO-U-Z₁-" of the compound of the formula (I) with -U-Z₁- with U = "C₁₋₈-alkylene" and Z₁ = "-C(O)-O-" is: "O₂NO-C₁₋₈-alkyl-C(O)-O-".

If not further specified, C₁₋₈-alkyl and alkoxy radicals can be linear or branched. Examples of C₁₋₈-alkyl and alkoxy radicals are methyl, ethyl, n-propyl, isopropyl, n-butyl, isobutyl, sec-butyl, tert-butyl, pentyl, hexyl and methoxy, ethoxy, propoxy, isopropoxy, butoxy, isobutoxy, sec-butoxy and tert-butoxy. C₁₋₈-Alkylene-dioxy radicals are preferably methylenedioxy, ethylenedioxy and propylenedioxy. Examples of C-₁₋₈-alkanoyl radicals are acetyl, propionyl and butyryl. Cycloalkyl is a saturated, cyclic hydrocarbon radical with 3 up to 12 hydrocarbon atoms, for example cyclopropyl, cyclobutyl, cyclopentyl, cyclohexyl, cycloheptyl, bicyclo[2.2.1]heptyl, cyclooctyl, bicyclo[2.2.2]octyl and adamantyl. Cycloalkyl can be unsubstituted or mono- or polysubstituted, e.g. mono- or disubstituted, by C₁₋₈-alkanoyl, C₂₋₈-alkenyl, C₂₋₈-alkynyl, C₁₋₈-alkoxy, C₁₋₈-alkoxy-C₁₋₈-alkoxy, C₁₋₈-alkoxy-C₁₋₈-alkyl, C₁₋₈-alkoxycarbonylamino, C₁₋₈-alkyl, C₀₋₈-alkylcarbonylamino, C₁₋₈-alkylcarbonyloxy, C₁₋₈-alkylene-dioxy, optionally N-mono- or N,N-di-C₁₋₈-alkylated amino, aryl, optionally N-mono- or N,N-di-C₁₋₈-alkylated carbamoyl, optionally esterified carboxyl, cyano, C₃₋₈-cycloalkoxy, halogen, heterocyclyl, hydroxy, oxo, polyhalo-C₁₋₈-alkoxy or polyhalo-C₁₋₈-alkyl. C₁₋₈-Alkylene radicals can be linear or branched and are, for example, methylene, ethylene, 1-methylmethylene, propylene, 1-methylethylene, 1-ethylmethylene, 1,1-dimethylmethylene, 2-methylpropylene, 2-methyl-butylene, 2-methylpropyl-2-ene, butyl-2-ene, butyl-3-ene, propyl-2-ene, tetra-, penta- and hexamethylene;

C₂₋₈-alkenylene radicals are, for example, vinylene and propenylene; C₂₋₈-alkynylene radicals are, for example, ethynylene; acyl radicals are alkanoyl radicals, preferably C₁₋₈-alkanoyl radicals, or aroyl radicals, such as benzoyl.

The meaning of "C₀-alkyl" in the above (and hereinafter) mentioned C₀₋₈-alkyl groups is a bond or, if located at a terminal position, a hydrogen atom.

The meaning of "C₀-alkoxy" in the above (and hereinafter) mentioned C₀₋₈-alkoxy groups is "-O-" or, if located at a terminal position, an -OH group.

Aryl designates mono- or polynuclear aromatic radicals, which can be mono- or polysubstituted, such as, for example, phenyl, substituted phenyl, naphthyl, substituted naphthyl. Examples of substituents on aryl radicals of this type are acetamidinyl-C₁₋₈-alkyl, acyl-C₁₋₈-alkoxy-C₁₋₈-alkyl, (N-acyl)-C-₁₋₈-alkoxy-C₁₋₈-alkyl-amino, C₂₋₈-alkenyl, C₂₋₈-alkenyloxy, C₁₋₈-alkoxy, C₁₋₈-alkoxy-C₁₋₈-alkoxy, C₁₋₈-alkoxy-C₁₋₈-alkoxy-C₁₋₈-alkyl, C₁₋₈-alkoxy-C₁₋₈-alkyl , (N-C₁₋₈-alkoxy)-C₁₋₈-alkylamino-carbonyl-C₁₋₈-alkoxy, (N-C₁₋₈-alkoxy)-C₁₋₈-alkylamino-carbonyl-C₁₋₈-alkyl, C₁₋₈-alkoxy-C₁₋₈-alkylcarbamoyl, C₁₋₈-alkoxy-C₁₋₈-alkylcarbonyl, C₁₋₈-alkoxy-C₁₋₈-alkyl-carbonylamino, C₁₋₈-alkoxy-C₁₋₈-alkylheterocyclyl, 2-C₁₋₈-alkoxy-C₁₋₈-alkyl-4-oxoimidazol-1-yl, 6-alkoxyamino-carbonyl-C₁₋₈-alkoxy, C₁₋₈-alkoxyaminocarbonyl-C₁₋₈-alkyl, C-₁₋₈-alkoxycarbonyl, C₁₋₈-alkoxycarbonyl-C₁₋₈-alkoxy, C₁₋₈-alkoxycarbonyl-C₁₋₈-alkyl, C₁₋₈-alkoxycarbonylamino-C₁₋₈-alkoxy, C₁₋₈-alkoxycarbonylamino-C₁₋₈-alkyl, C₁₋₈-alkoxycarbonylphenyl, C₁₋₈-alkyl, (N-C₁₋₈-alkyl)-C₁₋₈-alkoxy-C₁₋₈-alkylcarbamoyl, (N-C₁₋₈-alkyl)-C₁₋₈-alkoxy- C₁₋₈-alkylcarbonylamino, (N-C₁₋₈-alkyl)-C₁₋₈-alkoxycarbonylamino, (N-C₁₋₈-alkyl)-C₀₋₈-alkylcarbonylamino- C₁₋₈-alkoxy, (N-C₁₋₈-alkyl)-C₀₋₈-alkylcarbonylamino-C₁₋₈-alkyl, (N-C₁₋₈-alkyl)-C₁₋₈-alkysulphonylamino-C₁₋₈-alkoxy, (N-C₁₋₈-al kyl)-C₁₋₈-alkysulphonylamino-C₁₋₈-alkyl, C₁₋₈-alkylamidinyl, optionally N-mono- or N,N-di-C₁₋₈-alkylated amino, C₁₋₈-alkylaminO-C₂₋₈-alkoxy, di-C₁₋₈-alkylamino-C₂₋₈-alkoxy, C₁₋₈-alkylamino-C₁₋₈-alkyl, di- C₁₋₈-alkylamino-C₁₋₈-alkyl, C₁₋₈-alkylaminocarbonyl-C₁₋₈-alkoxy, di-C₁₋₈-alkylaminocarbonyl-C₁₋₈-alkoxy, C₁₋₈-alkylaminocarbonyl-C₁₋₈-alkoxy-C₁₋₈-alkyl, C₁₋₈-alkyl-aminocarbonyl-C₁₋₈-alkyl, di-C₁₋₈-alkylaminocarbonyl-C₁₋₈-alkyl, C₁₋₈-alkylaminocarbonylamino-C₁₋₈-alkoxy, C-₁₋₈-alkylaminocarbonylamino-C₁₋₈-alkyl , C₁₋₈-alkylcarbamoyl, di-C₁₋₈-alkylcarbamoyl, C₀₋₈-alkylcarbonylamino, C₀₋₈-alkylcarbonylamino-C₁₋₈-alkoxy, C₀₋₈-alkylcarbonylamino-C₁₋₈-alkyl , C₁₋₈-alkylcarbonyloxy, C₁₋₈-alkylcarbonyloxy-C₁₋₈-alkoxy, C₁₋₈-alkylcarbonyloxy-C₁₋₈-alkyl, C₁₋₈-alkylenedioxy, C₁₋₈-alkylsulphonyl, C₁₋₈-alkysulphonyl-C₁₋₈-alkoxy, C₁₋₈-alkylsulphonyl-C₁₋₈-alkyl, C₁₋₈-alkyl-sulphonylamino-C₁₋₈-alkoxy, C₁₋₈-alkylsulphonylamino-C₁₋₈-alkyl , aryi-C₁₋₈-alkanoyl, aryl-C₀₋₈-alkoxy, aryl-C₀₋₈-alkyl, arylamino, arylthio, benzoyloxy-C₁₋₈-alkoxy, benzyloxy, carbamoyl, carbamoyl-C₁₋₈-alkoxy, carbamoyl-oxy-C₁₋₈-alkoxy, carbamoyl-C₁₋₈-alkyl, carboxyl, carboxy-C₁₋₈-alkoxy, carboxy-C₁₋₈-alkoxy-C₁₋₈-alkyl, carboxy-C₁₋₈-alkyl, cyano, cyano-C₁₋₈-alkoxy, cyano-C₁₋₈-alkyl, C₃₋₈-cydoalkyl-C₁₋₈-alkanoyl, C₃₋₈-cycloalkylcarbonylamino-C₁₋₈-alkoxy, C₃₋₈-cycloalkylcarbonylamino-C₁₋₈-alkyl, cyclo-propyl-C₁₋₈-alkoxy, cyclopropyl-C₁₋₈-alkyl, O,N-dimethyl-hydroxyamino-C₁₋₈-alkyl, dioxolanyl-C₁₋₈-alkoxy, halogen, halogeno-C₁₋₈-alkoxy, halogeno-C₁₋₈-alkyl, heterocyclyl, heterocyclyl-C₁₋₈-alkanoyl, heterocyclyl-C₁₋₈-alkoxy, heterocyclyl-C₁₋₈-alkoxy-C₁₋₈-alkoxy, hetero-cyclyl-C₁₋₈-alkoxy-C₁₋₈-alkyl, heterocyclyl-C₁₋₈-alkyl, heterocyclylamino, heterocyclyloxy, heterocyclylthio, hydroxy, hydroxy-C₁₋₈-alkoxy, hydroxy-C₁₋₈-alkoxy-C₁₋₈-alkoxy, hydroxy-C₁₋₈-alkoxy-C₁₋₈-alkyl, hydroxy-C₁₋₈-alkyl, (N-hydroxy)-C₁₋₈-alkylaminocarbonyl-C₁₋₈-alkoxy, (N-hydroxy)-C₁₋₈-alkylaminocarbonyl-C₁₋₈-alkyl, hydroxy-C₁₋₈-alkylphenyl, (N-hydroxy)aminocarbonyl-C₁₋₈-alkoxy, (N-hydroxy)aminocarbonyl-C₁₋₈-alkyl, hydroxybenzyloxy, methylenedioxybenzyloxy, methoxybenyloxy, O-methyl-oximyl-C₁₋₈-alkyl , nitro, 2-oxooxazolidinyl-C₁₋₈-alkoxy, 2-oxooxazolidinyl-C₁₋₈-alkyl, phenethyloxy, pyridyl-carbamoyloxy-C₁₋₈-alkoxy or pyridylcarbonylamino-C₁₋₈-alkyl.

The expression heterocyclyl designates mono- or bicyclic, saturated and unsaturated heterocyclic radicals with 1 to 4 nitrogen and/or 1 or 2 sulphur or oxygen atoms, which can be mono- or polysubstituted, in particular by (in the case of unsaturated heterocyclyl radicals) oxide or oxo or by substituents, such as defined above for aryl radicals, or (in the case of saturated heterocyclyl radicals) can be saturated by alkoxy, alkyl or oxo. Heterocyclyl radicals which contain a nitrogen atom can either be bonded to the remaining molecule via the N atom or via a C atom. Preferred heterocyclic radicals each have 1 nitrogen, oxygen or sulphur atom per ring, 1-2 nitrogen atoms and 1-2 oxygen atoms or 1-2 nitrogen atoms and 1-2 sulphur atoms, where at least one, preferably 1-7 carbon atoms, are present.

Examples of heterocyclyl radicals are benzimidazolyl, benzo[1,3]dioxolyl, benzofuranyl, benzooxazolyl, benzo-thiazolyl, benzo[b]thienyl, quinazolinyl, quinolyl, quinoxalinyl, 2H-chromenyl, dihydro-2H-benzo[1,4]-oxazinyl, dihydro-3H-benzo[1,4]oxazinyl, dihydro-2H-benzo[1,4]thiazinyl, 2,3-dihydroindolyl, dihydro-1H-pyrido[2,3-b][1,4]oxazinyl, furyl, imidazolyl, imidazo- [1,2-a]pyridyl, imidazo[1,5-a]pyridyl, indazolyl, indolyl, isobenzofuranyl, isoquinolyl, isoxazolyl, [1,5]naphthyridyl, oxazolyl, phthalazinyl, pyranyl, pyrazinyl, pyrazolyl, pyridyl, pyrimidinyl, 1H-pyrrolizinyl, 1H-pyrrolo[2,3-b]pyridyl, 1H-pyrrolo[2,3-c]pyridyl, 1H-pyrrolo[3,2-b]pyridyl, pyrrolyl, tetra-hydroquinolyl, tetrahydroquinoxalinyl, tetrahydro-imidazo[1,2-a]pyridyl, tetrahydroimidazo[1,5-a]pyridyl, tetrahydroisoquinolyl, tetrazolyl, thiazolyl, thienyl, [1,2,3]triazolo[1,5-a]pyridyl, [1,2,4]triazolo[4,3-a]-pyridyl or triazolyl. Examples of substituted heterocyclyl radicals are 2,2-dimethyl-3-oxo-4H-benzo[1,4)oxazinyl, 2,2-dimethyl-3,4-dihydro-2H-benz[1,4]oxazinyl, 2-aryl-2-methyl-3,4-dihydro-2H-benz[1,4]oxazinyl, 2,2-dimethyl-2H-chromen-6-yl, 2-aryl-2-methyl-2H-chromen-6-yl, 2-oxobenz-imidazolyl, 2-oxodihydrobenzo[d][1,3]oxazinyl, 4-oxo-dihydroimidazolyl, 5-oxo-4H-[1,2,4]triazinyl, 3-oxo-4H-benzo[1,4]thiazinyl, 1,1,3-trioxodihydro-2H-lλ⁶-benzo-[1,4]thiazinyl, 1-oxopyridyl, 2-oxotetrahydrobenzo-[e][1,4]diazepinyl, 2-oxo-dihydrobenzo[e][1,4]diazep-inyl, 1-oxo-3H-isobenzofuranyl, 4-oxo-3H-thieno[2,3-d]pyrimidinyl, 3-oxo-4H-benzo[1,4]oxazinyl, 1,1-dioxo-dihydro-2H-benzo[1,4]thiazinyl, 2-oxo-1H-pyrido[2,3-b]-[1,4]oxazinyl, 2-oxobenzooxazolyl, 2-oxo-1,3-dihydro-indolyl, 2-oxodihydro-1 H-quinazolinyl, nitrobenzo-thiazolyl, phenyltetrazolyl, phenyloxa-diazolyl, phenyl-piperidinyl, phenylpiperazinyl, phenylpyrrolidinyl, thienyloxadiazolyl, furany1oxadiazolyl, benzyloxa-diazolyl or phenyloxazolyl.

Examples of saturated heterocyclyl radicals are azetidinyl, dioxolanyl, dioxanyl, dithiolanyl, dithianyl, pyrrolidinyl, piperidinyl, piperazinyl, 4-methylpiperazinyl, morpholinyl, thiomorpholinyl, 2-hydroxymethylpyrrolidinyl, 3-hydroxypyrrolidinyl, 3,4-dihydroxypyrrolidinyl, 4-hydroxypiperidinyl oxazolidin-yl, 4-oxopiperidinyl, 3,5-dimethylmorpholinyl, 4,4-dioxothiomorpholinyl, 4-oxothiomorpholinyl, 2,6-dimethyl-morpholinyl, tetrahydropyranyl, 2-oxo-imidazolidinyl, 2-oxooxazolidinyl, 2-oxopiperidinyl, 2-oxopyrrolidinyl, 2-oxo[1,3]oxazinyl, 2-oxoazepanyl, 2-oxotetrahydro-pyrimidinyl and the like.

Examples of bi- or polycyclic heterocyclyl radicals are 2,5-dioxabicyclo[4.1.0]heptanyl, 2-oxabicyclo[2.2.1]-heptanyl, 2-oxabicyclo[4.1.0]heptanyl, 3-oxabicyclo- [4.1.0]heptanyl, 7-oxabicydo[2.2.1]heptanyl, 2-oxa-bicyclo[3.1.0]hexanyl, 3-oxabicyclo[3.1.0]hexanyl, 1-oxaspiro[2.5]octanyl, 6-oxaspiro[2.5]octanyl, 3-oxa-bicyclo[3.3.1]nonanyl, 2-oxo-1a,7b-dihydro-1 H-cyclo-propa[c]chromenyl or 1,1 a,2,7b-tetrahydrocyclopropa-[c]chromenyl.

The expression polyhydroxyalkyl designates C₁₋₈-alkyl radicals which can be substituted with 2-8 hydroxy groups, such as, for example, glyceryl, arabityl, sorbityl etc.

The expression halogen or halo designates, for example, fluorine, chlorine or bromine, or a radical which is monosubstituted or polysubstituted by fluorine, chlorine or bromine.

The compounds of the formula (I) have at least three asymmetric carbon atoms and can therefore be present in the form of optically pure diastereomers, diastereomer mixtures, diastereomeric racemates, mixtures of diastereomeric racemates or as meso compounds. The invention comprises all these forms. Diastereomer mixtures, diastereomeric racemates or mixtures of diastereomeric racemates can be separated according to customary methods, e.g. by column chromatography, thin-layer chromatography, HPLC and the like.

Salts of compounds with salt-forming groups are in particular acid addition salts, salts with bases or, in the presence of a number of salt-forming groups, optionally also mixed salts or internal salts.

Salts are primarily the pharmaceutically usable or non-toxic salts of compounds of the formula (I).

Salts of this type are formed, for example, from compounds of the formula (I) with an acidic group, e.g. a carboxyl or sulpho group, and are, for example, their salts with suitable bases, such as non-toxic metal salts derived from metals of group Ia, Ib, IIa and IIb of the periodic table of the elements, e.g. alkali metal salts, in particular lithium, sodium or potassium salts, alkaline earth metal salts, for example magnesium or calcium salts, furthermore zinc salts or ammonium salts, also those salts which are formed with organic amines, such as mono-, di- or trialkylamines optionally substituted by hydroxy, in particular mono-, di- or tri-lower alkylamines, or with quaternary ammonium bases, e.g. methyl-, ethyl-, diethyl- or triethylamine, mono-, bis- or tris(2-hydroxy-lower alkyl)amines, such as ethanol-, diethanol- or triethanolamine, tris(hydroxymethyl)methylamine or 2-hydroxy-tertiary-butylamine, N,N-di(lower alkyl)-N-(hydroxy-lower alkyl)amine, such as N,N-di-N-dimethyl-N-(2-hydroxyethyl)amine, or N-methyl-D-glucamine, or quaternary ammonium hydroxides, such as tetrabutylammonium hydroxide. The compounds of the formula I with a basic group, e.g. an amino group, can form acid addition salts, e.g. with suitable inorganic acids, e.g. hydrohalic acid, such as hydrochloric acid, hydrobromic acid, sulphuric acid with replacement of one or both protons, phosphoric acid with replacement of one or more protons, e.g. orthophosphoric acid or metaphosphoric acid, or pyrophosphoric acid with replacement of one or more protons, or with organic carboxylic, sulphonic or phosphonic acids or N-substituted sulphamic acids, e.g. acetic acid, propionic acid, glycolic acid, succinic acid, maleic acid, hydroxymaleic acid, methylmaleic acid, fumaric acid, malic acid, tartaric acid, gluconic acid, glucaric acid, glucuronic acid, citric acid, benzoic acid, cinnamic acid, mandelic acid, salicylic acid, 4-aminosalicylic acid, 2-phenoxybenzoic acid, 2-acetoxybenzoic acid, embonic acid, nicotinic acid, isonicotinic acid, furthermore amino acids, such as, for example, the α-amino acids mentioned further in advance, and methanesulphonic acid, ethanesulphonic acid, 2-hydroxyethanesulphonic acid, ethane-1,2-disulphonic acid, benzenesulphonic acid, 4-toluenesulfonic acid, naphthalene-2-sulfonic acid, 2- or 3-phosphoglycerate, glucose 6-phosphate, N-cyclohexysulphamic acid (with formation of the cyclamates) or with other acidic organic compounds, such as ascorbic acid. Compounds of the formula I with acidic and basic groups can also form internal salts.

For isolation and purification, pharmaceutically unsuitable salts can also be used.

The compound groups mentioned below are not to be considered as closed, but in a useful manner, for example for the replacement of general by more specific definitions, parts of these compound groups can mutually be replaced with one another or by the definitions given above or omitted. The definitions mentioned apply in the context of the general chemical principles, such as, for example, the customary valencies for atoms.

Preferred compounds according to the invention are those of the general formula (II) 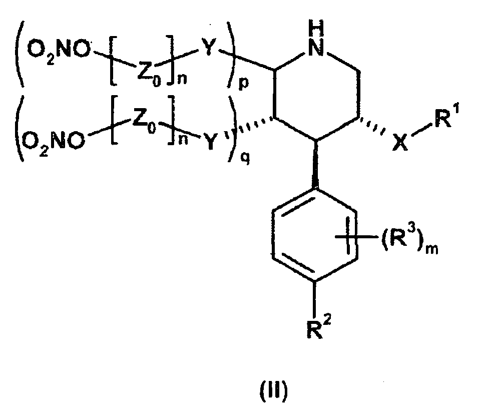 wherein R¹, R², R³, X, Y and Z₀, and m, n, p and q have the meaning indicated above for the compounds of the formula (I).

A further, preferred group of compounds of the formula (I), or particularly preferably of the formula (II) and their salts, preferably their pharmaceutically usable salts, are compounds wherein
m is 0, 1 or 2;
n is 0 or 1;
p is 0; and
q is 1.

A further, preferred group of compounds of the formula (I), or particularly preferably of the formula (II) and their salts, preferably their pharmaceutically usable salts, are compounds wherein
m is 0, 1 or 2;
n is 0 or 1;
p is 1; and
q is 0.

A further, preferred group of compounds of the formula (I), or particularly preferably of the formula (II) and their salts, preferably their pharmaceutically usable salts, are compounds wherein
Y a) is unsubstituted or heterocyclyl- and/or halogen- and/or hydroxy- and/or C₁₋₈-alkoxy-substituted
- Alk-,
- Alk-O-Alk-,
- Alk-NR⁴-Alk-,
- Alk-C(O)-Alk-,
- Alk-C(O)-NR⁴-Alk-,
- Alk-C(O)-Alk-NR⁴-Alk-,
- Alk-NR⁴-C(O)-Alk- or
- Alk-NR⁴-Alk-C(O)-Alk-,
   where Alk is linear or branched C₁₋₈-alkylene; or
   b) if p = 0, additionally
   i) a bond
   ii) unsubstituted or heterocyclyl- and/or halogen- and/or hydroxy- and/or C₁₋₈-alkoxy-substituted
- Alk-O-,
- Alk-NR⁴-
- Alk-C(O)- or
- Alk-NR⁴-C(O)-Alk-O-,
where Alk designates linear or branched C₁₋₈-alkylene;
m is 0, 1 or 2;
n is 0;
p is 0 or 1; and
q is 0 or 1;
where, if p is = 0, q is = 1 and if p is = 1, q is = 0.

A further, preferred group of compounds of the formula (I), or particularly preferably of the formula (II) and their salts, preferably their pharmaceutically usable salts, are compounds wherein

R¹ is aryl or heterocyclyl, in particular benz-imidazolyl, benzo[1,3]dioxolyl, benzofuranyl, benzothiazolyl, benzo[b]thienyl, 2H-chromenyl, dihydro-2H-benzo[1,4]oxazinyl, 3,4-dihydro-3H-benzo[1,4]oxazinyl, dihydro-2H-benzo[1,4]thiazinyl, 1 a,7b-dihydro-1H-cyclopropa[c]chromenyl, 2,3-dihydro-1H-indolyl, imidazo[1,2-a]pyridyl, imidazo[1,5-a]pyridyl, indazolyl, indolyl, isobenzofuranyl, phenyl, pyridyl or 1,1a,2,7b-tetra-hydrocyclopropa[c]chromenyl, which are substituted by 1-4 acyl-C₁₋₈-alkoxy-C₁₋₈-alkoxy, acyl- C₁₋₈-alkoxy-C₁₋₈-alkyl, (N-acyl)-C₁₋₈-alkoxy-C₁₋₈-alkyl-amino, C₁₋₈-alkanoyl, C₁₋₈-alkoxy, C₁₋₈-alkoxy-C₁₋₈-alkanoyl, C₁₋₈-alkoxy-C₁₋₈-alkoxy, C₁₋₈-alkoxy-C₁₋₈-alkoxy-C₁₋₈-alkyl, C-₁₋₈-alkoxy-C₁₋₈-alkyl, (N-C₁₋₈-alkoxy)-C₁₋₈-alkylaminocarbonyl-C₁₋₈-alkoxy, (N-C₁₋₈-alkoxy)-C₁₋₈-alkylaminocarbonyl-C₁₋₈-alkyl, C₁₋₈-alkoxy-C₁₋₈-alkyl-carbamoyl, C₁₋₈-alkoxy-C₁₋₈-alkylcarbonyl, C₁₋₈-alkoxy-C₁₋₈-alkylcarbonylamino, C₁₋₈-alkoxyaminocarbonyl-C₁₋₈-alkoxy, C₁₋₈-alkoxy-aminocarbonyl-C₁₋₈-alkyl, C₁₋₈-alkoxycarbonyl, C₁₋₈-alkoxycarbonyl-C-₁₋₈-alkoxy, C₁₋₈-alkoxycarbonyl-C₁₋₈-alkyl, C₁₋₈-alkoxycarbonylamino-C₁₋₈-alkoxy, C₁₋₈-alkoxy-carbonylamino-C₁₋₈-alkyl, C₁₋₈-alkyl, (N-C₁₋₈-alkyl)-C₁₋₈-alkoxy-C₁₋₈-alkylcarbamoyl, (N-C₁₋₈-alkyl)-C₁₋₈-alkoxy-C₁₋₈-alkylcarbonylamino, (N-C₁₋₈-alkyl)-C₁₋₈-alkoxy-carbonylamino, (N-C₁₋₈-alkyl)-C₁₋₈-alkylcarbonylamino-C₁₋₈-alkoxy, (N-C₁₋₈-alkyl)-C₁₋₈-alkylcarbonylamino-C₁₋₈-alkyl, (N-C₁₋₈-alkyl)-C₁₋₈-alkylsulphonylamino-C₁₋₈-alkoxy, (N-C₁₋₈-alkyl)-C₁₋₈-alkylsulphonylamino-C₁₋₈-alkyl, C₁₋₈-alkylaminocarbonyl-C₁₋₈-alkoxy, di-C₁₋₈-alkylaminocarbonyl-C₁₋₈-alkoxy, C₁₋₈-alkylaminocarbonyl-C₁₋₈-alkoxy-C₁₋₈-alkyl, C₁₋₈-alkyl-aminocarbonyl-C₁₋₈-alkyl, di-C₁₋₈-alkylaminocarbonyl-C₁₋₈-alkyl, C₁₋₈-alkylaminocarbonylamino-C₁₋₈-alkoxy, C₁₋₈-alkylaminocarbonylamino-C₁₋₈-alkyl, C₁₋₈-alkylcarbonylamino-C₁₋₈-alkoxy, C₁₋₈-alkyl-carbonylamino-C₁₋₈-alkyl, C₁₋₈-alkylcarbonyloxy-C₁₋₈-alkoxy, C₁₋₈-alkylcarbonyloxy-C₁₋₈-alkyl, C₁₋₈-alkylsulphonyl-C₁₋₈-alkoxy, C₁₋₈-alkyl-sulphonyl-C₁₋₈-alkyl, C₁₋₈-alkylsulphonylamino-C₁₋₈-alkoxy, C₁₋₈-alkylsulphonylamino-C₁₋₈-alkyl, optionally N-mono- or N,N-di-C₁₋₈-alkylated carbamoyl-C₀₋₈-alkoxy, optionally N-mono- or N,N-di-C₁₋₈-alkylated carbamoyl-C₀₋₈-alkyl, cyano-C₁₋₈-alkoxy, cyano-C₁₋₈-alkyl, C₃₋₈-cycloalkyl-C₁₋₈-alkoxy, C₃₋₈-cycloalkyl-C₁₋₈-alkyl, C₃₋₈-cycloalkylcarbonylamino-C₁₋₈-alkoxy, C₃₋₈-cycloalkylcarbonylamino-C₁₋₈-alkyl, halogen, halo-C₁₋₈-alkoxy, halo-C₁₋₈-alkyl, hydroxy-C₁₋₈-alkoxy-C₁₋₈-alkoxy, hydroxy-C₁₋₈-alkoxy-C₁₋₈-alkyl, hydroxy-C₁₋₈-alkyl, oxide or oxo; where heterocyclyl in C₁₋₈-alkoxy-C₁₋₈-alkylhetero-cyclyl, heterocyclyl-C₀₋₈-alkoxy, heterocyclyl-C₀₋₈-alkyl and heterocyclylcarbonyl is preferably a monocyclic, saturated or unsaturated heterocyclic radical with 3 to 8, particularly preferably 3 to 6, ring atoms, among them 1 to 4 nitrogen and/or 1 or 2 sulphur and/or oxygen atoms, for example imidazolyl, isoxazolyl, morpholinyl, oxazolyl, oxazolidinyl, oxetanyl, oxiranyl, pyridyl, pyrazolyl, piperidinyl, pyrrolidinyl, tetrahydropyranyl, tetrazolyl or triazolyl, which can be unsubstituted or mono- or polysubstituted, e.g. mono-, di-, tri- or tetra-substituted, in particular by (in the case of unsaturated heterocyclyl radicals) oxide or oxo or by C₁₋₈-alkoxy, C₁₋₈-alkyl, optionally N-mono- or N,N-di-C₁₋₈-alkylated amino, cyano, halogen or hydroxy;
where aryl in aryl-C₀₋₈-alkoxy and aryi-C₀₋₈-alkyl is preferably a mononuclear aromatic radical, for example phenyl; which can be unsubstituted or mono- or polysubstituted, e.g. mono- or disubstituted, by C₁₋₈-alkoxy, C₁₋₈-alkyl, optionally N-mono- or N,N-di-C₁₋₈-alkylated amino, cyano, halogen or hydroxy; and
where cycloalkyl in C₃₋₈-cydoalkyl-C₁₋₈-alkoxy, C₃₋₈-cycloalkyl-C₁₋₈-alkyl, C₃₋₈-cycloalkylcarbonylamino-C₁₋₈-alkoxy and C₃₋₈-cycloalkylcarbonylamino-C₁₋₈-alkyl is a saturated, cyclic hydrocarbon radical with 3 to 8, preferably 3-6, carbon atoms, for example cyclopropyl, cyclobutyl, cyclopentyl, cyclohexyl;
which can be unsubstituted or mono- or polysubstituted, e.g. mono- or disubstituted, by C₁₋₈-alkoxy, C₁₋₈-alkyl , optionally N-mono- or N,N-di-C₁₋₈-alkylated amino, cyano, halogen, hydroxy or oxo.

Particularly preferably, R¹ is benzimidazolyl, 2H-chromenyl, 3,4-dihydro-2H-benzo[1,4]oxazinyl, 1a,7b-dihydro-1H-cyclopropa[c]chromenyl, indazolyl, indolyl, 2,3-dihydro-1H-indolyl, phenyl or 1,1a,2,7b-tetrahydro-cyclopropa[c]chromenyl, which are mono- or poly-substituted, as indicated above; very particularly preferably mono- or poly-substituted by C₁₋₈-alkoxy, C₁₋₈-alkoxy-C₁₋₈-alkoxy, C₁₋₈-alkoxy-C₁₋₈-alkoxy-C₁₋₈-alkyl, C-₁₋₈-alkoxy-C₁₋₈-alkyl , C₁₋₈-alkoxycarbonylamino-C₁₋₈-alkoxy, C₁₋₈-alkoxycarbonylamino-C₁₋₈-alkyl, C₁₋₈-alkyl, (N-C₁₋₈-alkyl)-C₁₋₈-alkylcarbonylamino-C₁₋₈-alkoxy, (N-C₁₋₈-alkyl)-C₁₋₈-alkylcarbonylamino-C₁₋₈-alkyl , (N-C₁₋₈-alkyl)-C₁₋₈-alkylsulphonylamino-C₁₋₈-alkoxy, (N-C₁₋₈-alkyl)-C₁₋₈-alkylsulphonylamino-C₁₋₈-alkyl, C₁₋₈-alkylcarbonylamino-C₁₋₈-alkoxy, C₁₋₈-alkyl-carbonylamino-C₁₋₈-alkyl, C₁₋₈-alkylsulphonyl-C₁₋₈-alkoxy, C₁₋₈-alkyl-sulphonyl-C₁₋₈-alkyl, C₁₋₈-alkylsulphonylamino-C₁₋₈-alkoxy, C₁₋₈-alkylsulphonylamino-C₁₋₈-alkyl, C₃₋₈-cycloalkylcarbonylamino-C₁₋₈-alkoxy, C₃₋₈-cycloalkyl-carbonylamino-C₁₋₈-alkyl, halogen, halo-C₁₋₈-alkoxy, halo-C₁₋₈-alkyl or oxide.

A further, preferred group of compounds of the formula (I), or particularly preferably of the formula (II) and their salts, preferably their pharmaceutically usable salts, are compounds wherein
R² is C₁₋₈-alkoxy, C₁₋₈-alkoxy-C₁₋₈-alkoxy, optionally halogen-substituted C₁₋₈-alkoxy-C₁₋₈-alkoxy-C₁₋₈-alkyl, C₁₋₈-alkoxy-C₁₋₈-alkyl, C₁₋₈-alkoxy-C₁₋₈-alkylamino-C₁₋₈-alkoxy, C₁₋₈-alkoxy-C₁₋₈-alkylamino-C₁₋₈-alkyl, C₁₋₈-alkoxy-C₁₋₈-alkylsulphanyl-C₁₋₈-alkoxy, C₁₋₈-alkoxy-C-₁₋₈-alkylsulphanyl-C₁₋₈-alkyl, C₁₋₈-alkoxy-C₀₋₈-alkyl-C₃₋₈-cycloalkyl-C₀₋₈-alkoxy-C₁₋₈-alkyl, C₁₋₈-alkyl, C₁₋₈-alkylsulphanyl-C₁₋₈-alkoxy, C₁₋₈-alkylsulphanyl-C₁₋₈-alkoxy-C₁₋₈-alkoxy, C₁₋₈alkylsulphanyl-C₁₋₈-alkoxy-C₁₋₈-alkyl, C₁₋₈-alkylsulphanyl-C₁₋₈-alkyl, aryl-C₀₋₈-alkoxy-C₁₋₈-alkoxy, aryl-C₀₋₈-alkoxy-C₁₋₈-alkyl, aryloxy, C₃₋₈-cycloalkyl-C₀₋₈-alkoxy-C₁₋₈-alkoxy, C₃₋₈-cycloalkyl-C₀₋₈-alkoxy-C₁₋₈-alkoxy-C₁₋₈-alkyl, C₃₋₈-cycloalkyl-C₀₋₈-alkoxy-C₁₋₈-alkyl, heterocydyl-C₀₋₈-alkoxy-C₁₋₈-alkoxy, heterocyclyl-C₀₋₈-alkoxy-C₁₋₈-alkyl or heterocyclyloxy;
where heterocyclyl in heterocyclyl-C₀₋₈-alkoxy-C₁₋₈-alkoxy, heterocyclyl-C₀₋₈-alkoxy-C₁₋₈-alkyl and hetero-cyclyloxy is preferably a monocyclic, saturated or unsaturated heterocyclic radical with 3 to 8, particularly preferably 3 to 6 ring atoms, among them 1 to 4 nitrogen and/or 1 or 2 sulphur and/or oxygen atoms, for example imidazolyl, isoxazolyl, morpholinyl, oxazolyl, oxazolidinyl, oxetanyl, oxiranyl, pyridyl, pyrazolyl, piperidinyl, pyrrolidinyl, tetrahydropyranyl, tetrazolyl or triazolyl, which can be unsubstituted or mono- or polysubstituted, e.g. mono-, di-, tri- or tetra-substituted, in particular by (in the case of unsaturated heterocyclyl radicals) oxide or oxo or by C₁₋₈-alkoxy, C₁₋₈-alkyl, optionally N-mono or N,N-di-C₁₋₈-alkylated amino, aryl, cyano, halogen or hydroxy;
where aryl in aryl-C₀₋₈-alkoxy-C₁₋₈-alkoxy, aryl-C₀₋₈-alkoxy-C₁₋₈-alkyl, aryloxy, and also in aryl as a substituent on heterocyclyl, is preferably a mononuclear aromatic radical, for example phenyl; which can be unsubstituted or mono- or polysubstituted, e.g. mono- or disubstituted, by C₁₋₈-alkoxy, C₁₋₈-alkyl, optionally N-mono- or N,N-di-C₁₋₈-alkylated amino, cyano, halogen or hydroxy;
and
where cycloalkyl in C₃₋₈-cydoalkyl-C₀₋₈-alkoxy-C₁₋₈-alkoxy, C₃₋₈-cycloalkyl-C₀₋₈-alkoxy-C₁₋₈-alkoxy-C₁₋₈-alkyl and C₃₋₈-cycloalkyl-C₀₋₈-alkoxy-C₁₋₈-alkyl is a saturated, cyclic hydrocarbon radical with 3 to 8, preferably 3-6, carbon atoms, for example cyclo_propyl, cyclobutyl, cyclopentyl, cyclohexyl; which can be unsubstituted or mono- or polysubstituted, e.g. mono- or disubstituted, by C₁₋₈-alkoxy, C₁₋₈-alkyl, optionally N-mono- or N,N-di-C₁₋₈-alkylated amino, cyano, halogen, hydroxy or oxo.

Particularly preferably, R² is C₁₋₈-alkoxy, C₁₋₈-alkoxy-C₁₋₈-alkoxy, optionally halogen-substituted C₁₋₈-alkoxy-C₁₋₈-alkoxy-C₁₋₈-alkyl, C₁₋₈-alkoxy-C₁₋₈-alkyl, C₁₋₈-alkoxy-C₀₋₈-alkyl-C₃₋₈-cydoalkyl-C₀₋₈-alkoxy-C₁₋₈-alkyl, C₁₋₈-alkyl, aryl-C₀₋₈-alkoxy-C₁₋₈-alkoxy, aryl-C₀₋₈-alkoxy-C₁₋₈-alkyl, aryloxy, C₃₋₈₋cycloalkyl-C₀₋₈-alkoxy-C₁₋₈-alkoxy, C₃₋₈-cycloalkyl-C₀₋₈-alkoxy-C₁₋₈-alkoxy-C₁₋₈-alkyl, C₃₋₈-cycloalkyl-C₀₋₈-alkoxy-C₁₋₈-alkyl, heterocyclyl-C₀₋₈-alkoxy-C₁₋₈-alkoxy, heterocyclyl-C₀₋₈-alkoxy-C₁₋₈-alkyl or heterocyclyloxy, where heterocyclyl in heterocyclyl-C₀₋₈-alkoxy-C₁₋₈-alkoxy, heterocyclyl-C₁₋₈-alkoxy-C₁₋₈-alkyl and hetero-cyclyloxy is preferably a monocyclic, saturated or unsaturated heterocyclic radical with 3 to 8, particularly preferably 3 to 6, ring atoms, among them 1 to 4 nitrogen and/or 1 or 2 sulphur and/or oxygen atoms, for example imidazolyl, isoxazolyl, morpholinyl, oxazolyl, oxazolidinyl, oxetanyl, oxiranyl, pyridyl, pyrazolyl, piperidinyl, pyrrolidinyl, tetrahydropyranyl, tetrazolyl or triazolyl, which can be unsubstituted or mono- or polysubstituted, e.g. mono-, di-, tri- or tetrasubstituted, in particular by (in the case of unsaturated heterocyclyl radicals) oxide or oxo or by C₁₋₈-alkoxy, C₁₋₈-alkyl, optionally N-mono- or N,N-di-C₁₋₈-alkylated amino, aryl, cyano, halogen or hydroxy;
where aryl in aryl-C₀₋₈-alkoxy-C₁₋₈-alkoxy, aryl-C₀₋₈-alkoxy-C₁₋₈-alkyl, aryloxy, and also in aryl as a substituent on heterocyclyl, is preferably a mononuclear aromatic radical, for example phenyl; which can be unsubstituted or mono- or polysubstituted, e.g. mono- or disubstituted, by C₁₋₈-alkoxy, C₁₋₈-alkyl, optionally N-mono- or N,N-di-C₁₋₈-alkylated amino, cyano, halogen or hydroxy;
and
where cycloalkyl in C₃₋₈-cycloalkyl-C₀₋₈-alkoxy-C₁₋₈-alkoxy, C₃₋₈-cycloalkyl-C₀₋₈-alkoxy-C₁₋₈-alkoxy-C₁₋₈-alkyl and C₃₋₈-cycloalkyl-C₀₋₈-alkoxy-C₁₋₈-alkyl is a saturated, cyclic hydrocarbon radical with 3 to 8, preferably 3-6, carbon atoms, for example cyclopropyl, cyclobutyl, cyclopentyl, cyclohexyl; which can be unsubstituted or mono- or polysubstituted, e.g. mono- or disubstituted, by C₁₋₈-alkoxy, C₁₋₈-alkyl, optionally N-mono- or N,N-di-C₁₋₈-alkylated amino, cyano, halogen, hydroxy or oxo.

A further, preferred group of compounds of the formula (I), or particularly preferably of the formula (II) and their salts, preferably their pharmaceutically usable salts, are compounds wherein
X is -Alk-, -O-all-, -Alk-O-, -O-Alk-O-, -S-Alk-, -Alk-S-, -NR⁴-Alk-, -Alk-NR⁴-, -NR⁴-C(O)-, -C(O)-NR ⁴- or -O-Alk-C(O)-NR⁴, where Alk designates C₁₋₈-alkylene.

A further, preferred group of compounds of the formula (I), or particularly preferably of the formula (II) and their salts, preferably their pharmaceutically usable salts, are compounds wherein
m is = 0.

A further, preferred group of compounds of the formula (I), or particularly preferably of the formula (II) and their salts, preferably their pharmaceutically usable salts, are compounds wherein
Y is a) unsubstituted or heterocyclyl- and/or halogen- and/or hydroxy- and/or C₁₋₈-alkoxy-substituted
- Alk-,
- Alk-O-Alk-,
- Alk-C(O)-NR⁴-Alk- or
- Alk-NR⁴-C(O)-Alk-,
   where Alk designates linear or branched C₁₋₈-alkylene, or;
   b) if p = 0, is additionally
   i) a bond
   ii) unsubstituted or heterocyclyl- and/or halogen- and/or hydroxy- and/or C₁₋₈-alkoxy-substituted
- Alk-O-,
- Alk-NR⁴-,
- Alk-C(O)- or
- Alk-NR⁴-C(O)-Alk-O-,
where Alk designates linear or branched C₁₋₈-alkylene and
R⁴ is H or C₁₋₈-alkyl; and
where heterocyclyl in heterocyclyl-C₀₋₈-alkoxy-C₁₋₈-alkoxy, heterocyclyl-C₀₋₈-alkoxy-C₁₋₈-alkyl and hetero-cyclyloxy is preferably a monocyclic, saturated or un-saturated heterocyclic radical with 3 to 8, particularly preferably 3 to 6, ring atoms, among them 1 to 4 nitrogen and/or 1 or 2 sulphur and/or oxygen atoms, for example imidazolyl, isoxazolyl, morpholinyl, oxazolyl, oxazolidinyl, oxetanyl, oxiranyl, pyridyl, pyrazolyl, piperidinyl, pyrrolidinyl, tetra-hydropyranyl, tetrazolyl or triazolyl, which can be unsubstituted or mono- or polysubstituted, e.g. mono-, di-, tri- or tetrasubstituted, in particular by (in the case of unsaturated heterocyclyl radicals) oxide or oxo or by C-₁₋₈-alkoxy, C-₁₋₈-alkyl, optionally N-mono- or N,N-di-C₁₋₈-alkylated amino, aryl, cyano, halogen or hydroxy.

A further, preferred group of compounds of the formula (I), or particularly preferably of the formula (II) and their salts, preferably their pharmaceutically usable salts, are compounds wherein
Z₀ is equal to -U-Z₁-
wherein Z₁ is -C(O)-O-, -O-C(O)O-, -C(O)-NR⁷-C(O)- or -O-C(O)-NR⁷-C(O)-, wherein R⁷ is H or C₁-₄-alkyl;
U is a bivalent radical having the following meaning:
a)
   linear or branched C₁₋₈-alkylene
b) 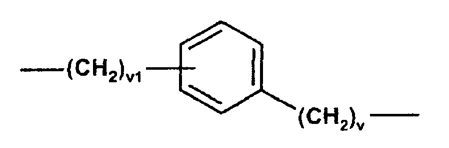 wherein v is 0 or 1, and v1 is 1; or
g) 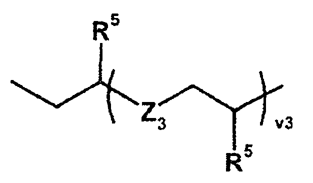 wherein Z₃ is -O- or -S-, v3 is 1 and R⁵ is H.

The compounds of the formula (I) and (II) can be prepared in an analogous manner to preparation processes known from the literature. Similar preparation processes are described, for example, in WO 97/09311. Details of the specific preparation variants can be taken from the examples. -

The compounds of the formulae (I) and (II) can also be prepared in optically pure form. Separation into antipodes can be carried out by methods known per se either preferably in an early stage in the synthesis by salt formation with an optically active acid such as, for example, (+)- or (-)-mandelic acid and separation of the diastereomeric salts by fractional crystallization or preferably in a rather late stage by derivatization with a chiral auxiliary structural unit such as, for example, (+)- or (-)-camphanyl chloride, and separation of the diastereomeric products by chromatography and/or crystallization and subsequent cleavage of the bond to the chiral auxiliary. The pure diastereomeric salts and derivatives can be analysed using customary spectroscopic methods for determination of the absolute configuration of the piperidine contained, X-ray spectroscopy on single crystals being a particularly suitable method.

The compounds of the formulae (I) and (II) also include those compounds in which one or more atoms are replaced by their stable, non-radioactive isotopes; for example a hydrogen atom by deuterium.

Prodrug derivatives of the compounds presently described are derivatives thereof which in *in vivo* use release the original compound by means of a chemical or physiological process. A prodrug can be reacted, for example, to the original compound on achieving a physiological pH or by enzymatic conversion. Prodrug derivatives can be, for example, esters of freely available carboxylic acids, S- and O-acyl derivatives of thiols, alcohols or phenols, the acyl group being defined as presently. Pharmaceutically usable ester derivatives, which can be reacted by solvolysis in physiological medium to the original carboxylic acid, such as, for example, lower alkyl esters, cycloalkyl esters, lower alkenyl esters, benzyl esters, mono- or disubstituted lower alkyl esters, such as lower ω-(amino, mono- or dialkylamino, carboxyl, lower alkoxycarbonyl)alkyl esters or such as lower ω-(alkanoyloxy, alkoxycarbonyl or dialkylaminocarbonyl)-alkyl esters are preferred; pivaloyloxymethyl esters and similar esters are conventionally utilized.

On account of the close relationship between a free compound and a salt compound, a certain compound in this invention also comprises its salt form, if this is possible and appropriate.

The compounds of the formula (I) or (II), in particular the compounds of the Examples 1-62, and their pharmaceutically usable salts show extended properties in that on the one hand they release nitrogen monoxide and on the other hand either directly inhibit the natural enzyme renin as illustrated by the Examples 1-45 or inhibit renin indirectly upon drug metabolism as illustrated by the Examples 46-62. The release of nitrogen monoxide and its pharmacokinetic distribution in the living organism together with the inhibition of renin in the systemic circulation and in the tissue results in unexpectedly useful pharmacological properties, which can be explained as follows.

The juxtaglomerular cells of the kidney secrete the enzyme renin into the systemic circulation, where renin proteolytically cleaves the substrate angiotensinogen into the decapeptide product angiotensin I. Angiotensin I is further processed proteolytically by the angiotensin-converting enyzme to the octapeptide angiotensin II. Angiotensin II increases the blood pressure by causing arterial vasoconstriction by receptor binding and a decrease in sodium excretion. Moreover, angiotensin II increases the activity of NADPH oxidase and thus leads to the breakdown of nitrogen monoxide, which leads to a decrease in endothelium-dependent vascular relaxation, to hypertrophy, proliferation and migration of the smooth muscle cells, to the formation of extracellular matrix, and to thrombotic and inflammatory reactions. The inhibition of the enzymatic renin activity leads to decreased formation of angiotensin I and thus to the lowering of the angiotensin II content. The suppression of angiotensin II formation leads to a decrease in the arterial blood pressure and to the prevention of blood pressure-dependent and blood pressure-independent tissue damage.

Nitrogen monoxide has the property of reversibly activating soluble guanylate cyclase, a widespread enzyme which can be found in the cells of any organ system. In this process, nitrogen monoxide binds to the haem-containing domain of the enzyme, by means of which its catalytic activity, which converts guanosine triphosphate to cyclic guanosine monophosphate, is increased. Increased concentrations of cyclic guanosine monophosphate relax the smooth muscle cells in the venous and, in some cases, arterial blood vessels, in the heart, in the intestine, in the urogenital tract, in the airways and in the uterus; they moreover inhibit the aggregation and adhesion of platelets and block the accumulation of leucocytes on the vessel walls. These effects, so it is believed, explain the vasculoprotective contributions of nitrogen monoxide. In fact, virtually any cardiovascular risk factor, such as, for example, high blood pressure, diabetes, hyperlipidaemia and smoking can be connected with a decrease in the basal and in the stimulated nitrogen monoxide-induced vascular relaxation.

The invention described herein makes available a new therapeutic approach for high blood pressure and blood pressure-dependent and blood pressure-independent diseases in that the compounds of the formulae (I) and (II) on the one hand inhibit high blood pressure-promoting and tissue-damaging renin and on the other hand release tissue-relaxing and tissue-protecting nitrogen monoxide. Pharmacologically, the compounds of the formulae (I) and (II) allow the relaxation of the arterial and of the venous blood vessels by systemic distribution of the compounds and thus systemic renin inhibition and nitrogen monoxide release. In contrast to the separate use of both active principles, i.e. of a renin inhibitor and of a nitrogen monoxide-releasing substance, the compounds of the invention described herein allow a uniform systemic distribution and thus a balanced effect on blood and blood vessels, blood pressure and tissue.

The pharmacological profile of the compounds of the formulae (I) and (II) can be worked out using the following system. The in vitro test systems described herein allow, independently of one another, the direct determination of the renin-inhibiting properties and the nitrogen monoxide-releasing properties of a compound. The *in vivo* test systems described herein allow the determination of the combined effect of renin inhibition be it directly or upon drug metabolism and nitrogen monoxide release on the blood pressure and on tissue functions.

In order to distinguish the blood pressure-lowering and tissue-protecting contributions of renin inhibition from nitrogen monoxide release of an orally administered compound, the blood pressure lowering and tissue function of a nitrate ester compound are compared with the nitrate-free parent substance with equal pharmacokinetic distribution. If the release of the nitrogen monoxide does not take place or takes place in a phramacollogically unsuitable manner, no additional blood pressure lowering or no tissue protection can be shown. If, however, the pharmacokinetic release and distribution, for probably not conclusively explainable reasons, take place on the nitrogen monoxide-sensitive and blood pressure-regulating tissues, as shown presently an additional blood pressure-lowering or tissue protection of the heart and kidneys can be functionally shown.

The determination of the direct renin-inhibiting properties of a compound can be carried out using in vitro enzymatic test systems. The in vitro test systems determine the formation of angiotensin I from natural or recombinant renin substrate in the presence of human plasma samples or purified renin enzyme. A frequently used in vitro test system has been described by Nussberger et al. (1987) in J. Cardiovascular Pharmacology, Vol. 9, pp. 39-44. The test quantitatively determines the formation of angiotensin I in human plasma in the presence or absence of renin inhibitors in various concentration ranges. The angiotensin I concentrations produced are measured by a radioimmuno investigation.

The compounds of the present invention, in particular the compounds illustrated in Examples 1-45, showed in these *in vitro* test systems direct renin-inhibiting actions in minimal concentration ranges from 10⁻¹⁰ to 10⁻⁷ mol/l. The efficacy of the compounds can be expressed using IC50 values, which represent the concentration of a certain compound which suppresses the angiotensin I content by 50%. The IC50 values of the compounds obtained were in the range from 0.1 nM to 100 nM, the majority of these in the range from 1 nM to 10 nM. Certain nitrate ester compounds showed similar, i.e. higher, identical or alternatively lower, IC50 values in comparison with their nitrate-free parent substances.

The determination of the nitrogen monoxide-releasing properties of a compound, be it by enzymatic or non-enzymatic action, can be carried out by means of *in vitro* vascular reactivity tests. The *in vitro* vascular reactivity test measures the ability of the released nitrogen monoxide to relax a pretensioned aorta ring or vein ring which is kept in an organ chamber. Frequently used instructions are described by Gonzales et al., in Adv. Physiol. Educ. (2000) 24:13-21. The thoracic aorta or the femoral vein is isolated from sacrificed and exsanguinated Wistar rats and cut up to give rings 2 mm long. The vascular rings are stored in organ chambers. Changes in the development of tension after the action of compounds are recorded by an isometric signal transmitter, which is connected to a computerized detection system. The computer program analyses time curves, period and size amounts between contractions. The test compounds described here showed vessel-relaxing actions in phenylephrine-precontracted vessels in concentration ranges of approximately 10⁻⁵ to 10⁻⁴ mol/l. The nitrogen monoxide-induced vessel-relaxing activity of the compounds can be expressed in percentage points of the maximal vessel relaxation achieved with sodium nitroprusside (SNP) relative to the phenylephrine (0.1 µM) -induced contractile tone; at 100 µM the compound of Example 6 showed 15% relaxation, the compound of Example 8 20%, the compound of Example 9 8%, the compound of Example 14 25%, the compound of Example 21 20% and the compound of Example 34 8%.

The determination of the in vitro inhibition of platelet aggregation (IPA) by nitrogen monoxide can be carried out using the following test system. Platelet-enriched plasma from rat blood is obtained by centrifugation. The aggregability of the platelets can be measured optically using a turbidimetric aggregometer. The aggregation-inhibiting action of the compounds of the formulae (I) and (II) can be determined relative to aggregation-stimulating agents using adenosine diphosphate (ADP). The test compounds (10 - 500 uM) can be added to the platelet solution here 1-5 minutes before introduction of ADP (1-10 uM). The optical density determined in the test system determines the degree of platelet aggregation.

The compounds of the formulae (I) and (II) led to a decrease in the ADP-induced platelet aggregation. The percentage decrease was between 20 and 60%.

The in vivo blood pressure-lowering actions of the compounds of the formulae (I) and (II) can be shown in doubly transgenic rats (dTGR), which overexpress both the gene for human angiotensinogen and the gene for human renin and as a result of this develop hypertension. Blood pressure values and heart rate values can be determined continuously, for example, by means of chronically implanted telemetry equipment. A telemetry system can consist of a radio frequency transmitter, a receiver apparatus and a data-detecting system. The pressure transmission catheter of the pressure sensor can be implanted into the abdominal aorta. After the operation, the rats are allowed a recovery period of 7 days, where the telemetry recording should indicate the restoration of a 24 hour oscillation rhythm of blood pressure and heart rate before compounds can be tested.

Compounds of the formula (I) and (II) can be administered orally by means of stomach tubes. Blood pressure changes can be recorded over 24 hours after a single dosage or continuously over 2 to 42 days after multiple dosage. The dose administered, consisting of vehicle-suspended or dissolved compound, is in the range from 0.5 mg/kg of body weight up to 100 mg/kg of body weight. Blood pressure changes can be expressed by means of mean arterial blood pressure values (MAP), in order to describe the average pressure within a cardiac cycle.

The use of the compounds of the formulae (I) and (II) resulted in mean arterial blood pressure lowerings (MAP) of 20 to 70 mmHg. Moreover, certain nitrate ester compounds showed stronger blood pressure lowerings in comparison to their nitrate-free parent compounds.

The pharmacokinetic distribution of a compound of the formula (I) or (II) can be determined by comparison of the time-dependent plasma concentrations of a compound after oral or intravenous administration to a living organism such as, for example, a mouse, a rat or a dog. The doses used for oral administration range from 0.5 to 50 mg/kg of body weight; for intravenous administration doses of 0.5 to 20 mg/kg of body weight are administered. For intravenous use, the usable formulation of a compound can be administered to groups of 3 to eight animals, for example, to the caudal vein of a rat and, for oral use, by means of a stomach tube. Ethically justifiable blood sample volumes can be taken from the animals, according to a suitable time pattern, for example automatically by means of an AccuSampler (DiLab Europe, Lund, Sweden), and transferred to heparinized containers. Plasma samples produced are stored, up to the determination of the concentration of a compound contained, for example by liquid chromatography and mass spectral analysis, at -17 to -23°C. Compounds of the formulae (I) and (II) showed absolute bioavailabilities in the range from 10-50% and elimination half-lives of 2 - 12 h. The plasma levels of a compound can also be expressed by area under the curve (AUC) values, which allow an additional comparison of the compounds. Thus, compounds of the formulae (I) and (II) showed AUC values in the range from 500 to 15 000 ng x h/ml.

The influencing of the coronary flow and anti-ischaemic effects of the compounds of the formulae (I) and (II) can also be measured in vivo by means of a perfusion model. For this, the heart is removed from the rats pretreated with the compounds of the formulae (I) and (II) after anaesthesia and this is mounted in a Langendorff apparatus after cannulation of the aorta, whereby it can be supplied with oxygen-rich perfusate. After tying off the vena cava and pulmonary veins, and the cannulation of the pulmonary artery, the coronary flow can be determined by means of a flow meter. The coronary flow was measured volumetrically and expressed as the millilitres of perfusate which are collected in one minute. The coronary flow can be measured before and after induction of ischaemia, ischaemia being initiated by interruption of the perfusion for 30 minutes and a 30-minute reperfusion period following after opening of the flow. Thus the coronary flow in the reperfusion period can be compared with that in the pre-ischaemic period. Compounds of the formulae (I) and (II) are thereby able to increase the coronary flow by 50-200%.

The tissue-protecting action of a compound of the formula (I) and (II) after long-term use can be determined in vivo by proteinuria and kidney function measurements, as indicators of kidney damage. The investigations can be carried out in 4-week-old, male rats, for human renin and angiotensinogen doubly transgenic rats (dTGR). The animals are divided into treatment groups and are given drug, comparison substance or vehicle (control) for 7 weeks. The dose used for oral administration can range from 0.5 to 100 mg/kg of body weight. During the entire study period, the animals are given standard feed and tap water ad libitum. The animals are placed in a metabolic cage once weekly in order to determine the 24-hour albumin excretion (AE) in the urine, diuresis, natriuresis and urine osmolality. Moreover, the kidneys can be functionally investigated by determining the glomerular filtration rate, for example using the lohexol method, creatinine excretion, for example by means of the plasma creatinine concentration, and the renal perfusion rate, for example using the para-aminohippurate method. At the end of the study, the animals are sacrificed and the kidneys and hearts are removed for weight determination and immunohistological investigations (tissue fibrosis, leucocyte infiltration, inflammation markers etc.). The extent of tissue fibrosis can be shown, for example, with the following polyclonal antibodies; anti-fibronectin, anti-collagen IV. The extent of cell infiltration can be shown, for example, with the following monoclonal antibodies: anti-CD4, anti-CD8, anti-ED1, anti-MHCII, anti-CD68 and anti-Ox6. As inflammation markers, immunologically captured TGFβ, MCP-1, TNFα or IL-6 can be used. The semiquantitative evaluation of various kidneys and heart sections showed a decrease in tissue fibrosis and tissue inflammation after use of the compounds of the formulae (I) and (II).

| | IC50 µM | IPA | MAP mmHg | AUC | AE ug/24h | CF % | Fib Cl |
|---|---|---|---|---|---|---|---|
| Compound X | 10-100 | 50% | 20-70 | 500-15 000 | 100-5000 | 50-200 | + |
| Parent compound X | 0 | 20% | 20-50 | 500-15 000 | 200-10000 | 20-50 | ++ |

The comparison of these pharmacological parameters allows not only the improved profile of a nitrate ester compound to be observed compared to its nitrate-free parent compound, but also the optimal nitrate ester compounds of this invention to be identified. Abbreviations: IC, inhibitory concentration; pIC, negativer logarithm of the IC; IPA, inhibition of platelet aggregation; MAP, mean arterial pressure;

AUC, area under the curve; AE, albumin excretion; CF, coronary flow; Fib, fibrosis; CI, cell infiltration.

In order to achieve the desired effects in a patient to be treated, the compounds of the present invention can be administered orally or enterally, such as, for example, intravenously, intraperitoneally, intra- muscularly, rectally, subcutaneously or alternatively by direct injection of the active agent locally into tissue or tumours. The term patient paraphrases warm-blooded animals and mammals, such as, for example, humans, primates, cattle, dogs, cats, horses, sheep, mice, rats and pigs. The compounds can be administered as a pharmaceutical preparation or incorporated into an administration device which guarantees lasting effusion of the compound. The amount of substance to be administered can vary over a wide range and can be any effective dose. Depending on the patients to be treated or condition to be treated and type of administration, the dose of the effective active agent can be between approximately 0.005 and 50 milligrams per kilogram of body weight daily, but preferably between approximately 0.05 and 15 milligrams per kilogram of body weight daily.

For oral administration, the compounds can be formulated in solid or liquid pharmaceutical forms such as, for example, as capsules, pills, tablets, coated tablets, granules, powders, solutions, suspensions or emulsions. The dose of a solid pharmaceutical form can be an ordinary hard gelatin capsule, which can be filled with active agents and excipients, such as lubricants and fillers, such as, for example, lactose, sucrose and maize starch. A further administration form can be the tabletting of the active substance of the present invention. The tabletting can be carried out with conventional tabletting auxiliaries such as, for example, lactose, sucrose, maize starch, combined with binders consisting of Acacia gum, maize starch or gelatin, disintegrants such as potato starch or cross-linked polyvinylpyrrolidone (PVPP) and lubricants such as stearic acid or magnesium stearate. Suitable excipients for soft gelatin capsules are, for example, vegetable oils, waxes, fats, semisolid and liquid polyols etc. Suitable excipients for the preparation of solutions and syrups are, for example, water, polyols, sucrose, invert sugar, glucose etc.

For rectal administration, the compounds can be formulated in solid or liquid pharmaceutical forms such as, for example, suppositories. Suitable excipients for suppositories are, for example, natural or hardened oils, waxes, fats, semiliquid or liquid polyols etc.

For parenteral administration, the compounds can be formulated as an injectable dose of the active agent in a liquid or suspension. The preparations usually contain a physiologically tolerable sterile solvent, which can contain a water-in-oil emulsion, with or without surfactant, and other pharmaceutically acceptable excipients. Oils which can be used for preparations of this type are paraffins and triglycerides of plant, animal or synthetic origin, such as, for example peanut oil, soya bean oil and mineral oil. In general, injectable solutions contain liquid carriers such as more preferably water, common salt, dextrose or related sugar solutions, ethanol and glycols, such as propylene glycol or polyethylene glycol.

The substances can be administered as a transdermal patch system, as a depot injection or implant if the formulation makes possible a lasting release of the active agent. The active agent can be compressed as granules or to give narrow cylinders and can be administered subcutaneously or intramuscularly as a depot injection or implant.

In addition, the pharmaceutical preparations can further contain preservatives, solubilizers, viscosity-increasing substances, stabilizers, wetting agents, emulsifiers, sweeteners, colourants, flavourings, salts for alteration of the osmotic pressure, buffers, coating agents or antioxidants. They can also contain other therapeutically valuable substances.

A further subject of the present invention is the use of the compounds of the formula (I), or preferably of the formula (II), and their pharmaceutically usable salts in the treatment and/or prevention of renin-mediated diseases which are complicated by nitrogen monoxide deficiency, such as, for example, high blood pressure, left-ventricular hypertrophy, heart failure, stable angina pectoris, unstable angina pectoris, angina, acute coronary syndrome, vasospastic angina, stroke, ischaemic disorders, cardiac infarction, ischaemic reperfusion injuries, Raynaud's syndrome, thrombosis, atrial fibrillation, cardiac arrhythmias, dyslipid-aemia, atherosclerosis, prevention of stenoses after angioplasties, peripheral arterial occlusive diseases, erectile disorders, diabetes type 1 and type 2, diabetic complications, nephropathy, retinopathy, neuropathy, pulmonary arterial hypertension, disorders of the digestive tract, portal hypertension, cirrhosis of the liver.

The presently described compounds of the invention allow the following methods of use:
- As a therapeutic combination in the form of a preparation or of a kit which is composed of individual components, consisting of a presently described compound, in free form or as a pharmaceutically usable salt, and at least one pharmaceutical form whose active agent has a blood pressure-lowering, an inotropic, an antidiabetic, a lipid-lowering or an antioxidizing action, which can be used either simultaneously or sequentially. The preparation and the kit can contain use instructions.
- As a method for combined use, such as, for example, in a simultaneous or sequential sequence, of a therapeutically efficacious amount of a compound described here, in free or in pharmaceutically usable salt form, and of a second active agent having blood pressure-lowering, inotropic, anti-diabetic, lipid-lowering or anti-oxidizing action.

The presently described compounds and their pharmaceutically usable salts can be used in combination with:
(i) one or more blood pressure-lowering active agents, as of the type, for example:
   - angiotensin II receptor inhibitors such as candesartan, irbesartan, olmesartan, losartan, valsartan, telmisartan, eprosartan etc.;
   - angiotensin-converting enzyme (ACE) inhibitors such as quinopril, ramipril, trandolapril, lisinopril, captopril, enalapril etc.;
   - calcium channel inhibitors such as nifedipine, nicardipine, verapamil, isradipine, nimodipine, amlodipine, felodipine, nisoldipine, diltiazem, fendiline, flunarizine, perhexiline, gallopamil etc.;
   - diuretics such as hydrochlorothiazide, chloro-thiazide, acetazolamide, amiloride, bumetanide, benzthiazide, ethacrynic acid, furosemide, indacrinone, metolazone, triamterene, chlor-thalidone etc.;
   - aldosterone receptor antagonists such as spironolactone, eplerenone;
   - endothelin receptor antagonists such as bosentan;
   - phosphodiesterase inhibitors such as amrinone, sildenafil;
   - direct vasodilators such as dihydralazine, minoxidil, pinacidil, diazoxide, flosequinan etc.;
   - α- and β-adrenergic receptor antagonists such as phentolamine, phenoxybenzamine, prazosine, doxazosine, terazosine, carvedilol, atenolol, metoprolol, nadolol, propranolol, timolol, carteolol etc.;
   - neutral endopeptidase (NEP) inhibitors;
   - sympatholytics such as methyldopa, clonidine, guanabenz, reserpine;
(ii) one or more inotropic active agents, as of the type, for example:
   - cardiac glycosides such as digoxin;
   - α-adrenergic receptor stimulators such as dobutamine
   - thyroid hormones such as thyroxine
(iii) one or more anti-diabetic active agents, as of the type, for example:
   - insulins such as insulin aspartate, human insulin, insulin lispro, insulin glargine and other fast-, medium- or long-acting insulin derivatives and combinations;
   - insulin sensitizers such as rosiglitazone, pioglitazone;
   - sulphonylureas such as glimepiride, chlorprop-amide, glipizide, glyburide etc.;
   - biguanides such as metformin;
   - glucosidase inhibitors such as acarbose, miglitol,
   - meglitinides such as repaglinide; nateglinide etc.;
(iv) one or more lipid-lowering active agents, as of the type, for example:
   - HMG-CoA reductase inhibitors such as lovastatin, fluvastatin, pravastatin, atorvastatin, simva-statin, rosuvastatin etc.;
   - fibrate derivatives such as fenofibrate, gemfibrozil etc.;
   - bile acid-binding active agents such as colestipol, colestyramine, colesevelam;
   - cholesterol absorption inhibitors such as ezetimibe;
   - lipase inhibitors such as orlistate;
   - nicotinic acid such as niacin
(v) one or more active agents with a direct or indirect anti-oxidant effect, as of the type, for example:
   - vitamins and vitamin derivatives such as beta-carotene, lycopene, vitamin A such as retinol (vitamin A1), 3,4-didehydroretinol (vitamin A2), and 3-hydroxyretinol (vitamin A3), vitamin C or ascorbic acid and vitamin E or α-tocopherol,
   - lipoic acid, 2-mercaptoethane sulphonate, cysteine,
   - enzymes such as superoxide dismutase, catalase etc.;
   and other active agents which are used for the treatment of high blood pressure, vascular, kidney and liver disorders and are also usable for the prevention of medicinal tolerance symptoms. Combinations of this type can be used separately or in preparations which contain a number of components.

The presently described compounds and their pharmaceutically usable salts can be of use as combinations with:
(i) a diagnostic test system which allows the quantitative determination of the plasma renin concentration (PRC)
(ii) a diagnostic test system which allows the quantitative determination of the plasma aldosterone concentration (PAC)
(iii)a diagnostic test system which allows the quantitative determination of the plasma renin activity (PRA)
(iv) a diagnostic test system which allows the quantitative determination of the ratio of the plasma aldosterone concentration to the renin concentration (ARC)
(v) a diagnostic test system which allows the quantitative determination of the ratio of the plasma aldosterone concentration to the plasma renin activity (ARR).

Such combinations of a diagnostic test system and a therapy can be used independently or as a preparation with individual components.

### EXAMPLES

The following examples illustrate the present invention. All temperatures are indicated in degrees Celsius, pressures in mbar. If not mentioned otherwise, the reactions take place at room temperature. The abbreviation "Rf = xx (A)" means, for example, that the Rf value xx is determined in the solvent system A. The quantitative ratio of solvents to one another is always indicated in parts by volume. Chemical names for final products and intermediates were generated with the aid of the programme AutoNom 2000 (Automatic Nomenclature).

Thin layer chromatography mobile phase systems:
A dichloromethane-methanol-ammonia conc. 25% = 200:20:1
B dichloromethane-methanol-ammonia conc. 25% = 200:20:0.5
C dichloromethane-methanol-ammonia conc. 25% = 200:10:1
D dichloromethane-methanol-ammonia conc. 25% = 90:10:1
E dichloromethane-methanol-ammonia conc. 25% = 60:10:1
F dichloromethane-methanol-ammonia conc. 25% = 200:30:1
G dichloromethane-methanol = 9:1
HPLC gradient on Hypersil BDS C-18 (5 µm); column: 4 × 125 mm
I 90% water*/10% acetonitrile* to 0% water*/100% acetonitrile* in 5 minutes + 2.5 minutes (1.5 ml/min)
H 95% water*/5% acetonitrile* to 0% water*/100% acetonitrile* in 40 minutes (0.8 ml/min)
*contains 0.1 % trifluoroacetic acid

The following abbreviations are used:
Rf ratio of running distance of a substance to distance of the eluent front from the starting point in thin layer chromatography
Rt retention time of a substance in HPLC (in minutes)
M.p. Melting point (temperature)

### General method A: (N-Boc deprotection I)

The solution of 1 mmol of "N-Boc derivative" in 5 ml of chloroform is treated successively with 15 ml of methanol and 2.5 ml of 2N HCl and stirred for 18 hours at 60°C. The reaction mixture is cooled to room temperature, poured onto 1 M aqueous sodium hydrogen-carbonate solution (40 ml) and extracted with tert-butyl methyl ether (2 × 60 ml). The organic phases are washed with brine (1 × 60 ml), dried using sodium sulphate and evaporated. The title compound is obtained from the residue by means of flash chromatography (SiO₂ 60F).

### General method B: (hydrogenation)

The solution of 1 mmol of "substrate" in 15 ml of tetrahydrofuran-methanol 1:1 is hydrogenated in the presence of 100 - 200 mg of Pd/C 10% for 2-20 hours at 15 - 20°C. The reaction mixture is subjected to clarifying filtration and the filtrate is evaporated. The title compound is obtained from the residue by means of flash chromatography (SiO₂ 60F).

### General method C: (9-BBN reduction)

The solution of 1 mmol of "lactam" in 3 ml of tetrahydrofuran is treated with 3.2 - 6.4 mmol of 9-BBN (0.5M in tetrahydrofuran) and stirred at reflux for 1-2 hours (conversion check using HPLC). The reaction mixture is cooled to room temperature, treated with 3.2 - 6.4 mmol of ethanolamine and evaporated. The residue is stirred overnight at 0°C in ethyl acetate - heptane 1:1 (30 ml), subjected to clarifying filtration and the filtrate is evaporated. The title compound is obtained from the residue by means of flash chromatography (SiO₂ 60F).

### General method D: (O-alkylation I)

The solution of 1 mmol of "alcohol" and 1.0 - 2.0 mmol of "benzyl halide" in 2.0 ml of N,N-dimethylformamide is treated with stirring at -10°C with 1.1 mmol of sodium hydride (60% dispersion in oil). The reaction mixture is stirred for 1 hour at -10°C and 18 hours at room temperature. The mixture is poured onto 1 M aqueous sodium hydrogencarbonate solution (50 ml) and extracted with tert-butyl methyl ether (2 X 50 ml). The organic phases are washed successively with water (1 X 50 ml) and brine (1 X 60 ml), dried with sodium sulphate and evaporated. The title compound is obtained from the residue by means of flash chromatography (SiO₂ 60F).

### General method E: (chlorination)

The solution of 40 mmol of "benzyl alcohol" in 6.40 ml of pyridine and 100 ml of dichloromethane is slowly added dropwise at 0 - 5°C to a precooled solution of 7.65 ml of thionyl chloride in 20 ml of dichloromethane. The reaction mixture is stirred for 1 hour each at 0°C and then at room temperature and subsequently poured onto 200 ml of ice water. The mixture is extracted with dichloromethane (2 X 200 ml). The organic phases are washed successively with 1 M aqueous sodium hydrogen-carbonate solution (2 X 200 ml) and brine, dried with sodium sulphate and evaporated. The title compound is obtained from the residue by means of flash chromatography (SiO₂ 60F).

### General method F: (phenol alkylation I)

The mixture of 20 mmol of "phenol" in 60 ml of N,N-dimethylformamide is stirred with 4.15 g of potassium carbonate and 30 mmol of "halide" or "tosylate" for 24 hours at 100°C. The reaction mixture is then evaporated. The residue is treated with 1 M aqueous sodium hydrogencarbonate solution (40 ml) and extracted with ethyl acetate (2 X 60 ml). The organic phases are washed with brine (1 X 60 ml), dried using sodium sulphate and evaporated. The title compound is obtained from the residue by means of flash chromatography (SiO₂ 60F).

### General method G: (phenol alkylation II)

A suspension of 1 mmol of "tosylate", 2 mmol of "phenol", 2 mmol of potassium carbonate and 20 ml of acetonitrile is stirred for 24 h at 90°C. The reaction mixture is then evaporated. The residue is treated with saturated aqueous sodium hydrogencarbonate solution and extracted with ethyl acetate (2X). The organic phases are washed with brine, dried using sodium sulphate and evaporated. The title compound is obtained from the residue by means of flash chromatography (SiO₂ 60F).

### General method H: (tosylation)

A solution of 12 mmol of p-toluenesulphonyl chloride in 15 ml of dichloromethane is added dropwise at 0°C to a solution of 10 mmol of "alcohol" or "amine", 15 mmol of triethylamine and 1 mmol of 4-dimethylaminopyridine in 90 ml of dichloromethane. The reaction mixture is stirred for 2-18 hours at room temperature. The reaction mixture is diluted with dichloromethane and subsequently washed with water and brine, dried using sodium sulphate and evaporated. The title compound is obtained from the residue by means of flash chromatography (SiO₂ 60F).

### General method I: (phenol alkylation III)

A suspension of 1 mmol of "phenol", 1.0-1.5 mmol of "tosylate" or "bromide", 1.5 mmol of caesium carbonate and 2 ml of acetonitrile is stirred for 2 hours at 80°C. The reaction mixture is cooled, poured onto water and extracted with ethyl acetate (2X). The organic phases are washed with brine, dried using sodium sulphate and evaporated. The title compound is obtained from the residue by means of flash chromatography (SiO₂ 60F).

### General method J (alcohol desilylation)

A solution of 1 mmol of "silyl ether" in 5 ml of tetrahydrofuran is treated with 1.5 - 2.0 mmol of tetrabutylammonium fluoride (1 M solution in tetrahydro-furan) and the solution is stirred for 1-2 hours at room temperature. The reaction solution is subsequently diluted with water and extracted 2X with tert-butyl methyl ether. The combined organic phases are dried using sodium sulphate and evaporated. The title compound is obtained from the residue by means of flash chromatography (SiO₂ 60F).

### General method K (borane reduction)

The solution of 1 mmol of "substrate" in 3 ml of tetrahydrofuran is treated with 3.0 - 6.0 mmol of borane-tetrahydrofuran complex (1 M in tetrahydrofuran) and stirred for 1 - 3 hours at room temperature (conversion check using HPLC or TLC). The reaction mixture is cooled to room temperature, treated with 3.0 - 6.0 mmol of methanol and evaporated. The title compound is obtained from the residue by means of flash chromatography (SiO₂ 6OF).

### General method L (N-Tos deprotection I)

0.44 mmol of sodium dihydrogenphosphate and 0.90 mmol of sodium amalgam (10% Na) are added successively at room temperature to a solution of 0.09 mmol of "tosyl amide" in 10 ml of methanol. The reaction mixture is stirred for 2-18 hours, diluted with water and extracted with ethyl acetate. The organic phase is separated off and washed with brine, dried using sodium sulphate and evaporated. The title compound is obtained from the residue by means of flash chromatography (SiO₂ 60F).

### General method M (O-alkylation II)

A solution of 1 mmol of "secondary alcohol" in 5 ml of tetrahydrofuran is treated at room temperature with 1 mmol of methylmagnesium bromide (35% solution in diethyl ether). The reaction solution is heated to reflux for 5 minutes and subsequently treated with a solution of 2.2 mmol of "oxirane" in 1 ml of THF. The reaction mixture is heated to reflux for 1-5 hours, poured onto saturated, aqueous sodium hydrogencarbonate solution and the mixture is extracted with tert-butyl methyl ether. The combined organic phases are dried over sodium sulphate and evaporated. The title compound is obtained from the residue by means of flash chromatography (SiO₂ 60F).

### General method N (N-Tos deprotection II)

0.5 ml of a blue-green sodium naphthalenide stock solution (of 0.04 g of sodium and 0.22 g of naphthalene in 5 ml of dimethoxyethane) are added at -60°C to a solution of 0.1 mmol of "tosylamide" in 2 ml of dimethoxyethane. After 3-6 hours, the reaction mixture is diluted with water and extracted with dichloromethane (2X). The combined organic phases are washed successively mit brine, dried using sodium sulphate and evaporated. The title compound is obtained from the residue by means of flash chromatography (SiO₂ 60F).

### General method O (N-Cbz deprotection)

The solution of 1 mmol of "N-Cbz derivative" in 15 ml of tetrahydrofuran is hydrogenated in the presence of 100-200 mg of Pd/C 10% for 2-20 hours at 15 - 20°C. The reaction mixture is subjected to clarifying filtration and the filtrate is evaporated. The title compound is obtained from the residue by means of flash chromatography (SiO₂ 60F).

### General method P (alcohol nitration)

A well-stirred suspension of 1.25 mmol of nitronium tetrafluoroborate in 2 ml of acetonitrile under an argon atmosphere is slowly treated at -10 - 0°C with a solution of 1.3 mmol of 2,4,6-trimethylpyridine in 1 ml of acetonitrile. The mixture is vigorously stirred for 30 minutes at 0°C and subsequently a solution of 1 mmol of "alcohol" in 2 ml of acetonitrile is slowly added. The mixture is stirred vigorously for 2 hours, subsequently poured onto ice water and extracted with diethyl ether (4X). The combined organic phases are dried over magnesium sulphate and evaporated. The title compound is obtained from the residue by means of flash chromatography (SiO₂ F60).

### General method Q (N-Boc deprotection II)

15 mmol of trifluoroacetic acid are added dropwise at 0°C to a solution of 1 mmol of "N-Boc derivative" in 10 ml of dichloromethane. The reaction mixture is stirred for 2 hours at room temperature. Subsequently, it is poured onto 15 ml of ice-cooled, saturated sodium hydrogencarbonate solution and the mixture is extracted three times with 30 ml each of ethyl acetate. The combined organic phases are dried using sodium sulphate and evaporated. The title compound is obtained from the residue by means of flash chromatography (SiO₂ F60).

### General method R (N-Boc protection)

1.15 mmol of di-tert-butyl dicarbonate are added to a solution of 1 mmol of "amine" and 1.2 mmol of triethylamine in 12 ml of dichloromethane. The reaction mixture is stirred for 14 hours at room temperature. Subsequently, it is diluted with 20 ml of dichloro-methane and the organic phase is extracted successively with 10 ml of 0.2N HCl, 10 ml of saturated sodium hydrogencarbonate solution and 10 ml of brine. The organic phase is dried using sodium sulphate and evaporated. The title compound is obtained from the residue by means of flash chromatography (SiO₂ F60).

### General method S (mesylate nitration)

1.6 mmol of tetrabutylammonium nitrate are added to a solution of 1 mmol of "mesylate" in 5 ml of toluene. The reaction mixture is stirred for 18 hours at 110°C. Subsequently, it is filtered through silica gel (eluent EtOAc-heptane 1:1 ) and the resulting solution is evaporated.

### General method T (alcohol mesylation)

1.2 mmol of methanesulphonyl chloride are added dropwise at room temperature to a solution of 1 mmol of "alcohol" and 3.5 mmol of triethylamine in 8 ml of dichloro-methane. The reaction mixture is stirred for 2 hours at room temperature. Subsequently, it is diluted with dichloromethane and the organic phase is washed successively with water, 1 M aqueous citric acid solution and saturated sodium hydrogencarbonate solution. The organic phase is dried using sodium sulphate and evaporated. The residue is employed in the next stage without further purification.

### General method U (amide coupling)

5.0 mmol of triethylamine and 1.0 mmol of tri-propylphosphonic cyclic anhydride [68957-94-8] (50% in ethyl acetate) are added at room temperature to a solution of 1.0 mmol of "acid" and 1.0 mmol of "amine" in 20 ml of dichloromethane. The reaction mixture is stirred for 1-3 hours, diluted with dichloromethane and washed with 1 N HCl and brine. The combined organic phases are dried using sodium sulphate and evaporated. The title compound is obtained from the residue by means of flash chromatography (SiO₂ F60).

### General method V (N-Cbz protection)

A solution of 1 mmol of "amine" in 6 ml of ethyl acetate is treated with 6 ml of saturated sodium hydrogencarbonate solution. The two-phase mixture is cooled to 0°C, slowly treated with 1.01 mmol of benzyl chloroformate and subsequently stirred for 2 hours. The reaction mixture is extracted with ethyl acetate-tetrahydrofuran. The organic phases are evaporated and the title compound is obtained from the residue by means of flash chromatography (SiO₂ F60).

### General method W: (O-alkylation II)

The solution of 1 mmol of "alcohol" in 2.0 ml of N,N-dimethylformamide is treated at 0°C with stirring with 2.2 mmol of sodium hydride (60% dispersion in oil) and stirred for 1 hour. Subsequently, the mixture is cooled to -5°C and 2 mmol of (R)-3-triisopropyl-silanyloxy-butyl toluene-4-sulphonate are added. The reaction mixture is stirred for 3 hours at 60°C and then cooled to room temperature. Subsequently, the mixture is diluted with tert-butyl methyl ether and poured onto ice water. The resulting mixture is extracted three times with tert-butyl methyl ether. The combined organic phases are washed with brine, dried using sodium sulphate and evaporated. The title compound is obtained from the residue by means of flash chromatography (SiO₂ 60F).

### General method X: (pentafluorophenyl ester coupling)

A solution of 1 mmol of "pentafluorophenyl ester" in 3 ml of N,N-dimethylformamide is added under a nitrogen atmosphere at 0°C with stirring to a solution of 1 mmol of "alcohol" or "amine" and 1 mmol of triethylamine in 15 ml of N,N-dimethylformamide. The reaction mixture is stirred at room temperature until the reaction is complete according to thin layer chromatography. It is poured onto pH 3 buffer solution, brought to pH 1 using 1 M HCl and extracted with dichloromethane (2X). The organic phase is dried using sodium sulphate and evaporated. The title compound is obtained from the residue by means of flash chromatography (SiO₂ F60).

### General method Y: (halide nitration)

A mixture of 1 mmol of "halide" and 2.58 mmol of silver nitrate in 5 ml of acetonitrile is heated to 70°C for 20 minutes in the microwave. Subsequently, it is filtered through Hyflo and the resulting solution is evaporated. The title compound is obtained from the residue by means of flash chromatography (SiO₂ F60).

### General method Z: (carbonate formation/carbamate formation)

A solution of 1 mmol of "p-nitrophenyl carbonate" in 3 ml of N,N-dimethylformamide is added with stirring at 0°C under a nitrogen atmosphere to a solution of 1 mmol of "alcohol" or "amine" and 1 mmol of triethylamine in 15 ml of N,N-dimethylformamide. The reaction mixture is stirred at room temperature until the reaction is complete according to thin layer chromatography. It is poured onto pH 3 buffer solution, brought to pH 1 using 1 M HCl and extracted with dichloromethane (2X). The organic phase is dried using sodium sulphate and evaporated. The title compound is obtained from the residue by means of flash chromatography (SiO₂ F60).

### General method AA: (4-nitrophenyl carbonate formation)

1 mmol of bis(4-nitrophenyl) carbonate [5070-13-3] and 3 mmol of N,N-dimethylaminopyridine in 15 ml of dichloromethane are added at room temperature under a nitrogen atmosphere to a solution of 1 mmol of "alcohol", and stirred until the reaction is complete according to thin layer chromatography. The mixture is poured onto pH 3 buffer solution, brought to pH 1 using 1 M HCl and extracted with dichloromethane (2X). The organic phase is dried using sodium sulphate and evaporated. The title compound is obtained from the residue by means of flash chromatography (SiO₂ F60).

### Example 1

### 6-[(3R,4R,5S)-4-(4-Methoxyphenyl)-5-nitrooxypiperidin-3-yloxymethyl]-4-(3-methoxypropyl)-3,4-dihydro-2H-benzo[1,4]oxazine

Analogously to method Q, starting from tert-butyl (3R,4R,5S)-4-(4-methoxyphenyl)-3-[4-(3-methoxypropyl)-3,4-dihydro-2H-benzo[1,4]oxazin-6-ylmethoxy]-5-nitro-oxypiperidine-1-carboxylate the title compound is identified by means of the Rf value.

The starting materials are prepared as follows:

### a) tert-Butyl (3R,4R,5S)-4-(4-methoxyphenyl)-3-[4-(3-methoxypropyl)-3,4-dihydro-2H-benzo[1,4]oxazin-6-ylmethoxy]-5-nitrooxypiperidine-1-carboxylate

Analogously to method P, starting from tert-butyl (3S,4S,5R)-3-hydroxy-4-(4-methoxyphenyl)-5-[4-(3-methoxypropyl)-3,4-dihydro-2H-benzo[1,4]oxazin-6-yl-methoxy]piperidine-1-carboxylate the title compound is identified by means of the Rf value.

### b) tert-Butyl (3S,4S,5R)-3-hydroxy-4-(4-methoxy-phenyl)-5-[4-(3-methoxypropyl)-3,4-dihydro-2H-benzo[1,4]oxazin-6-ylmethoxy]piperidine-1-carboxylate

Analogously to method R, starting from (3S,4S,5R)-4-(4-methoxyphenyl)-5-[4-(3-methoxypropyl)-3,4-dihydro-2H-benzo[1,4]oxazin-6-ylmethoxy]piperidin-3-ol [873945-55-2] the title compound is identified by means of the Rf value.

According to the process described in Example 1, the following compounds are prepared in an analogous manner:

### Examples

### 9 6-{(3R,4R,5S)-4-[4-((S)-3-Methoxy-2-methylproproxy-methyl)phenyl]-5-nitrooxypiperidin-3-yloxy-methyl}-4-(3-methoxypropyl)-3,4-dihydro-2H-benzo-[1,4]oxazine

starting from (3S,4S,5R)-4-[4-((S)-3-methoxy-2-methyl-propoxymethyl)phenyl]-5-[4-(3-methoxypropyl)-3,4-dihydro-2H-benzo[1,4]oxazin-6-ylmethoxy]piperidin-3-ol [911854-43-8].

### 29 6-((3R,4R,5S)-4-{4-[(S)-1-(3-Fluorophenyl)-pyrrolidin-3-yloxy]phenyl}-5-nitrooxypiperidin-3-yloxymethyl)-4-(3-methoxypropyl)-3,4-dihydro-2H-benzo[1,4]oxazine

starting from (3S,4S,5R)-4-{4-[(S)-1-(3-fluorophenyl)-pyrrolidin-3-yloxy]phenyl}-5-[4-(3-methoxypropyl)-3,4-dihydro-2H-benzo[1,4]oxazin-6-ylmethoxy]piperidin-3-ol [873945-18-7].

### Example 2

### 6-[(3R,4R,5S)-4-(4-Methoxyphenyl)-5-((S)-2-nitrooxy-propoxy)piperidin-3-yloxymethyl]-4-(3-methoxypropyl)-3,4-dihydro-2H-benzo[1,4]oxazine

Analogously to method Q, starting from tert-butyl (3R,4R,5S)-4-(4-methoxyphenyl)-3-[4-(3-methoxypropyl)-3,4-dihydro-2H-benzo[1,4]oxazin-6-ylmethoxy]-5-((S)-2-nitrooxypropoxy)piperidine-1-carboxylate the title compound is identified by means of the Rf value.

The starting materials are prepared as follows:

### a) tert-Butyl (3R,4R.5S)-4-(4-methoxyphenyl)-3-[4-(3-methoxypropyl)-3,4-dihydro 2H-benzo[1,4]oxazin-6-ylmethoxy]-5-((S)-2-nitrooxypropoxy)piperidine-1-carboxylate

Analogously to method S, starting from tert-butyl (3S,4R,5R)-3-((R)-2-methanesulphonyloxypropoxy)-4-(4-methoxyphenyl)-5-[4-(3-methoxypropyl)-3,4-dihydro-2H-benzo[1,4]oxazin-6-ylmethoxy]piperidine-1-carboxylate the title compound is identified by means of the Rf value.

### b) tert-Butyl (3S,4R,5R)-3-((R)-2-methanesulphonyl-oxypropoxy)-4-(4-methoxyphenyl)-5-[4-(3-methoxy-propyl)-3,4-dihydro-2H-benzo[1,4]oxazin-6-yl-methoxy]piperidine-1-carboxylate

Analogously to method T, starting from tert-butyl (3S,4R,5R)-3-((R)-2-hydroxypropoxy)-4-(4-methoxy-phenyl)-5-{4-(3-methoxypropyl)-3,4-dihydro-2H-benzo- [1,4]oxazin-6-ylmethoxy]piperidine-1-carboxylate the title compound is identified by means of the Rf value.

### c) tert-Butyl (3S,4R,5R)-3-((R)-2-hydroxypropoxy)-4-(4-methoxyphenyl)-5-[4-(3-methoxypropyl)-3,4-dihydro-2H-benzo[1,4]oxazin-6-ylmethoxy]-piperidine-1-carboxylate

Analogously to method R, starting from (R)-1-{(3S,4R,5R)-4-(4-methoxyphenyl)-5-[4-(3-methoxypropyl)-3,4-dihydro-2H-benzo[1,4]oxazin-6-ylmethoxy]piperidin-3-yloxy}propan-2-ol the title compound is identified by means of the Rf value.

### d) (R)-1-{(3S,4R,5R)-4-(4-Methoxyphenyl)-5-[4-(3-methoxypropyl)-3,4-dihydro-2H-benzo[1,4]oxazin-6-ylmethoxy]piperidin-3-yloxy}propan-2-ol

Analogously to method O, starting from benzyl (3R,4R,5S)-4-(4-methoxyphenyl)-3-[4-(3-methoxy-propyl)-3,4-dihydro-2H-benzo[1,4]oxazin-6-yl-methoxy]-5-((R)-1-oxiranyl-methoxy)piperidine-1-carboxylate the title compound is identified by means of the Rf value.

### e) Benzyl (3R,4R,5S)-4-(4-methoxyphenyl)-3-[4-(3-methoxypropyl)-3.4-dihydro-2H-benzo[1,4]oxazin-6-ylmethoxy]-5-((R)-1-oxiranylmethoxy)piperidine-1-carboxylate

A solution of 0.493 mmol of benzyl ((3S,4S,5R)-3-hydroxy-4-(4-methoxyphenyl)-5-[4-(3-methoxypropyl)-3,4-dihydro-2H-benzo[1,4]oxazin-6-ylmethoxy]piperidine-1-carboxylate [873945-56-3] in 2 ml of tetrahydrofuran is treated with 0.789 mmol of sodium hydride (60% dispersion in oil). The mixture is stirred for 45 minutes at 40°C. A solution of 0.986 mmol of (R)-1-oxiranymethyl toluene-4-sulphonate [113826-06-5] in 1.5 ml of tetrahydrofuran is added and the reaction mixture is warmed to 50°C for 3 hours. The reaction mixture is poured onto saturated aqueous sodium hydrogen-carbonate solution and the mixture is extracted with tert-butyl methyl ether (3X). The combined organic extracts are washed with brine, dried using sodium sulphate and evaporated. The title compound is identified by means of the Rf value from the residue by means of flash chromatography (SiO₂ 60F).

According to the process described in Example 2, the following compounds are prepared in an analogous manner:

### Examples

### 6 6-[(3R,4R,5S)-4-[4-(2-Methoxyethoxymethyl)phenyl]-5-((R)-2-nitrooxy-propoxy)piperidin-3-yloxymethyl]-4-(3-methoxypropyl)-3,4-dihydro-2H-benzo[1,4]-oxazine

starting from benzyl (3S,4S,5R)-3-hydroxy-4-[4-(2-methoxyethoxymethyl)phenyl]-5-[4-(3-methoxypropyl)-3,4-dihydro-2H-benzo[1,4]oxazin-6-ylmethoxy]piperidine-1-carboxylate [912346-67-9] using (S)-1-oxiranymethyl toluene-4-sulphonate [70987-78-9].

### 7 6-[(3R,4R,5S)-4-[4-(4-Ethylphenoxy)phenyl]-5-((R)-2-nitrooxypropoxy)piperidin-3-yloxymethyl]-4-(3-methoxypropyl)-3,4-dihydro-2H-benzo[1,4]oxazine

starting from benzyl (3S,4S,5R)-4-[4-(4-ethylphenoxy)-phenyl]-3-hydroxy-5-[4-(3-methoxypropyl)-3,4-dihydro-2H-benzo-[1,4]oxazin-6-ytmethoxy]piperidine-1-carboxylate using (S)-1-oxiranylmethyl toluene-4-sulphonate [70987-78-9].

The starting materials are prepared as follows:

### a) Benzyl (3S,4S,5R)-4-[4-(4-ethylphenoxy)phenyl-3-hydroxy-5-[4-(3-methoxypropyl)-3,4-dihydro-2H-benzo[1,4]oxazin-6-ylmethoxy]piperidine-1-carboxylate

Analogously to method J, starting from benzyl (3R,4R,5S)-4-[4-(4-ethyl-phenoxy)phenyl]-3-[4-(3-methoxypropyl)-3,4-dihydro-2H-benzo[1,4]oxazin-6-yl-methoxy]-5-triisopropylsilanyloxypiperidine-1-carboxylate the title compound is obtained as a yellow oil. Rf (EtOAc-heptane 1:1)= 0.19; Rt = 5.73 (gradient I).

### b) Benzyl (3R,4R,5S)-4-[4-(4-ethylphenoxy)phenyl]-3-[4-(3-methoxypropyl)-3,4-dihydro-2H-benzo[1,4]-oxazin-6-ylmethoxy]-5-triisopropylsilanyloxy-piperidine-1-carboxylate

A solution of 1 mmol of benzyl (3R,4R,5S)-4-(4-hydroxyphenyl)-3-[4-(3-methoxypropyl)-3,4-dihydro-2H-benzo[1,4]oxazin-6-ylmethoxy]-5-triisopropylsilanyloxy-piperidine-1-carboxylate [912346-44-2], 3 mmol of 4-ethylphenylboronic acid [63139-21-9], 2.2 mmol of copper(II) acetate, 5 mmol of triethylamine and 700 mg of molecular sieve (4A, powder) in 10 ml of anhydrous dichloromethane is stirred at room temperature for 24 hours. The reaction mixture is subjected to clarifying filtration and the filtrate is evaporated. The title compound is obtained from the residue by means of flash chromatography (SiO₂ 60F) as a yellow oil. Rf (EtOAc-heptane 1:2) = 0.34; Rt = 5.78 (gradient I).

### 11 6-[(3R,4R,5S)-4-[4-(4-Methoxybutoxy)phenyl]-5-((S)-2-nitrooxy-propoxy)piperidin-3-yloxymethyl]-4-(3-methoxypropyl)-3,4-dihydro-2H-benzo[1,4]oxazine

starting from benzyl (3S,4S,5R)-3-hydroxy-4-[4-(4-methoxybutoxy)phenyl]-5-[4-(3-methoxypropyl)-3,4-dihydro-2H-benzo[1,4]oxazin-6-ylmethoxy]piperidine-1-carboxylate.

The starting materials are prepared as follows:

### a) Benzyl (3S,4S,5R)-3-hydroxy-4-[4-(4-methoxy-butoxy)phenyl]-5-[4-(3-methoxypropyl)-3,4-dihydro-2H-benzo[1.4]oxazin-6-ylmethoxy]piperidine-1-carboxylate

A solution of 1.379 mmol of benzyl (3R,4R,5S)-4-[4-(4-methoxybutoxy)phenyl]-3-[4-(3-methoxypropyl)-3,4-dihydro-2H-benzo[1,4]oxazin-6-ylmethoxy]-5-triiso-propyl-silanyloxypiperidine-1-carboxylate in 10 ml of tetrahydrofuran is treated at room temperature with 2.758 mmol of tetrabutylammonium fluoride trihydrate. After 1 hour, the reaction mixture is treated with 0.5 ml of water and the mixture is evaporated to dryness. The title compound is obtained from the residue as a turbid white oil by means of flash chromatography (SiO₂ 60F). Rf = 0.60 (EtOAc); Rt = 4.94 (gradient I).

### b) Benzyl (3R,4R,5S)-4-[4-(4-methoxybutoxy)phenyl]-3-[4-(3-methoxypropyl)-3,4 dihydro-2H-benzo[1,4]-oxazin-6-ylmethoxy]-5-triisopropylsilanyloxy-piperidine-1-carboxylate

Analogously to method K, starting from benzyl (3R,4R,5S)-4-[4-(4-methoxy-butoxy)phenyl]-3-[4-(3-methoxypropyl)-3-oxo-3,4-dihydro-2H-benzo[1,4]oxazin-6-ylmethoxy]-5-triisopropylsilanyloxypiperidine-1-carboxylate the title compound is obtained as a colourless oil. Rf = 0.19 (EtOAc-heptane 1:1).

### c) Benzyl (3R,4R,5S)-4-[4-(4-methoxybutoxy)phenyl]-3-[4-(3-methoxypropyl)-3-oxo-3,4-dihydro-2H-benzo[1,4]oxazin-6-ylmethoxy]-5-triisopropyl-silanyloxypiperidine-1-carboxylate

Analogously to method D, starting from benzyl (3R,4R,5S)-3-hydroxy-4-[4-(4-methoxybutoxy)phenyl]-5-triisopropylsilanyloxypiperidine-1-carboxylate and 6-chloromethyl-4-(3-methoxypropyl)-4H-benzo[1,4]oxazin-3-one [857272-02-7] the title compound is obtained as a yellowish oil. Rt = 7.24 (gradient I).

### d) Benzyl (3R,4R,5S)-3-hydroxy-4-[4-(4-methoxy-butoxy)phenyl]-5-triiso-propylsilanyloxy-piperidine-1-carboxylate

Analogously to method F, starting from benzyl (3R,4R,5S)-3-hydroxy-4-[4-hydroxyphenyl]-5-triiso-propylsilanyloxypiperidine-1-carboxylate [873945-27-8] and 1-bromo-4-methoxybutane [4457-67-4] the title compound is obtained as a yellow oil. Rt = 6.63 (gradient I).

### 17 (3R,4R,5S)-4-[4-(2-Methoxyethoxymethyl)phenyl]-3-[4-(3-methoxypropyl)-2,2-dimethyl-2H-chromen-6-ylmethoxy]-5-((S)-2-nitrooxypropoxy)piperidine

starting from benzyl (3S,4S,5R)-3-hydroxy-4-[4-(2-methoxyethoxymethyl)phenyl]-5-[4-(3-methoxypropyl)-2,2-dimethyl-2H-chromen-6-ylmethoxy]piperidine-1-carboxylate.

The starting material is prepared as follows:

### a) Benzyl (3S,4S,5R)-3-hydroxy-4-[4-(2-methoxy-ethoxymethyl)phenyl]-5-[4-(3-methoxypropyl)-2,2-dimethyl-2H-chromen-6-ylmethoxy]piperidine-1-carboxylate

Analogously to Example 91, starting from (3S,4S,5R)-4-[4-(2-methoxyethoxymethyl)phenyl]-5-[4-(3-methoxy-propyl)-2,2-dimethyl-2H-chromen-6-ylmethoxy]piperidin-3-ol [911855-53-3] the title compound is identified by means of the Rf value.

### 23 (3R,4R,5S)-4-{4-[3-(2-Methoxybenzyloxy)propoxy]-phenyl}-3-[4-methoxy-3-(3-methoxypropoxy)benzyl-oxy]-5-((S)-2-nitrooxypropoxy)piperidine

starting from benzyl (3S,4S,5R)-3-hydroxy-4-{4-[3-(2-methoxybenzyloxy)propoxy]phenyl}-5-[4-methoxy-3-(3-methoxypropoxy)benzyloxy]piperidine-1-carboxylate.

The starting materials are prepared as follows:

### a) Benzyl (3S,4S,5R)-3-hydroxy-4-{4-[3-{2-methoxy-benzyloxy)propoxy]pheny}-5-[4-methoxy-3-(3-methoxyproxy)benzyloxyl]piperidine-1-carboxylate

Analogously to method J, starting from benzyl (3R,4R,5S)-4-{4-[3-(2-methoxy-benzyloxy)propoxy]phenyl}-3-[4-methoxy-3-(3-methoxypropoxy)benzyloxy]-5-triiso-propylsilanyloxypiperidine-1-carboxylate the title compound is identified by means of the Rf value.

### b) Benzyl (3R,4R,5S)-4-{4-[3-(2-methoxybenzyloxy)-propoxy]phenyl}-3-[4-methoxy-3-(3-methoxy-propoxy)benzyloxy]-5-triisoipropylsilanyl-oxypiperidine-1-carboxylate

Analogously to method D, starting from benzyl (3R,4R,5S)-3-hydroxy-4-{4-[3-(2-methoxybenzyloxy)-propoxy]phenyl}-5-triisopropylsilanyloxypiperidine-1-carboxylate [873945-54-1] and 4-bromomethyl-1-methoxy-2-(3-methoxypropoxy)benzene [172900-73-1 ] the title compound is identified by means of the Rf value.

### 24 N-[2-(2-{2-[(3R,4R,5S)-4-{4-[3-(2-Methoxy-benzyloxy)propoxy]phenyl}-5-((R)-2-nitro-oxypropoxy)piperidin-3-yloxy]ethoxy}phenyl)ethyl]-acetamide

starting from benzyl (3R,4S,5S)-3-{2-[2-(2-acetylamino-ethyl)phenoxy]ethoxy}-5-hydroxy-4-{4-[3-(2-methoxy-benzyloxy)propoxy]phenyl}piperidine-1-carboxylate using (S)-I-oxiranymethyl toluene-4-sulphonate [70987-78-9].

The starting materials are prepared as follows:

### a) Benzyl (3R,4S,5S)-3-{2-[2-(2-acetylaminoethyl)-phenoxy]ethoxy}-5-hydroxy-4-{4-[3-(2-methoxy-benzyloxy)propoxy]phenyl}piperidine-1-carboxylate

Analogously to method J, starting from benzyl (3R,4R,5S)-3-{2-[2-(2-acetylaminoethyl)phenoxy]ethoxy}-4-{4-[3-(2-methoxybenzyloxy)propoxy]phenyl}-5-triiso-propylsilanyloxypiperidine-1-carboxylate the title compound is identified by means of the Rf value.

### b) Benzyl (3R,4R,5S)-3-{2-[2-(2-acetylaminoethyl)-phenoxy]ethoxy}-4-{4-[3-(2-methoxybenzyloxy)-propoxy]phenyl}-5-triisopropylsilanyloxy-piperidine-1-carboxylate

Analogously to method G, starting from benzyl (3R,4R,5S)-4-{4-[3-(2-methoxybenzyloxy)propoxy]phenyl}-3-[2-(toluene-4-sulphonyloxy)ethoxy]-5-triisopropyl-silanyloxypiperidine-1-carboxylate and N-[2-(2-hydroxyphenyl)ethyl]acetamide the title compound is identified by means of the Rf value.

### c) Benzyl (3R,4R,5S)-4-{4-[3-(2-Methoxybenzyloxy)-propoxy]phenyl}-3-[2-(toluene-4-sulphonyloxy)-ethoxy]-5-triisopropylsilanyloxypiperidine-1-carboxylate

Analogously to method H, starting from benzyl (3R,4R,5S)-3-(2-hydroxyethoxy)-4-{4-[3-(2-methoxy-benzyloxy)propoxy]phenyl}-5-triisopropylsilanyloxy-piperidine-1-carboxylate the title compound is identified by means of the Rf value.

### d) Benzyl (3R,4R,5S)-3-(2-hydroxyethoxy)-4-{4-[3-(2-methoxybenzyloxy)propoxy]phenyl}-5-triisopropyl-silanyloxypiperidine-1-carboxylate

1 mmol of benzyl (3R,4R,5S)-3-allyloxy-4-{4-[3-(2-methoxybenzyloxy)propoxy]phenyl}-5-triisopropylsilanyl-oxypiperidine-1-carboxylate is initially introduced with stirring at room temperature in the mixture tetrahydrofuran/water 3:1 (4 ml) and treated with 0.01 mmol of osmium tetroxide (2.5 wt% in tert-BuOH). Subsequently, 2.54 mmol of sodium periodate are added in portions. The reaction mixture is stirred further for 2 hours. The reaction mixture is evaporated. The residue is suspended in a mixture of dichloro-methane/methanol 1:1 (12 ml), cooled to 5°C and treated in portions with 6.08 mmol of sodium borohydride. The reaction mixture is stirred at 5°C for 1.5 hours and subsequently evaporated. The residue is stirred in dichloromethane, filtered through a glass fibre filter and washed with dichloromethane. The filtrate is washed with a mixture of brine/water 1:1, filtered through glass wool, dried using sodium sulphate and evaporated. The title compound is identified by means of the Rf value from the residue by means of flash chromatography (SiO₂ 60F).

### e) Benzyl (3R,4R,5S)-3-allyloxy-4-{4-[3-(2-methoxy-benzyloxy)propoxy]phenyl}-5-triisopropylsilanyl-oxypiperidine-1-carboxylate

Analogously to method D, starting from benzyl (3R,4R,5S)-3-hydroxy-4-{4-[3-(2-methoxybenzyloxy)-propoxy]phenyl}-5-triisopropylsilanyloxypiperidine-1-carboxylate [873945-54-1] and allyl bromide the title compound is identified by means of the Rf value.

### 25 6-[(3R,4R,5S)-4-{4-[3-(2-Methoxybenzyloxy)-propoxy]phenyl}-5-((S)-2-nitrooxy-propoxy)-piperidin-3-yloxymethyl-1-(3-methoxyprol)-3,3-dimethyl-2,3-dihydro-1 H-indole

starting from benzyl (3S,4S,5R)-3-hydroxy-4-{4-[3-(2-methoxybenzyloxy)propoxy]phenyl)-5-[1-(3-methoxy-propyl)-3,3-dimethyl-2,3-dihydro-1H-indol-6-ylmethoxy]-piperidine-1 - carboxylate.

The starting materials are prepared as follows:

### a) Benzyl (3S,4S,5R)-3-hydroxy-4-{4-[3-(2-methoxy-benzyloxy)propoxy]pheny1}-5-[1-(3-methoxypropyl)-3,3-dimethyl-2,3-dihydro-1H-indol-6-ylmethoxyl-piperidine-1-carboxylate

Analogously to method J, starting from benzyl (3R,4R,5S)-4-{4-[3-(2-methoxybenzyloxy)propoxy]phenyl}-3-[1-(3-methoxypropyl)-3,3-dimethyl-2,3-dihydro-1H-indol-6-ylmethoxy]-5-triisopropylsilanyloxypiperidine-1-carboxylate the title compound is identified by means of the Rf value.

### b) Benzyl (3R,4R,5S)-4-{4-[3-(2-methoxybenzyloxy)-propoxy]phenyl}-3-[1-(3-methoxypropyl)-3,3-dimethyl-2,3-dihydro-1H-indol-6-ylmethoxyl-5-triisopropylsilanyloxypiperidine-1-carboxylate

Analogously to method D, starting from benzyl (3R,4R,5S)-3-hydroxy-4-{4-[3-(2-methoxybenzyloxy)-propoxy]phenyl}-5-triisopropylsilanyloxypiperidine-1-carboxylate [873945-54-1] and 6-bromomethyl-1-(3-methoxypropyl)-3,3-dimethyl-1,3-dihydroindol-2-one [857274-09-0] the title compound is identified by means of the Rf value.

### 27 6-[(3R,4R,5S)-4-[4-(2-Methoxyethoxymethyl)-phenyl]-5-((S)-2-nitrooxy-propoxy)piperidin-3-yloxymethyl]-4-(3-methoxypropyl)-3,4-dihydro-2H-benzo[1,4]oxazine

starting from benzyl (3S,4S,5R)-3-hydroxy-4-[4-(2-methoxyethoxymethyl)phenyl]-5-[4-(3-methoxypropyl)-3,4-dihydro-2H-benzo[1,4]oxazin-6-ylmethoxy]piperidine-1-carboxylate [912346-67-9].

### 30 6-[(3R,4R,5S)-4-[4-(4-Methoxybutoxy)phenyl]-5-((R)-2-nitrooxy-propoxy)piperidin-3-yloxymethyl]-4-(3-methoxypropyl)-3,4-dihydro-2H-benzo[1,4]oxazine

starting from benzyl (3S,4S,5R)-3-hydroxy-4-[4-(4-methoxybutoxy)phenyl]-5-[4-(3-methoxypropyl)-3,4-dihydro-2H-benzo[1,4]oxazin-6-ylmethoxy]piperidine-1-carboxylate (Example 11 a) using (S)-I-oxiranymethyl toluene-4-sulphonate [70987-78-9] in stage e.

### 31 6-[(3R,4R,5S)-4-(4-Methoxyphenyl)-5-((R)-2-nitrooxypropoxy)piperidin-3-yloxymethyl]-4-(3-methoxypropyl)-3,4-dihydro-2H-benzo[1,4]oxazine

starting from benzyl (3S,4S,5R)-3-hydroxy-4-(4-methoxy-phenyl)-5-[4-(3-methoxypropyl)-3,4-dihydro-2H-benzo-[1,4]oxazin-6-ylmethoxy]piperidine-1-carboxylate [873945-56-3] using (S)-1-oxiranymethyl toluene-4-sulphonate [70987-78-9] in stage e.

### Example 3

### 6-[(3R,4R,5S)-4-(4-Methoxyphenyl)-5-nitrooxymethyl-piperidin-3-yloxymethyl]-4-(3-methoxypropyl)-3,4-dihydro-2H-benzo[1,4]oxazine

Analogously to method Q, starting from tert-butyl (3R,4R,5S)-4-(4-methoxyphenyl)-3-[4-(3-methoxypropyl)-3,4-dihydro-2H-benzo[1,4]oxazin-6-ylmethoxy]-5-nitrooxy-methylpiperidine-1-carboxylate the title compound is identified by means of the Rf value.

The starting materials are prepared as follows:

### a) tert-Butyl (3R,4R,5S)-4-(4-methoxyphenyl)-3-[4-(3-methoxypropyl)-3,4-dihydro-2H-benzo[1,41oxazin-6-ylmethoxy]-5-nitrooxymethylpiperidine-1-carboxylate

Analogously to method S, starting from tert-butyl (3S,4R,5R)-3-methane-sulphonyloxymethyl-4-(4-methoxy-phenyl)-5-[4-(3-methoxypropyl)-3,4-dihydro-2H-benzo- [1,4]oxazin-6-ylmethoxy]piperidine-1-carboxylate the title compound is identified by means of the Rf value.

### b) tert-Butyl (3S,4R,5R)-3-methanesulphonyloxy-methyl-4-(4-methoxyphenyl)-5-[4-(3-methoxypropyl)-3,4-dihydro-2H-benzo[1,4]oxazin-6-ylmethoxy]-piperidine-1-carboxylate

Analogously to method T, starting from tert-butyl (3S,4R,5R)-3-hydroxymethyl-4-(4-methoxyphenyl)-5-[4-(3-methoxypropyl)-3,4-dihydro-2H-benzo[1,4]oxazin-6-ylmethoxy]piperidine-1-carboxylate the title compound is identified by means of the Rf value.

### c) tert-Butyl (35,4R,5R)-3-hydroxymethyl-4-(4-methoxyphenyl)-5-[4-(3-methoxypropyl)-3,4-dihydro-2H-benzo[1,4]oxazin-6-ylmethoxy]piperidine-1-carboxylate

Analogously to method R, starting from {(3S,4R,5R)-4-(4-methoxyphenyl)-5-[4-(3-methoxypropyl)-3,4-dihydro-2H-benzo[1,4]oxazin-6-ylmethoxy]piperidin-3-yl}-methanol the title compound is identified by means of the Rf value.

### d) {(3S,4R,5R)-4-(4-Methoxyphenyl)-5-[4-(3-methoxy-propyl)-3,4-dihydro-2H-benzo[1,4]oxazin-6-yl-methoxy]ipiperidin-3-yl}methanol

Analogously to method B, starting from benzyl (3S,4R,5R)-3-hydroxymethyl-4-(4-methoxyphenyl)-5-[4-(3-methoxypropyl)-3,4-dihydro-2H-benzo[1,4]oxazin-6-yl-methoxy]piperidine-1-carboxylate the title compound is identified by means of the Rf value.

### e) Benzyl (3S,4R,5R)-3-hydroxymethyl-4-(4-methoxy-phenyl)-5-[4-(3-methoxypropyl)-3,4-dihydro-2H-benzo[1,4]oxazin-6-vlmethoxy]piperidine-1 - carboxylate

Analogously to method K, starting from benzyl (3S,4R,5R)-3-hydroxymethyl-4-(4-methoxyphenyl)-5-[4-(3-methoxypropyl)-3-oxo-3,4-dihydro-2H-benzo[1,4]oxazin-6-ylmethoxy]piperidine-1-carboxylate the title compound is obtained as a yellow oil. Rf = 0.18 (EtOAc-heptane = 2:1); Rt = 4.79 (gradient I).

### f) Benzyl (3S,4R,5R)-3-hydroxymethyl-4-(4-methoxy-phenyl)-5-[4-(3-methoxypropyl)-3-oxo-3,4-dihydro-2H-benzo[1,4]oxazin-6-ylmethoxy]piperidine-1-carboxylate

A solution of 1.919 mmol of benzyl (3R,4R,5S)-4-(4-methoxyphenyl)-3-[4-(3-methoxypropyl)-3-oxo-3,4-dihydro-2H-benzo[1,4]oxazin-6-ylmethoxy]-5-trityloxy-methylpiperidine-1-carboxylate in 20 ml of methanol and 4 ml of tetrahydrofuran is treated with 7.960 mmol of p-toluenesulphonic acid monohydrate and stirred at room temperature for 1.5 hours. The reaction mixture is poured onto saturated, aqueous sodium hydrogen-carbonate solution and extracted with dichloromethane (3x). The combined organic phases are dried using sodium sulphate and evaporated. The title compound is obtained as a colourless oil and used in crude form in the next stage. Rf = 0.10 (EtOAc-heptane = 2:1); Rt = 4.44 (gradient I).

### g) Benzyl (3R,4R,5S)-4-(4-methoxyphenyl)-3-[4-(3-methoxypropyl)-3-oxo-3,4-dihydro-2H-benzo[1,4]-oxazin-6-ylmethoxy]-5-trityloxymethylpiperidine-1-carboxylate

Analogously to method D, starting from benzyl (3R,4R,5S)-3-hydroxy-4-(4-methoxyphenyl)-5-trityloxy-methylpiperidine-1-carboxylate the title compound is obtained as a colourless wax. Rf = 0.26 (EtOAc-heptane = 1:1); Rt = 6.25 (gradient I).

### h) Benzyl (3R,4R,5S)-3-hydroxy-4-(4-methoxyphenyl)-5-trityloxymethylpiperidine-1-carboxylate

Analogously to the process described in method B (methanol -tetrahydrofuran 3:1 is used as a solvent) and in method V, starting from (3R,4R,5S)-1-benzyl-4-(4-methoxyphenyl)-5-trityloxymethylpiperidin-3-ol [303043-53-0] the title compound is obtained as a yellow oil. The title compound is used in crude form in the next stage. Rf = 0.25 (EtOAc-heptane = 1:1).

According to the process described in Example 3, the following compounds are prepared in an analogous manner:

### Examples

### 20 6-{(3R,4R,5S)-4-[4-(4-Methoxybutoxyphenyl-5-nitro-oxymethylpiperidin-3-yloxymethil}-4-(3-methoxy-propyl)-3,4-dihydro-2H-benzo[1,4]oxazine

starting from {(3S,4R,5R)-4-[4-(4-methoxybutoxy)-phenyl]-5-[4-(3-methoxypropyl)-3,4-dihydro-2H-benzo-[1,4]oxazin-6-ylmethoxy]piperidin-3-yl}methanol.

The starting materials are prepared as follows:

### a) {(3S,4R,5R)-4-[4-(4-Methoxybutoxy)phenyl]-5-[4-(3-methoxypropyl)-3,4-dihydro-2H-benzo[1,4]oxazin-6-ylmethoxy]piperidin-3-yl)methanol

Analogously to method L, starting from [(3S,4R,5R)-4-[4-(4-methoxybutoxy)phenyl]-5-[4-(3-methoxypropyl)-3,4-dihydro-2H-benzo[1,4]oxazin-6-ylmethoxy]-1-(toluene-4-sulphonyl)piperidin-3-yl]methanol the title compound is identified by means of the Rf value.

### b) [(3S,4R,5R)-4-[4-(4-Methoxybutoxy)phenyl]-5-[4-(3-methoxypropyl)-3,4-dihydro-2H-benzo[1,4]oxazin-6-ylmethoxy]-1-(toluene-4-sulphonyl)piperidin-3-yl]-methanol

Analogously to method F, starting from 4-[(3S,4R,5R)-3-hydroxymethyl-5-[4-(3-methoxypropyl)-3,4-dihydro-2H-benzo[1,4]oxazin-6-ylmethoxy]-1-(toluene-4-sulphonyl)-piperidin-4-yl]phenol and I-bromo-4-methoxybutane [4457-67-4] the title compound is obtained as a yellowish oil, Rf = 0.28 (EtOAc-heptane 2:1); Rt = 5.07 (gradient I).

### c) 4-[(3S,4R,5R)-3-Hydroxymethyl-5-[4-(3-methoxy-propyl)-3,4-dihydro-2H-benzo[1,4]oxazin-6-yl-methoxy]-1-(toluene-4-sulphonyl)piperidin-4-yl]-phenol

A solution of 3.99 mmol of [(3S,4R,5R)-4-(4-methoxy-phenyl)-5-[4-(3-methoxypropyl)-3,4-dihydro-2H-benzo-[1,4]oxazin-6-ylmethoxy]-1-(toluene-4-sulphonyl)-piperidin-3-yl]methanol in 35 ml of N,N-dimethyl-formamide is treated with 39.9 mmol of sodium ethanethiolate and the resulting suspension is heated at 120°C for 21 hours. The reaction mixture is cooled to 0°C and adjusted to pH 2 using 2M HCl. The mixture is diluted with water and extracted with tert-butyl methyl ether (2X). The combined organic phases are dried using sodium sulphate and evaporated. The title compound is obtained from the residue as a yellowish foam by means of flash chromatography (SiO₂ 60F). Rf = 0.19 (EtOAc-heptane 2:1); Rt = 4.35 (gradient I).

### d) [(3S,4R,5R)-4-(4-Methoxyphenyl)-5-[4-(3-methoxy-propyl)-3,4-dihydro-2H-benzo[1,4]oxazin-6-yl-methoxy]-1-(toluene-4-sulphonyl)piperidin-3-yl]-methanol

Analogously to method Example 9e, starting from {(3S,4R,5R)-4-(4-methoxyphenyl)-5-[4-(3-methoxypropyl)-3,4-dihydro-2H-benzo[1,4]oxazin-6-ylmethoxy]piperidin-3-yl}methanol (Example 3c) the title compound is obtained as a colourless oil. Rf = 0.25 (EtOAc-heptane 2:1); Rt = 4.83 (gradient I).

### 21 6-{(3R,4R,5S)-4-[4-((S)-3-Methoxy-2-methyl-propoxymethyl)phenyl]-5-nitrooxymethylpiperidin-3-yloxymethyl}-4-(3-methoxypropyl)-3,4-dihydro-2H-benzo[l,4]oxazine

starting from (3R,4R,5S)-1-benzyl-4-[4-((S)-3-methoxy-2-methylpropoxymethyl)phenyl]-5-trityloxymethyl-piperidin-3-ol.

The starting materials are prepared as follows:

### a) (3R,4R,5S)-1-Benzyl-4-[4-((S)-3-methoxy-2-methyl-propoxymethyl)phenyl]-5-trityloxymethylipiperidin-3-ol

A solution of 23.84 mmol of 1-benzyl-4-[4-((S)-3-methoxy-2-methylpropoxy-methyl)phenyl]-5-trityloxy-methylpiperidin-3-ol in 171 ml of tetrahydrofuran is treated at 65°C with 11.92 mmol of L-(+)-mandelic acid. After 1 minute, a further 158 ml of hexane are added. The solution is slowly cooled to room temperature, subsequently treated with 23 ml of hexane and stirred at room temperature for 15 hours. The white suspension is cooled to 10°C and filtered off. The white crystals are washed with 40 ml of tetrahydrofuran-hexane 1:3, dried under vacuum and subsequently dissolved in 200 ml of dichloromethane. The organic phase is washed successively with 50 ml of aqueous, saturated sodium carboxylate solution, 30 ml of water and 20 ml of brine, dried using sodium sulphate and evaporated. The crude product contains the title compound with an isomeric purity of 80% (determined by means of chiral HPLC). In order to increase the isomeric purity to 100%, the above racemate cleavage process is repeated with 0.75 equivalent of L-(+)-mandelic acid. The title compound is obtained from the residue as a yellowish oil. Rt= 4.89 (gradient I).

### b) 1-Benzyl-4-[4-((S)-3-methoxy-2-methylpropoxy-methyl)phenyl]-5-trityloxymethylpiperidin-3-ol (diastereomer mixture)

88.6 mmol of borane-tetrahydrofuran complex (1 M in tetrahydrofuran) are added dropwise at 0°C to a solution of 44.3 mmol of 1-benzyl-4-[4-((S)-3-methoxy-2-methylpropoxymethyl)phenyl-3-trityloxymethyl-1,2,3,6-tetrahydropyridine in 220 ml of tetrahydrofuran. The reaction solution is subsequently stirred at room temperature for 18 hours. The reaction mixture is cooled to 10°C and successively hydrolysed with a solution of 256.8 mmol of KOH in 20 ml of water and with a solution of hydrogen peroxide (30% in water). The reaction mixture is subsequently stirred at 60°C for 4 hours, cooled to room temperature, and diluted with 300 ml of tert-butyl methyl ether. The mixture is washed successively with 150 ml of water and 200 ml of brinewater 1:1. The combined aqueous phases are extracted with 100 ml of tert-butyl methyl ether. The combined organic phases are dried using sodium sulphate, filtered and concentrated. The title compound is obtained from the residue as a yellowish foam by means of flash chromatography (SiO₂ F60). Rf = 0.09 (EtOAc-heptane-triethylamine 100:100:1); Rt = 4.89 (gradient I).

### c) 1-Benzyl-4-[4-((S)-3-methoxy-2-methylpropoxy-methyl)phenyl]-3-trityloxymethyl-1,2,3,6-tetra-hydropyridine (diastereomer mixture)

A solution of 85.53 mmol of 1-benzyl-4-[4-((S)-3-methoxy-2-methylpropoxy-methyl)phenyl]-3-trityloxy-methylpiperidin-4-ol in 250 ml of pyridine is treated dropwise with 102.64 mmol of thionyl chloride at 0°C. The brown reaction mixture is stirred at 0°C for 5 minutes, then treated with 50 ml of 4N NaOH and evaporated. The residue is taken up in 750 ml of ethyl acetate and washed successively with 500 ml of aqueous, saturated sodium hydrogencarbonate solution, 500 ml of water and 100 ml of brine. The combined aqueous phases are additionally extracted with 250 ml of ethyl acetate. The combined organic phases are dried using sodium sulphate, filtered and evaporated. The title compound is obtained from the residue as a yellowish oil by means of flash chromatography (SiO₂ 60F). Rf = 0.28 (EtOAc-heptane-triethylamine 100:200:1); Rt = 5.64 (gradient I).

### d) 1-Benzyl-4-[4-((S)-3-methoxy-2-methylpropoxy-methyl)phenyl]-3-trityloxymethylpiperidin-4-ol (diastereomer mixture)

A suspension of 151.5 mmol of magnesium in 20 ml of tetrahydrofuran under Ar is treated with 0.125 mmol of 1,2-dibromoethane and warmed to 60°C until slight gas formation is observed. A solution of 151.5 mmol of 1-bromo-4-((S)-3-methoxy-2-methylpropoxymethyl)benzene in 170 ml of tetrahydrofuran is added dropwise. The orange reaction mixture is stirred under reflux for 1 hour, cooled to room temperature and treated dropwise with a solution of 125.2 mmol of 1-benzyl-3-trityloxy-methyl-piperidin-4-one [234757-27-8] in 170 ml of tetrahydrofuran. The internal temperature is kept below 40°C. The reaction mixture is stirred at room temperature for 16 hours and treated successively with 300 ml of aqueous, saturated ammonium chloride solution and 300 ml of ethyl acetate. The organic phase is separated off and washed successively with 2 X 100 ml of water and 100 ml of brine. The combined aqueous phases are extracted with 100 ml of ethyl acetate. The combined organic phases are dried using sodium sulphate and evaporated. The title compound is obtained from the residue as a yellowish oil by means of flash chromatography (SiO₂ 60F). Rf = 0.30 (EtOAc-heptane-Et₃N 100:100:1); Rt = 5.43 (gradient I).

### e) 1-Bromo-4-((S)-3-methoxy-2-methylpropoxymethyl)-benzene A suspension of 360.13 mmol of sodium hydride (60% dispersion in oil) in 100 ml of

N,N-dimethylformamide is cooled to 0°C under argon and treated dropwise with a solution of 261.09 mmol of (R)-3-methoxy-2-methyl-propan-1-ol [911855-78-2] in 50 ml of N,N-dimethyl-formamide. The reaction mixture is stirred at 0°C-10°C for 20 minutes and then treated with 180.0 mmol of I-bromo-4-chlormethylbenzene [158328-45-1] within 30 minutes. The reaction mixture is stirred at 0°C-10°C for 75 minutes, then it is diluted with 400 ml of tert-butyl methyl ether and poured onto 400 ml of aqueous, saturated sodium hydrogencarbonate solution. The organic phase is separated off and the aqueous phase is extracted with 3 X 500 ml of tert-butyl methyl ether. The combined organic phases are washed successively with 350 ml of water and 350 ml of brine, dried using sodium sulphate and evaporated. The title compound is obtained from the residue as a yellow oil by means of flash chromatography (SiO₂ 60F). Rf = 0.44 (EtOAc-heptane 1:6); Rt = 5.29 (Gradient I).

### 22 6-{(3R,4R,5S)-4-[4-(2-Methoxyethoxymethyl)phenyl]-5-nitrooxymethylpiperidin-3-yloxymethyl}-4-(3-methoxypropyl)-3,4-dihydro-2H-benzo[1,4]oxazine

starting from (3R,4R,5S)-1-benzyl-4-[4-((S)-3-methoxy-2-methylpropoxy-methyl)phenyl]-5-trityloxymethyl-piperidin-3-ol.

The starting material is prepared as follows:

### a) (3R,4R,5S)-1-Benzyl-4-[4-(2-methoxyethoxymethyl)-phenyl]-5-trityloxymethyl-piperidin-3-ol

Analogously to the process described in Examples 21 a, 21 b, 21 c and 21 d, starting from 1-benzyl-3-trityloxymethylpiperidin-4-one [234757-27-8] and 1-bromo-4-(2-methoxyethoxymethyl)benzene [166959-29-1] the title compound is obtained as a colourless foam. Rf = 0.21 (EtOAc-heptane 4:1); Rt = 4.90 (gradient 1).

### Example 4

### 6-[(3R,4S,5S)-4-(4-Methoxyphenyl )-5-(3-nitrooxy-propoxy)piperidin-3-yloxymethyl]-4-(3-methoxypropyl)-3,4-dihydro-2H-benzo[1,4]oxazine

Analogously to method Q, starting from tert-butyl (3R,4S,5S)-4-(4-methoxyphenyl)-3-[4-(3-methoxypropyl)-3,4-dihydro-2H-benzo[1,4]oxazin-6-ylmethoxy]-5-(3-nitrooxy-propoxy)piperidine-1-carboxylate the title compound is identified by means of the Rf value.

The starting materials are prepared as follows:

### a) tert-Butyl (3R,45,5S)-4-(4-methoxyphenyl)-3-[4-(3-methoxyprovpyl)-3,4-dihydro-2H-benzo[1,4]oxazin-6-ylmethoxy]-5-(3-nitrooxypropoxy)piperidine-1-carboxylate

Analogously to method S, starting from tert-butyl (3S,4S,5R)-3-(3-methanesulphonyl-oxypropoxy)-4-(4-methoxyphenyl)-5-[4-(3-methoxypropyl)-3,4-dihydro-2H-benzo[1,4]oxazin-6-ylmethoxy]piperidine-1-carboxylate the title compound is identified by means of the Rf value.

### b) tert-Butyl (3S,4S,5R)-3-(3-methanesulphonyloxy-propoxy)-4-(4-methoxyphenyl)-5-[4-(3-methoxy-propyl)-3,4-dihydro-2H-benzo[1,4]oxazin-6-yl-methoxy]piperidine-1-carboxylate

Analogously to method T, starting from tert-butyl (3S,4S,5R)-3-(3-hydroxypropoxy)-4-(4-methoxyphenyl)-5-[4-(3-methoxypropyl)-3,4-dihydro-2H-benzo[1,4]oxazin-6-ylmethoxy]piperidine-1-carboxylate the title compound is identified by means of the Rf value.

### c) tert-Butyl (3S,4S,5R)-3-(3-hydroxypropoxy)-4-(4-methoxyphenyl)-5-[4-(3-methoxypropyl)-3,4-dihydro-2H-benzo[1,4]oxazin-6-ylmethoxy]piperidine-1-carboxylate

Analogously to method R, starting from 3-{(3S,4S,5R)-4-(4-methoxyphenyl)-5-[4-(3-methoxypropyl)-3,4-dihydro-2H-benzo[1,4]oxazin-6-ylmethoxy]piperidin-3-yloxy}-propan-1-ol the title compound is identified by means of the Rf value.

### d) 3-{(3S,4S,5R)-4-(4-Methoxyphenyl)-5-[4-(3-methoxy-propyl)-3,4-dihydro-2H-benzo[1,4]oxazin-6-ylmethoxy]-piperidin-3-yloxy}propan-1-ol

Analogously to method B, starting from benzyl (3S,4S,5R)-3-(3-hydroxypropoxy)-4-(4-methoxyphenyl)-5-[4-(3-methoxypropyl)-3,4-dihydro-2H-benzo[1,4]oxazin-6-ylmethoxy]piperidine-1-carboxylate the title compound is identified by means of the Rf value.

### e) Benzyl (3S,4S,5R)-3-(3-hydroxypropoxy)-4-(4-methoxyphenyl)-5-[4-(3-methoxypropyl)-3,4-dihydro-2H-benzo[1,4]oxazin-6-ylmethoxylipiperidine-1 - carboxylate

Analogously to method J, starting from benzyl (3R,4S,5S)-4-(4-methoxyphenyl)-3-[4-(3-methoxypropyl)-3,4-dihydro-2H-benzo[1,4]oxazin-6-ylmethoxy]-5-(3-tri-isopropyl-silanyloxypropoxy)piperidine-1-carboxylate the title compound is identified by means of the Rf value.

### f) Benzyl (3R,45,5S)-4-(4-methoxyphenyl-3-[4-(3-methoxypropyl)-3,4-dihydro-2H-benzo[1,4]oxazin-6-ylmethoxy]-5-(3-triisoproipylsilanyloxypropoxy)-piperidine-1-carboxylate

A solution of 0.495 mmol of benzyl (3S,4S,5R)-3-hydroxy-4-(4-methoxyphenyl)-5-[4-(3-methoxypropyl)-3,4-dihydro-2H-benzo[1,4]oxazin-6-ylmethoxy]piperidine-1-carboxylate [873945-56-3] in 3 ml of N,N-dimethyl-formamide is treated at 0°C with 0.742 mmol of sodium hydride (60% dispersion in oil). The reaction mixture is stirred at room temperature for 30 minutes and subsequently treated with 0.050 mmol of sodium iodide and 0.742 mmol of (3-bromopropoxy)triisopropylsilane [215650-24-1]. The reaction mixture is stirred at room temperature for 2 hours. The reaction mixture is poured onto saturated aqueous sodium hydrogencarbonate solution and the mixture is extracted with tert-butyl methyl ether (3X). The combined organic extracts are washed with brine, dried using sodium sulphate and evaporated. The title compound is identified from the residue by means of the Rf value by means of flash chromatography (SiO₂ 60F).

According to the process described in Example 4, the following compound is prepared in an analogous manner:

### Example

### 19 6-[(3R,4S,5S)-4-[4-(2-Methoxyethoxymethyl)phenyl]-5-(3-nitrooxy-propoxy)piperidin-3-yloxymethyl-4-(3-methoxypropyl)-3,4-dihydro-2H-benzol[1,4]oxazine

Starting from 3-{(3S,4S,5R)-4-[4-(2-methoxyethoxy-methyl)phenyl]-5-[4-(3-methoxypropyl)-3,4-dihydro-2H-benzo[1,4]oxazin-6-ylmethoxy]piperidin-3-yloxy}propan-1-ol.

The starting materials are prepared as follows:

### a) 3-{(3S,4S,5R)-4-[4-(2-Methoxyethoxymethyl)phenyl]-5-[4-(3-methoxylprolpyl)-3,4-dihydro-2H-benzo[1,4]-oxazin-6-ylmethoxylpiperidin-3-yloxy}propan-1-ol

Analogously to method L, starting from 3-[(3S,4S,5R)-4-[4-(2-methoxyethoxymethyl)phenyl]-5-[4-(3-methoxy-propyl)-3,4-dihydro-2H-benzo[1,4]oxazin-6-ylmethoxy]-1-(toluene-4-sulphonyl)piperidin-3-yloxy]propan-1-ol [912346-74-8] the title compound is identified by means of the Rf value.

### Example 5

### (S)-1-{(3S,4R,5R)-4-(4-Methoxyphenyl)-5-[4-(3-methoxy-propyl)-3,4-dihydro-2H-benzo[1,4]oxazin-6-ylmethoxy]-piperidin-3-yloxy}-3-nitrooxypropan-2-ol

Analogously to method Q, starting from tert-butyl (3S,4R,5R)-3-((S)-2-hydroxy-3-nitrooxypropoxy)-4-(4-methoxyphenyl)-5-[4-(3-methoxypropyl)-3,4-dihydro-2H-benzo[1,4]oxazin-6-ylmethoxy]piperidine-1-carboxylate the title compound is identified by means of the Rf value.

The starting materials are prepared as follows:

### a) tert-Butyl (3S,4R,5R)-3-((S)-2-hydroxy-3-nitrooxy-propoxy)-4-(4-methoxyphenyl)-5-[4-(3-methoxy-propyl)-3,4-dihydro-2H-benzo[1,4]oxazin-6-yl-methoxy]piperidine-1-carboxylate

A solution of 1 mmol of tert-butyl (3R,4R,5S)-4-(4-methoxyphenyl)-3-[4-(3-methoxypropyl)-3,4-dihydro-2H-benzo[1,4]oxazin-6-ylmethoxy]-5-((R)-1-oxiranylmethoxy)piperidine-1-carboxylate, 3 mmol of tetrabutyl-ammonium nitrate and 0.7 mmol of cerammonium nitrate in 4 ml of dry acetonitrile is stirred at 80°C for 4 hours. The solvent is evaporated and the residue is suspended in water and extracted with tert-butyl methyl ether (3X), and the combined organic phases are dried using sodium sulphate and evaporated. The title compound is identified by means of the Rf value from the residue by means of flash chromatography (SiO₂ 60F).

### b) tert-Butyl (3R,4R,5S)-4-(4-methoxyphenyl)-3-[4-(3-methoxypropyl)-3,4-dihydro-2H-benzo[1,4]oxazin-6-ylmethoxy]-5-((R)-1-oxiranylmethoxy)piperidine-1-carboxylate

Analogously to the process described in Example 2e, starting from tert-butyl (3S,4S,5R)-3-hydroxy-4-(4-methoxyphenyl)-5-[4-(3-methoxypropyl)-3,4-dihydro-2H-benzo[1,4]oxazin-6-ylmethoxy]piperidine-1-carboxylate the title compound is identified by means of the Rf value.

### c) tert-Butyl (3S,4S,5R)-3-hydroxy-4-(4-methoxy-phenyl)-5-[4-(3-methoxypropyl)-3,4-dihydro-2H-benzo[1,4]oxazin-6-ylmethoxy]piperidine-1-carboxylate

Analogously to method R, starting from (3S,4S,5R)-4-(4-methoxyphenyl)-5-[4-(3-methoxypropyl)-3,4-dihydro-2H-benzo[1,4]oxazin-6-ylmethoxy]piperidin-3-ol [873945-55-2] the title compound is identified by means of the Rf value.

According to the process described in Example 5, the following compounds are prepared in an analogous manner:

### Examples

### 8 (S)-1-{(3S,4R,5R)-4-[4-((S)-3-Methoxy-2-methyl-propoxymethyl)phenyl]-5-[4-(3-methoxypropyl)-3,4-dihydro-2H-benzo[1,4]oxazin-6-ylmethoxy]-piperidin-3-yloxy}-3-nitrooxypropan-2-ol

starting from (3S,4S,5R)-4-[4-((S)-3-methoxy-2-methyl-propoxymethyl)phenyl]-5-[4-(3-methoxypropyl)-3,4-di-hydro-2H-benzo[1,4]oxazin-6-ylmethoxy]piperidin-3-ol [911854-43-8].

### 12 (R)-1-{(3S,4R,5R)-4-[4-((S)-3-Methoxy-2-methyl-propoxymethyl)phenyl]-5-[4-(3-methoxypropyl)-3,4-dihydro-2H-benzo[1,4]oxazin-6-ylmethoxyl-piperidin-3-yloxy}-3-nitrooxypropan-2-ol

starting from (3S,4S,5R)-4-[4-((S)-3-methoxy-2-methyl-propoxymethyl)phenyl]-5-[4-(3-methoxypropyl)-3,4-dihydro-2H-benzo[1,4]oxazin-6-ylmethoxy]piperidin-3-ol [911854-43-8] using (S)-I-oxiranylmethyl toluene-4-sulphonate [70987-78-9] in stage b.

### 15 (S)-1-{(3S,4R,5R)-4-[4-(4-Methoxybutoxy)phenyl]-5-[4-(3-methoxypropyl)-3,4-dihydro-2H-benzo[1,4]-oxazin-6-ylmethoxylpiperidin-3-yloxy}-3-nitrooxy-propan-2-ol

starting from (3S,4S,5R)-4-[4-(4-methoxybutoxy)phenyl]-5-[4-(3-methoxypropyl)-3,4-dihydro-2H-benzo[1,4]oxazin-6-ylmethoxy]piperidin-3-ol.

The starting material is prepared as follows:

### a) (3S,45,5R)-4-[4-(4-Methoxybutoxy)phenyl]-5-[4-(3-methoxypropyl)-3,4-dihydro-2H-benzo[1,4]oxazin-6-ylmethoxy]piperidin-3-ol

Analogously to method B, starting from benzyl (3S,4S,5R)-3-hydroxy-4-[4-(4-methoxybutoxy)phenyl]-5-[4-(3-methoxypropyl)-3,4-dihydro-2H-benzo[1,4]oxazin-6-ylmethoxy]piperidine-1-carboxylate (Example 11a) the title compound is obtained as a yellow oil.

Rf= 0.08 (dichlormethane-methanol-ammonia conc. 25% = 200:10:1); Rt = 3.56 (gradient I).

### 16 (R)-1-{(3S,4R,5R)-4-[4-(4-Methoxybutoxy)phenyl]-5-[4-(3-methoxypropyl)-3,4-dihydro-2H-benzo[1,4]-oxazin-6-ylmethoxy]piperidin-3-yloxy}-3-nitrooxy-propan-2-ol

starting from (3S,4S,5R)-4-[4-(4-methoxybutoxy)phenyl]-5-[4-(3-methoxypropyl)-3,4-dihydro-2H-benzo[1,4]oxazin-6-ylmethoxy]piperidin-3-ol (Example 15a) using (S)-I-oxiranymethyl toluene-4-sulphonate [70987-78-9] in stage b.

### 32 (S)-1-{(3S,4R,5R)-4-[4-(2-Methoxyethoxymethyl)-phenyl]-5-[4-(3-methoxypropyl)-3,4-dihydro-2H-benzo[1,4]oxazin-6-ylmethoxy]piperidin-3-yloxy}-3-nitrooxypropan-2-ol

starting from (3S,4S,5R)-4-[4-(2-methoxyethoxymethyl)-phenyl]-5-[4-(3-methoxypropyl)-3,4-dihydro-2H-benzo-[1,4]oxazin-6-ylmethoxy]piperidin-3-ol.

The starting material is prepared as follows:

### a) (3S,4S,5R)-4-[4-(2-Methoxyethoxymethyl)phenyl]-5-[4-(3-methoxypropyl)-3,4-dihydro-2H-benzo[1,4]-oxazin-6-ylmethoxy]piperidin-3-ol

Analogously to method B, starting from benzyl (3S,4S,5R)-3-hydroxy-4-[4-(2-methoxyethoxymethyl)-phenyl]-5-[4-(3-methoxypropyl)-3,4-dihydro-2H-benzo-[1,4]oxazin-6-ylmethoxy]piperidine-1-carboxylate [912346-67-9] the title compound is obtained as a yellow resin. Rf = 0.18 (dichloromethane-methanol-ammonia conc. 25% = 200:20:1); Rt = 3.22 (gradient I).

### 33 (R)-1-{(3S,4R,5R)-4-(4-Methoxyphenyl)-5-[4-(3-methoxypropyl)-3,4-dihydro-2H-benzo[1,4]oxazin-6-ylmethoxy]piperidin-3-yloxy}-3-nitrooxypropan-2-ol

using (S)-I-oxiranymethyl toluene-4-sulphonate [70987-78-9] in stage b.

### Example 10

### N-{(3S,4R,5R)-4-(4-Methoxyphenyl)-5-[4-(3-methoxy-propyl)-3,4-dihydro-2H-benzo[1,4]oxazin-6-ylmethox]-piperidin-3-ylmetyl}-2-nitrooxyacetamide

Analogously to method Q, starting from tert-butyl (3R,4R,5R)-4-(4-methoxyphenyl)-3-[4-(3-methoxypropyl)-3,4-dihydro-2H-benzo[1,4]oxazin-6-ylmethoxy]-5-[(2-nitrooxyacetylamino)methyl]piperidine-1-carboxylate the title compound is identified by means of the Rf value.

The starting materials are prepared as follows:

### a) tert-Butyl (3R,4R,5R)-4-(4-methoxyphenyl)-3-[4-(3-methoxypropyl)-3,4-dihydro-2H-benzo[1,4]oxazin-6ylmethoxy]-5-[(2-nitrooxyacetylamino)methyl-piperidine-1-carboxylate

Analogously to method S, starting from tert-butyl (3R,4R,5R)-3-[(2-methanesulphonyloxyacetylamino)-methyl]-4-(4-methoxyphenyl)-5-[4-(3-methoxypropyl)-3,4-dihydro-2H-benzo[1,4]oxazin-6-ylmethoxy]piperidine-1-carboxylate the title compound is identified by means of the Rf value.

### b) tert-Butyl (3R,4R,5R)-3-[(2-methanesulphonyloxy-acetylamino)methyl]-4-(4-methoxyphenyl)-5-[4-(3-methoxypropyl)-3,4-dihydro-2H-benzo[1,4]oxazin-6-ylmethoxy]lpiperidine-1-carboxylate

Analogously to method T, starting from tert-butyl (3R,4R,5R)-3-[(2-hydroxyacetylamino)methyl]-4-(4-methoxyphenyl)-5-[4-(3-methoxypropyl)-3,4-dihydro-2H-benzo[1,4]oxazin-6-ylmethoxy]piperidine-1-carboxylate the title compound is identified by means of the Rf value.

### c) tert-Butyl (3R,4R,5R)-3-[(2-hydroxyacetylamino)-methyl]-4-(4-methoxyphenyl)-5-[4-(3-methoxy-propyl)-3,4-dihydro-2H-benzo[1,4]oxazin-6-yl-methoxy]piperidine-1-carboxylate

Analogously to method R, starting from 2-hydroxy-N-{(3S,4R,5R)-4-(4-methoxyphenyl)-5-[4-(3-methoxypropyl)-3,4-dihydro-2H-benzo[1,4]oxazin-6-ylmethoxy]piperidin-3-ylmethyl}acetamide the title compound is identified by means of the Rf value.

### d) 2-Hydroxy-N-{(3S,4R,5R)-4-(4-methoxyphenyl)-5-[4-(3-methoxypropyl)-3,4-dihydro-2H-benzo[1,4]oxazin-6-ylmethoxy]piperidin-3-ylmethyl}acetamide

Analogously to method B, starting from benzyl (3R,4R,5R)-3-[(2-hydroxyacetyl-amino)methyl]-4-(4-methoxyphenyl)-5-[4-(3-methoxypropyl)-3,4-dihydro-2H-benzo[1,4]oxazin-6-ylmethoxy]piperidine-1-carboxylate the title compound is identified by means of the Rf value.

### e) Benzyl (3R,4R,5R)-3-[(2-hydroxyacetylamino)-methyl]-4-(4-methoxyphenyl)-5-[4-(3-methoxy-propyl)-3,4-dihvdro-2H-benzo[1,41oxazin-6-yl-methoxy]piperidine-1-carboxylate

Analogously to method J, starting from benzyl (3R,4R,5R)-3-{[2-(tert-butyldimethyl-silanyloxy)acetyl-amino]methyl}-4-(4-methoxyphenyl)-5-[4-(3-methoxy-propyl)-3,4-dihydro-2H-benzo[1,4]oxazin-6-ylmethoxy]-piperidine-1-carboxylate the title compound is identified by means of the Rf value.

### f) Benzyl (3R,4R,5R)-3-{[2-(tert-butyldimethyl-silanyloxy)acetylamino]methyl}-4-(4-methoxy-phenyl)-5-[4-(3-methoxypropyl)-3,4-dihydro-2H-benzo[1,4]oxazin-6-ylmethoxy]piperidine-1-carboxylate

A solution of 1 mmol of benzyl (3R,4R,5R)-3-amino-methyl-4-(4-methoxyphenyl)-5-[4-(3-methoxypropyl)-3,4-dihydro-2H-benzo[1,4]oxazin-6-ylmethoxy]piperidine-1-carboxylate and 1.1 mmol of (tert-butyldimethyl-silanyloxy)acetyl chloride [78826-45-6] in 20 ml of dichloromethane at 0°C is treated with 3 mmol of triethylamine. After 1.5 hours, the reaction mixture is poured onto 1 M sodium hydrogencarbonate solution and extracted with tert-butyl methyl ether (3X), the combined organic phases are washed with brine, dried using sodium sulphate and evaporated. The title compound is identified by means of the Rf value from the residue by means of flash chromatography (SiO₂ 60F).

### g) Benzyl (3R,4R,5R)-3-aminomethyl-4-(4-methoxy-phenyl)-5-[4-(3-methoxypropyl)-3,4-dihydro-2H-benzo[1,4]oxazin-6-ylmethoxylpiperidine-1-carboxylate

A solution of 0.325 mmol of benzyl (3S,4R,5R)-3-azidomethyl-4-(4-methoxyphenyl)-5-[4-(3-methoxypropyl)-3,4-dihydro-2H-benzo[1,4]oxazin-6-ylmethoxy]piperidine-1-carboxylate in 0.95 ml of tetrahydrofuran and 0.23 ml of water is treated at room temperature with a solution of 2.6 mmol of ammonia conc. 25% in 0.9 ml of methanol. After addition of 0.488 mmol of triphenylphosphine, the reaction mixture is stirred at room temperature for 16 hours. The mixture is diluted with ethyl acetate and washed with semisaturated aqueous sodium hydrogen-carbonate solution (2X), dried using sodium sulphate and evaporated. The title compound is obtained from the residue by means of flash chromatography (SiO₂ 60F) as a colourless oil. Rf = 0.31 (dichloromethane-methanol-ammonia conc. 25% = 200:20:1); Rt = 4.24 (gradient I).

### h) Benzyl (3S,4R,5R)-3-azidomethyl-4-(4-methoxyphenyl-5-[4-(3-methoxypropyl)-3,4-dihydro-2H-benzo[1,4]oxazin-6-ylmethoxy]piperidine-1-carboxylate

A solution of 0.50 mmol of benzyl (3R,4R,5S)-4-(4-methoxyphenyl)-3-[4-(3-methoxypropyl)-3,4-dihydro-2H-benzo[1,4]oxazin-6-ylmethoxy]-5-(toluene-4-sulphonyl-oxymethyl)piperidine-1-carboxylate and 2 mmol of sodium azide in 5 ml of 1,3-dimethyl-tetrahydropyrimidin-2-one (DMPU) is stirred at 80°C for 4 hours. The reaction mixture is diluted with water and extracted with tert-butyl methyl ether (3X). The combined organic phases are dried using sodium sulphate and evaporated. The title compound is obtained from the residue as a yellowish oil by means of flash chromatography (SiO₂ 60F). Rf = 0.33 (EtOAc-heptane = 1:1); Rt = 5.61 (gradient I).

### i) Benzyl (3R,4R,5S)-(4-methoxyphenyl)-3-[4-(3-methoxypropyl)-3,4-dihydro-2H-benzo[1,4]oxazin-6-ylmethoxy]-5-(toluene-4-sulphonyloxymethyl)-piperidine-1-carboxylate

Analogously to method H, starting from benzyl (3S,4R,5R)-3-hydroxymethyl-4-(4-methoxyphenyl)-5-[4-(3-methoxypropyl)-3,4-dihydro-2H-benzo[1,4]oxazin-6-yl-methoxy]piperidine-1-carboxylate (Example 3e) the title compound is obtained as a yellow oil. The title compound is used in crude form in the next stage. Rf = 0.39 (EtOAc-heptane=2:1).

According to the process described in Example 10, the following compound is prepared in an analogous manner:

### Example

### 28 N-{(3S,4R,5R)-4-[4-((S)-3-Methoxy-2-methyl-propoxymethyl)phenyl]-5-[4-(3-methoxypropyl)-3,4-dihydro-2H-benzo[1,4]oxazin-6-ylmethoxy]piperidin-3-ylmethyl}-2-nitrooxyacetamide

starting from benzyl (3S,4R,5R)-3-hydroxymethyl-4-[4-((S)-3-methoxy-2-methyl-propoxymethyl)phenyl]-5-[4-(3-methoxypropyl)-3,4-dihydro-2H-benzo[1,4]oxazin-6-yl-methoxy]piperidine-1-carboxylate.

The starting material is prepared as follows:

### a) Benzyl (3S,4R,5R)-3-hydroxymethyl-4-[4-((S)-3-methoxy-2-methylpropoxymethyl)phenyl]-5-[4-(3-methoxypropyl)-3,4-dihydro-2H-benzo[1,4]oxazin-6-ylmethoxy]piperidine-1-carboxylate

Analogously to the processes described in Examples 3e, 3f, 3g and 3h, starting from (3R,4R,5S)-1-benzyl-4-[4-((S)-3-methoxy-2-methylpropoxymethyl)phenyl]-5-trityl-oxymethylpiperidin-3-ol (L)-(+)-mandelate (Example 21 a) the title compound is identified by means of the Rf value.

### Example 13

### 6-[(3R,4R,5S)-5-((S)-3-Methoxy-2-nitrooxyropoxy)-4-(4-methoxyphenyl)piperidin-3-yloxymethyl-4-(3-methoxy-propyl)-3,4-dihydro-2H-benzo[1,4]oxazine

Analogously to method Q, starting from tert-butyl (3S,4R,5R)-3-((S)-3-methoxy-2-nitrooxypropoxy)-4-(4-methoxyphenyl)-5-[4-(3-methoxypropyl)-3,4-dihydro-2H-benzo[1,4]oxazin-6-ylmethoxy]piperidine-1-carboxylate the title compound is identified by means of the Rf value.

The starting materials are prepared as follows:

### a) tert-Butyl (3S,4R,5R)-3-((S)-3-methoxy-2-nitrooxy-propoxy)-4-(4-methoxyphenyl)-5-[4-(3-methoxy-propyl)-3,4-dihydro-2H-benzo[1,4]oxazin-6-yl-methoxy]piperidine-1-carboxylate

Analogously to method S, starting from tert-butyl (3S,4R,5R)-3-((R)-2-methane-sulphonyloxy-3-methoxy-propoxy)-4-(4-methoxyphenyl)-5-[4-(3-methoxypropyl)-3,4-dihydro-2H-benzo[1,4]oxazin-6-ylmethoxy]piperidine-1-carboxylate the title compound is identified by means of the Rf value.

### b) tert-Butyl (3S,4R,5R)-3-((R)-2-methanesulphonyl-oxy-3-methoxypropoxy)-4-(4-methoxyphenyl)-5-[4-(3-methoxypropyl)-3,4-dihydro-2H-benzo[1,4]oxazin-6-ylmethoxy]piperidine-1-carboxylate

Analogously to method T, starting from tert-butyl (3S,4R,5R)-3-((R)-2-hydroxy-3-methoxypropoxy)-4-(4-methoxyphenyl)-5-[4-(3-methoxypropyl)-3,4-dihydro-2H-benzo[1,4]oxazin-6-ylmethoxy]piperidine-1-carboxylate the title compound is identified by means of the Rf value.

### c) tert-Butyl (3S,4R,5R)-3-((R)-2-hydroxy-3-methoxy-propoxy)-4-(4-methoxyphenyl)-5-[4-(3-methoxy-propyl)-3,4-dihydro-2H-benzo[1,4]oxazin-6-yl-methoxy]piperidine-1-carboxylate

tert-Butyl (3S,4S,5R)-3-hydroxy-4-(4-methoxyphenyl)-5-[4-(3-methoxypropyl)-3,4-dihydro-2H-benzo[1,4]oxazin-6-ylmethoxy]piperidine-1-carboxylate (Example 3c) and S-(+)-glycidyl methyl ether [64491-68-5] are reacted analogously to method M. The title compound is identified by means of the Rf value.

According to the process described in Example 13, the following compounds are prepared in an analogous manner:

### Examples

### 14 6-[(3R,4R,5S)-4-[4-((S)-3-Methoxy-2-methyl-propoxymethyl)phenyl-5-((R)-3-methoxy-2-nitrooxypropoxy)piperidin-3-yloxymethyl-4-(3-methoxypropyl)-3,4-dihydro-2H-benzo[1,4]oxazine

Starting from tert-butyl (3S,4R,5R)-3-((S)-2-hydroxy-3-methoxypropoxy)-4-[4-((S)-3-methoxy-2-methylpropoxy-methyl)phenyl]-5-[4-(3-methoxypropyl)-3,4-dihydro-2H-benzo[1,4]oxazin-6-ylmethoxy]piperidine-1-carboxylate.

The starting material is prepared as follows:

### a) tert-Butyl (3S,4R,5R)-3-((S)-2-hydroxy-3-methoxy-propoxy)-4-[4-((S)-3-methoxy-2-methylpropoxy-methyl)phenyl-5-[4-(3-methoxypropyl)-3,4-dihydro-2H-benzo[1,4]oxazin-6-ylmethoxy]piperidine-1-carboxylate

Analogously to method R, starting from (S)-1-methoxy-3-{(3S,4R,5R)-4-[4-((S)-3-methoxy-2-methylpropoxy-methyl)phenyl]-5-[4-(3-methoxypropyl)-3,4-dihydro-2H-benzo[1,4]oxazin-6-ylmethoxy)piperidin-3-yloxy}propan-2-ol [911854-51-8] the title compound is identified by means of the Rf value.

### 34 6-[(3R,4R,5S)-4-[4-((S)-3-Methoxy-2-methylpropoxy-methyl)phenyl]-5-((S)-3-methoxy-2-nitrooxy-propoxy)piperidin-3-yloxymethyl]-4-(3-methoxy-propyl)-3,4-dihydro-2H-benzo[1,4]oxazine

Starting from (3S,4S,5R)-4-[4-((S)-3-methoxy-2-methyl-propoxymethyl)phenyl]-5-[4-(3-methoxypropyl)-3,4-di-hydro-2H-benzo[1,4]oxazin-6-ylmethoxy]-1-(toluene-4-sulphonyl)piperidin-3-ol [911855-76-0] using S-(+)-glycidyl methyl ether [64491-68-5].

### 35 6-[f(3R,4R,5S)-5-((R)-3-Methoxy-2-nitrooxypropoxy)-4-(4-methoxyphenyl)piperidin-3-yloxymethyl]-4-(3-methoxypropyl)-3,4-dihydro-2H-benzo[1,4]oxazine

using S-(+)-glycidyl methyl ether [64491-68-5].

### Example 18

### 6-[(3R,4S,5S)-4-[4-(2-Methoxyethoxymethyl)iphenyl]-5-((S)-3-nitrooxybutox)piperidin-3-yloxymethyl]-4-(3-methoxypropyl)-3,4-dihydro-2H-benzo[1,4]oxazine Analogously to method Q, starting from tert-butyl (3R,4S,5S)-4-[4-(2-methoxyethoxymethyl)phenyl]-3-[4-(3-methoxypropyl)-3,4-dihydro-2H-benzo[1,4]oxazin-6-yl-methoxy]-5-((S)-3-nitrooxybutoxy)piperidine-1-carboxylate the title compound is identified by means of the Rf value.

The starting materials are prepared as follows:

### a) tert-Butyl (3R,4S,5S)-4-[4-(2-methoxyethoxy-methyl)phenyl-3-[4-(3-methoxypropyl)-3,4-dihydro-2H-benzo[1,4]oxazin-6-ylmethoxy]-5-((S)-3-nitrooxybutoxy)piperidine-1-carboxylate

Analogously to method S, starting from tert-butyl (3S,4S,5R)-3-((R)-3-methanesulphonyloxybutoxy)-4-[4-(2-methoxyethoxymethyl)phenyl]-5-[4-(3-methoxypropyl)-3,4-dihydro-2H-benzo[1,4]oxazin-6-ylmethoxy]piperidine-1-carboxylate the title compound is identified by means of the Rf value.

### b) tert-Butyl (3S,4S,5R)-3-((R)-3-methanesulphonyl-oxybutoxy)-4-[4-(2-methoxyethoxymethyl)phenyl]-5-[4-{3-methoxypropyl)-3,4-dihydro-2H-benzo[1,4]-oxazin-6-ylmethoxy]piperidine-1-carboxylate

Analogously to method T, starting from tert-butyl (3S,4S,5R)-3-((R)-3-hydroxybutoxy)-4-[4-(2-methoxy-ethoxymethyl)phenyl]-5-[4-(3-methoxypropyl)-3,4-di-hydro-2H-benzo[1,4]oxazin-6-ylmethoxy]piperidine-1-carboxylate the title compound is identified by means of the Rf value.

### c) tert-Butyl (3S,4S,5R)-3-((R)-3-hydroxybutoxy)-4-[4-(2-methoxyethoxymethyl)phenyl]-5-[4-(3-methoxy-propyl)-3,4-dihydro-2H-benzo[1,4]oxazin-6-yl-methoxy]piperidine-1-carboxylate

Analogously to method R, starting from (R)-4-{(3S,4S,5R)-4-[4-(2-methoxyethoxymethyl)phenyl]-5-[4-(3-methoxypropyl)-3,4-dihydro-2H-benzo[1,4]oxazin-6-yl-methoxy]piperidin-3-yloxy}butan-2-ol [911855-23-7] the title compound is identified by means of the Rf value.

According to the process described in Example 18, the following compound is prepared in an analogous manner:

### Example

### 43 6-[(3R,4S,5S)-4-(4-Methoxyphenyl)-5-((S)-3-nitro-oxybutoxy)piperidin-3-yloxymethyl]-4-(3-methoxy-propyl)-3,4-dihydro-2H-benzo[1,4]oxazine

Starting from tert-butyl (3S,4S,5R)-3-((R)-3-hydroxy-butoxy)-4-(4-methoxyphenyl)-5-[4-(3-methoxypropyl)-3,4-dihydro-2H-benzo[1,4]oxazin-6-ylmethoxy]piperidine-1-carboxylate

The starting material is prepared as follows:

### a) tert-Butyl (3S,4S,5R)-3-((R)-3-hydroxybutoxy)-4-(4-methoxyphenyl)-5-[4-(3-methoxypropyl)-3,4-di-hydro-2H-benzo[1,4]oxazin-6-ylmethoxy]piperidine-1-carboxylate

Analogously to the processes described in method J and in method W, starting from tert-butyl (3S,4S,5R)-3-hydroxy-4-(4-methoxyphenyl)-5-[4-(3-methoxypropyl)-3,4-dihydro-2H-benzo[1,4]oxazin-6-ylmethoxy]piperidine-1-carboxylate (Example 3c) the title compound is identified by means of the Rf value.

### Example 26

### 2-{(3S,4R,5R)-4-{4-[3-(2-Chlorophenoxy)propoxy]phenyl}-5-[4-(3-methoxypropyl)-3,4-dihydro-2H-benzo[1,4]oxazin-6-ylmethoxylpiperidin-3-yloxy}-N-(2-nitrooxyethyl)-acetamide

Analogously to method Q, starting from tert-butyl (3R,4R,5S)-4-{4-[3-(2-chlorophenoxy)propoxy]phenyl}-3-[4-(3-methoxypropyl)-3,4-dihydro-2H-benzo[1,4]oxazin-6-ylmethoxy]-5-[(2-nitrooxyethylcarbamoyl)methoxy]-piperidine-1-carboxylate the title compound is identified by means of the Rf value.

The starting materials are prepared as follows:

### a) tert-Butyl (3R,4R,5S)-4-{4-[3-(2-chlorophenoxy)-propoxy]phenyl}-3-[4-(3-methoxypropyl)-3,4-di-hydro-2H-benzo[1,4]oxazin-6-ylmethoxyl-5-[(2-nitrooxyethylcarbamoyl)methoxy]piperidine-1-carboxylate

Analogously to method S, starting from tert-butyl (3S,4R,5R)-4-{4-[3-(2-chlorophenoxy)propoxy]phenyl}-3-[(2-methanesulphonyloxyethylcarbamoyl)methoxy]-5-[4-(3-methoxypropyl)-3,4-dihydro-2H-benzo[1,4]oxazin-6-yl-methoxy]piperidine-1-carboxylate the title compound is identified by means of the Rf value.

### b) tert-Butyl (3S,4R,5R)-4-{4-[3-(2-chlorophenoxy)-propoxy]phenyl}-3-[(2-methanesulphonyloxyethyl-carbamoyl)methoxy]-5-[4-(3-methoxypropyl)-3,4-dihydro-2H-benzo[1,4]oxazin-6-ylmethoxy]piperidine- 1-carboxylate

Analogously to method T, starting from tert-butyl (3S,4R,5R)-4-{4-[3-(2-chlorophenoxy)propoxy]phenyl}-3-[(2-hydroxyethylcarbamoyl)methoxy]-5-[4-(3-methoxypropyl)-3,4-dihydro-2H-benzo[1,4]oxazin-6-ylmethoxy]-piperidine-1-carboxylate the title compound is identified by means of the Rf value.

### c) tert-Butyl (3S,4R,5R)-4-{4-[3-(2-chlorophenoxy)-propoxy]phenyl}-3-[(2-hydroxyethylcarbamoyl)-methoxy]-5-[4-(3-methoxypropyl)-3,4-dihydro-2H-benzo[1,4]oxazin-6-ylmethoxy]piperidine-1 -carboxylate

Analogously to method U, starting from tert-butyl (3S,4R,5R)-3-carboxymethoxy-4-{4-[3-(2-chlorophenoxy)-propoxy]phenyl}-5-[4-(3-methoxypropyl)-3,4-dihydro-2H-benzo[1,4]oxazin-6-ylmethoxy]piperidine-1-carboxylate and 2-aminoethanol [141-43-5] the title compound is identified by means of the Rf value.

### d) tert-Butyl (3S,4R,5R)-3-carboxymethoxy-4-{4-[3-(2-chlorophenoxy)propoxy]phenyl}-5-[4-(3-methoxy-propyl)-3,4-dihydro-2H-benzo[1,4]oxazin-6-yl-methoxy]piperidine-1-carboxylate

A solution of 0.324 mmol of tert-butyl (3S,4R,5R)-4-{4-[3-(2-chlorophenoxy)propoxy]phenyl}-3-methoxycarbonyl-methoxy-5-[4-(3-methoxypropyl)-3,4-dihydro-2H-benzo-[1,4]oxazin-6-ylmethoxy]piperidine-1-carboxylate in 3 ml of tetrahydrofuran is treated with 38.880 mmol of 3M LiOH and stirred at room temperature for 1 hour. The mixture is adjusted to pH2 using 2M HCl and extracted with ethyl acetate (3X). The combined organic phases are dried using sodium sulphate and evaporated. The title compound is identified from the residue by means of the Rf value. The crude title compound is used in the next stage without further purification.

### e) tert-Butyl (3S,4R,5R)-4-{4-[3-(2-chlorolphenoxy)-prolpoxy]phenyl}-3-methoxycarbonylmethoxy-5-[4-(3-methoxypropyl)-3,4-dihydro-2H-benzo[1,4]oxazin-6-ylmethoxy]piperidine-1-carboxylate

A solution of 1.513 mmol of tert-butyl (3S,4S,5R)-4-{4-[3-(2-chlorophenoxy)propoxy]phenyl}-3-hydroxy-5-[4-(3-methoxypropyl)-3,4-dihydro-2H-benzo[1,4]oxazin-6-yl-methoxy]piperidine-1-carboxylate in 12 ml of N,N-dimethylformamide is treated with stirring with 1.993 mmol of sodium hydride (60% dispersion in oil). The reaction mixture is stirred at room temperature for 1 hour and then treated at -20°C with 6.132 mmol of methyl bromoacetate. After 1 hour at -20°C and 3 hours at room temperature, the reaction mixture is diluted with tert-butyl methyl ether and washed with saturated aqueous sodium hydrogencarbonate solution. The aqueous phase is extracted with tert-butyl methyl ether. The combined organic phases are dried using sodium sulphate and evaporated. The title compound is identified by means of the Rf value from the residue by means of flash chromatography (SiO₂ 60F).

### f) tert-Butyl (3S,4S,5R)-4-{4-[3-(2-chlorophenoxy)-propoxy]henyl}-3-hydroxy-5-[4-(3-methoxypropyl)-3,4-dihydro-2H-benzo[1,4]oxazin-6-ylmethoxy]-piperidine-1-carboxylate

Analogously to method R, starting from (3S,4S,5R)-4-{4-[3-(2-chlorophenoxy)propoxy]phenyl}-5-[4-(3-methoxy-propyl)-3,4-dihydro-2H-benzo[1,4]oxazin-6-ylmethoxy]-piperidin-3-ol [873945-66-5] the title compound is identified by means of the Rf value.

Alternative synthesis for tert-butyl (3R,4R,5S)-4-{4-[3-(2-chlorophenoxy)propoxy]phenyl}-3-[4-(3-methoxy-propyl)-3,4-dihydro-2H-benzo[1,4]oxazin-6-ylmethoxy]-5-[(2-nitrooxyethylcarbamoyl)methoxy]piperidine-1-carboxylate:

### a2) tert-Butyl (3R,4R,5S)-4{4-[3-(2-chlorophenoxy)-propoxy]phenyl}-3-[4-(3-methoxypropyl)-3,4-dihydro-2H-benzo[1,4]oxazin-6-ylmethoxy]-5-[(2-nitrooxyethylcarbamoyl)methoxy]piperidine-1-carboxylate

Analogously to method U, starting from tert-butyl (3S,4R,5R)-3-carboxymethoxy-4-{4-[3-(2-chlorophenoxy)-propoxy]phenyl}-5-[4-(3-methoxypropyl)-3,4-dihydro-2H-benzo[1,4]oxazin-6-ylmethoxy]piperidine-1-carboxylate (Example 26d) and 2-nitratoethylamine [646-02-6] the title compound is identified by means of the Rf value.

### Example 36

### 3-{(2S,4R,5R)-4-(4-Methoxyphenyl)-5-[4-(3-methoxy-propyl)-3,4-dihydro-2H-benzo[1,4]oxazin-6-ylmethoxy]-piperidin-2-yl}-2,2-dimethyl-N-(2-nitrooxyethyl)-propionamide

Analogously to method Q, starting from tert-butyl (2S,4R,5R)-4-(4-methoxyphenyl)-5-[4-(3-methoxypropyl)-3,4-dihydro-2H-benzo[1,4]oxazin-6-ylmethoxy]-2-[2-methyl-2-(2-nitrooxyethylcarbamoyl)propyl]piperidine-1-carboxylate the title compound is identified by means of the Rf value.

The starting materials are prepared as follows:

### a) tert-Butyl (2S,4R,5R)-4-(4-methoxyphenyl)-5-[4-(3-methoxypropyl)-3,4-dihydro-2H-benzo[1,4]oxazin-6-ylmethoxy]-2-[2-methyl-2-(2-nitrooxyethylcarbamoyl)propyl]piperidine-1-carboxylate

Analogously to method S, starting from tert-butyl (2S,4R,5R)-2-[2-(2-methane-sulphonyloxyethylcarbamoyl)-2-methylpropyl]-4-(4-methoxyphenyl)-5-[4-(3-methoxypropyl)-3,4-dihydro-2H-benzo[1,4]oxazin-6-ylmethoxy]-piperidine-1-carboxylate the title compound is identified by means of the Rf value.

### b) tert-Butyl (2S,4R,5R)-2-[2-(2-methanesulphonyloxy-ethylcarbamoyl)-2-methylpropyl]-4-(4-methoxy-phenyl)-5-[4-(3-methoxypropyl)-3,4-dihydro-2H-benzo[1,4]oxazin-6-ylmethoxy]piperidine-1-carboxylate

Analogously to method T, starting from tert-butyl (2S,4R,5R)-2-[2-(2-hydroxyethylcarbamoyl)-2-methyl-propyl]-4-(4-methoxyphenyl)-5-[4-(3-methoxypropyl)-3,4-dihydro-2H-benzo[1,4]oxazin-6-ylmethoxy]piperidine-1-carboxylate the title compound is identified by means of the Rf value.

### c) tert-Butyl (2S,4R,5R)-2-[2-(2-hydroxyethyl-carbamoyl)-2-methylprpil]-4-(4-methoxyphenyl)-5-[4-(3-methoxypropyl)-3,4-dihydro-2H-benzo[1,4]-oxazin-6-ylmethoxy]piperidine-1-carboxylate

Analogously to method R, starting from N-(2-hydroxy-ethyl)-3-{(2S,4R,5R)-4-(4-methoxyphenyl)-5-[4-(3-methoxypropyl)-3,4-dihydro-2H-benzo[1,4]oxazin-6-ylmethoxy]piperidin-2-yl}-2,2-dimethylpropionamide the title compound is identified by means of the Rf value.

### d) N-(2-Hydroxyethyl)-3-{(2S,4R,5R)-4-(4-methoxy-phenyl)-5-[4-(3-methoxypropyl)-3,4-dihydro-2H-benzo[1,4]oxazin-6-ylmethoxy]lpiperidin-2-yl}-2 2-dimethylpropionamide

Analogously to method L, starting from N-(2-hydroxy-ethyl)-3-[(2S,4R,5R)-4-(4-methoxyphenyl)-5-[4-(3-methoxypropyl)-3,4-dihydro-2H-benzo[1,4]oxazin-6-ylmethoxy]-1-(toluene-4-sulphonyl)piperidin-2-yl]-2,2-dimethylpropionamide the title compound is identified by means of the Rf value.

### e) N-(2-Hydroxyethyl)-3[(2S,4R,5R)-4-(4-methoxy-phenyl)-5-[4-(3-methoxypropyl)-3,4-dihydro-2H-benzo[1,4]oxazin-6-ylmethoxy]-1-(toluene-4-sulphonyl)piperidin-2-yl]-2,2-dimethylpropionamide

Analogously to method U, starting from 3-[(2S,4R,5R)-4-(4-methoxyphenyl)-5-[4-(3-methoxypropyl)-3,4-dihydro-2H-benzo[1,4]oxazin-6-ylmethoxy]-1-(toluene-4-sulphonyl)piperidin-2-yl]-2,2-dimethylpropionic acid [911706-32-6] and 2-aminoethanol [141-43-5] the title compound is identified by means of the Rf value.

Alternative synthesis for tert-butyl (2S,4R,5R)-4-(4-methoxyphenyl)-5-[4-(3-methoxypropyl)-3,4-dihydro-2H-benzo[1,4]oxazin-6-ylmethoxy]-2-[2-methyl-2-(2-nitrooxyethylcarbamoyl)propyl]piperidine-1-carboxylate:

### a2) tert-Butyl (3S,4R,5R)-3-((S)-2-hydroxy-3-nitrooxy-propoxy)-4-(4-methoxyphenyl)-5-[4-(3-methoxy-propyl)-3,4-dihydro-2H-benzo[1,4]oxazin-6-ylmethoxy]piperidine-1-carboxylate

Analogously to method U, starting from tert-butyl (3S,4R,5R)-3-carboxymethoxy-4-{4-[3-(2-chlorophenoxy)-propoxy]phenyl}-5-[4-(3-methoxypropyl)-3,4-dihydro-2H-benzo[1,4]oxazin-6-ylmethoxy]piperidine-1-carboxylate (Example 36d) and 2-nitratoethylamine [646-02-6] the title compound is identified by means of the Rf value.

### Example 37

### 6-[(3R,4R,6R)-4-[4-(2-Methoxyethoxymethyl)phenyl)-6-((R)-2-nitrooxypropyl)piperidin-3-yloxymethyl-4-(3-methoxypropyl)-3,4-dihydro-2H-benzo[1,4]oxazine

Analogously to method Q, starting from tert-butyl (2R,4R,5R)-4-[4-(2-methoxyethoxymethyl)phenyl]-5-[4-(3-methoxypropyl)-3,4-dihydro-2H-benzo[1,4]oxazin-6-ylmethoxy]-2-((R)-2-nitrooxypropyl)piperidine-1-carboxylate the title compound is identified by means of the Rf value.

The starting materials are prepared as follows:

### a) tert-Butyl (2R,4R,5R)-4-[4-(2-methoxyethoxy-methyl)phenyl]-5-[4-(3-methoxypropyl)-3,4-dihydro-2H-benzo[1,4]oxazin-6-ylmethoxy]-2-((R)-2-nitrooxypropyl)piperidine-1-carboxylate

Analogously to method S, starting from tert-butyl (2R,4R,5R)-2-((S)-2-methane-sulphonyloxypropyl)-4-[4-(2-methoxyethoxymethyl)phenyl]-5-[4-(3-methoxypropyl)-3,4-dihydro-2H-benzo[1,4]oxazin-6-ylmethoxy]piperidine-1-carboxylate the title compound is identified by means of the Rf value.

### b) tert-Butyl (2R,2R,5R)-2-((S)-2-methanesulphonyl-oxypropyl )-4-[4-(2-methoxyethoxymethyl)phenyl-5-[4-(3-methoxypropyl)-3,4-dihydro-2H-benzo[1,4]-oxazin-6-ylmethoxylpiperidine-1-carboxylate

Analogously to method T, starting from tert-butyl (2R,4R,5R)-2-((S)-2-hydroxypropyl)-4-[4-(2-methoxy-ethoxymethyl)phenyl]-5-[4-(3-methoxypropyl)-3,4-di-hydro-2H-benzo[1,4]oxazin-6-ylmethoxy]piperidine-1-carboxylate the title compound is identified by means of the Rf value.

### c) tert-Butyl (2R,4R,5R)-2-((S)-2-hydroxypropyl)-4-[4-(2-methoxyethoxymethyl)phenyl-5-[4-(3-methoxy-propyl)-3,4-dihydro-2H-benzo[1,4]oxazin-6-ylmethoxy]piperidine-1-carboxylate

Analogously to method R, starting from (S)-1-{(2R,4R,5R)-4-[4-(2-methoxyethoxymethyl)phenyl]-5-[4-(3-methoxypropyl)-3,4-dihydro-2H-benzo[1,4]oxazin-6-ylmethoxy]piperidin-2-yl}propan-2-ol the title compound is identified by means of the Rf value.

### d) (S)-1-{(2R,4R,5R)-4-[4-(2-Methoxyethoxymethyl)phenyl]-5-[4-(3-methoxypropyl)-3,4-dihydro-2H-benzo[1,4]oxazin-6-ylmethoxy]piperidin-2-yl}-propan-2-ol

Analogously to method L, starting from (S)-1-[(2R,4R,5R)-4-[4-(2-methoxyethoxymethyl)phenyl]-5-[4-(3-methoxypropyl)-3,4-dihydro-2H-benzo[1,4]oxazin-6-ylmethoxy]-1-(toluene-4-sulphonyl)piperidin-2-yl]propan-2-ol the title compound is identified by means of the Rf value.

### e) (R)-1-[(2R,4R,5R)-4-[4-(2-Methoxyethoxymethyl)-phenyl]-5-[4-(3-methoxypropyl)-3,4-dihydro-2H-benzo[1,4]oxazin-6-ylmethoxyl-1-(toluene-4-sulphonyl)piperidin-2-yl]propan-2-ol and (S)-1-[(2R,4R,5R)-4-[4-(2-methoxyethoxymethyl)-phenyl]-5-[4-(3-methoxypropyl)-3,4-dihydro-2H-benzo[1,4]oxazin-6-ylmethoxy]-1-(toluene-4-sulphonyl)piperidin-2-yl]propan-2-ol

Analogously to method K, the title compounds are obtained from 1-[(2R,4R,5R)-4-[4-(2-methoxyethoxy-methyl)phenyl]-5-[4-(3-methoxypropyl)-3,4-dihydro-2H-benzo[1,4]oxazin-6-ylmethoxy]-1-(toluene-4-sulphonyl)-piperidin-2-yl]propan-2-one. The diastereomers are separated by means of flash chromatography (SiO₂ 60F). Diastereomer 1; yellow oil; Rf = 0.30 (EtOAc-heptane 1:1); Rt = 4.80 (gradient I). Diastereomer 2: yellow oil; Rf = 0.21 (EtOAc-heptane 1:1); Rt = 4.71 (gradient I).

### f) 1-[(2R,4R,5R)-4-[4-(2-Methoxyethoxymethyl)phenyl]-5-[4-(3-methoxypropyl)-3,4-dihydro-2H-benzo[1,4]-oxazin-6-ylmethoxyl-1-(toluene-4-sulphonyl)-piperidin-2-yl]propan-2-one

11.684 mmol of a 3M solution of methylmagnesium bromide in tetrahydrofuran are added at 0°C to a solution of 5.842 mmol of N-methoxy-2-[(2R,4R,5R)-4-[4-(2-methoxy-ethoxymethyl)phenyl]-5-[4-(3-methoxypropyl)-3,4-di-hydro-2H-benzo[1,4]oxazin-6-ylmethoxy]-1-(toluene-4-sulphonyl)piperidin-2-yl]-N-methylacetamide [911707-53-4] in 50 ml of tetrahydrofuran. The reaction mixture is stirred at room temperature for 1 hour, treated with an aqueous, 1 M potassium hydrogen-sulphate solution and extracted with tert-butyl methyl ether (3X). The combined organic phases are dried using sodium sulphate and evaporated. The title compound is obtained from the residue as a brown oil. The title compound is used in the next stage without further purification. Rt = 4.93 (gradient I).

### g) N-Methoxy-2-[(2R,4R,5R)-4-[4-(2-methoxyethoxy-methyl)phenyl]-5-[4-(3-methoxypropyl)-3,4-dihydro-2H-benzo[1,4]oxazin-6-ylmethoxy]-1-(toluene-4-sulphonyl)piperidin-2-yl-N-methylacetamide

Analogously to method U, starting from [(2R,4R,5R)-4-[4-(2-methoxyethoxymethyl)phenyl]-5-[4-(3-methoxy-propyl)-3,4-dihydro-2H-benzo[1,4]oxazin-6-ylmethoxy]-1-(toluene-4-sulphonyl)piperidin-2-yl]acetic acid and N,O-dimethyl-hydroxyamine hydrochloride the title compound is obtained as a colourless oil. Rf = 0.31 (EtOAc); Rt = 4.77 (gradient I).

### h) [(2R,4R,5R)-4-[4-(2-Methoxyethoxymethyl)phenyl]-5-[4-(3-methoxypropyl)-3,4-dihydro-2H-benzo[1,4]-oxazin-6-ylmethoxyl-1-(toluene-4-sulphonyl)-piperidin-2-yl]lacetic acid

A solution of 39.539 mmol of [(2S,4R,5R)-4-[4-(2-methoxyethoxymethyl)phenyl]-5-[4-(3-methoxypropyl)-3,4-dihydro-2H-benzo[1,4]oxazin-6-ylmethoxy]-1-(toluene-4-sulphonyl)piperidin-2-yl]acetonitrile in 400 ml of ethanol and 400 ml of 2N NaOH is stirred overnight at 85°C. The reaction mixture is cooled to room temperature and acidified with 1 M HCl and extracted with ethyl acetate (3X). The combined organic phases are dried using sodium sulphate and evaporated. The title compound is obtained from the residue as a brown oil by means of flash chromatography (SiO₂ 60F). Rf = 0.42 (EtOAc-heptane 1:1); Rt = 4.58 (gradient I).

### i) [(2R,4R,5R)-4-[4-(2-Methoxyethoxymethyl)phenyl]-5-[4-(3-methoxypropyl)-3,4-dihydro-2H-benzo[1,4]-oxazin-6-ylmethoxy]-1-(toluene-4-sulphonyl)-piperidin-2-yl]acetonitrile

A solution of 46.750 mmol of (2S,4R,5R)-4-[4-(2-methoxyethoxymethyl)phenyl]-5-[4-(3-methoxypropyl)-3,4-dihydro-2H-benzo[1,4]oxazin-6-ylmethoxy]-1-(toluene-4-sulphonyl)piperidin-2-yl methanesulphonate in 300 ml of dimethyl sulphoxide is treated with 189.00 mmol of sodium cyanide and the reaction mixture is heated at 55°C for 4 hours. The reaction mixture is diluted with water and extracted with ethyl acetate (3X). The combined organic phases are dried using sodium sulphate and evaporated. The title compound is obtained from the residue as a brown oil by means of flash chromatography (SiO₂ 60F). Rf = 0.24 (EtOAc-heptane 2-1); Rt = 5.02 (gradient I).

### j) (2S,4R,5R)-4-[4-(2-Methoxyethoxymethyl)phenyl]-5-[4-(3-methoxypropyl)-3,4-dihydro-2H-benzo[1,4]-oxazin-6-ylmethoxy]-1-(toluene-4-sulphonyl)-piperidin-2-ylmethyl methanesulphonate

Analogously to method T, starting from [(2S,4R,5R)-4-[4-(2-methoxyethoxymethyl)phenyl]-5-[4-(3-methoxy-propyl)-3,4-dihydro-2H-benzo[1,4]oxazin-6-ylmethoxy]-1-(toluene-4-sulphonyl)piperidin-2-yl]methanol the title compound is obtained as a red resin. Rf = 0.21 (EtOAc-heptane 3:1); Rt = 4.99 (gradient I).

### k) [(2S,4R,5R)-4-[4-(2-Methoxyethoxymethyl)phenyl]-5-[4-(3-methoxypropyl)-3,4-dihydro-2H-benzo[1,4]-oxazin-6-ylmethoxyl-1-(toluene-4-sulphonyl)-piperidin-2-yl]methanol

Analogously to method J, starting from 6-[(3R,4R,6S)-4-[4-(2-methoxyethoxymethyl)phenyl]-1-(toluene-4-sulphonyl)-6-triisopropylsilanyloxymethylpiperidin-3-yloxymethyl]-4-(3-methoxypropyl)-3,4-dihydro-2H-benzo-[1,4]oxazine the title compound is obtained as a yellow oil. Rf = 0.13 (EtOAc-heptane 3:1); Rt = 4.76 (gradient I).

### l) 6-[(3R,4R,6S)-4-[4-(2-Methoxyethoxymethyl)phenyl]-1-(toluene-4-sulphonyl)-6-triisopropylsilanyloxy-methylpiperidin-3-yloxymethyl-4-(3-methoxyoropyl)-3,4-dihydro-2H-benzo[1,4]oxazine

Analogously to Example 4h, starting from {4-[(2S,4R,5R)-5-[4-(3-methoxypropyl)-3,4-dihydro-2H-benzo[1,4]oxazin-6-ylmethoxy]-1-(toluene-4-sulphonyl)-2-triisopropyl-silanyloxymethylpiperidin-4-yl]phenyl}-methanol the title compound is obtained as a yellow oil. Rf = 0.31 (EtOAc-heptane 1:1); Rt = 6.65 (gradient I).

### m) [4-[(2S,4R,5R)-5-[4-(3-Methoxypropyl)-3,4-dihydro-2H-benzo[1,4]oxazin-6-ylmethoxy]-1-(toluene-4-sulphonyl)-2-triisopropylsilanyloxymethyl-piperidin-4-yl]phenyl}methanol

Analogously to method K (stirring at 50°C), starting from 4-[(2S,4R,5R)-5-[4-(3-methoxypropyl)-3-oxo-3,4-di-hydro-2H-benzo[1,4]oxazin-6-ylmethoxy]-1-(toluene-4-sulphonyl)-2-triisopropylsilanyloxymethylpiperidin-4-yl]benzoic acid the title compound is obtained as a yellow oil. Rf = 0.19 (EtOAc-heptane 1:1); Rt = 6.03 (gradient I).

### n) 4-[(2S,4R,5R)-5-[4-(3-Methoxypropyl)-3-oxo-3,4-dihydro-2H-benzo[1,4]oxazin-6-ylmethoxy]-1-(toluene-4-sulphonyl)-2-triisopropylsilanyloxy-methylpiperidin-4-yl]benzoic acid

A solution of 40.971 mmol of methyl 4-[(2S,4R,5R)-5-[4-(3-methoxypropyl)-3-oxo-3,4-dihydro-2H-benzo[1,4]-oxazin-6-ylmethoxy]-1-(toluene-4-sulphonyl)-2-triiso-propylsilanyloxymethylpiperidin-4-yl]benzoate in 330 ml of tetrahydrofuran is treated with 177.818 mmol of 20% NaOH and stirred overnight at 85°C. The reaction mixture is partially evaporated and the residue is brought to pH 2 using 6M HCl and extracted with ethyl acetate (3X). The combined organic phases are dried using sodium sulphate and evaporated. The title compound is obtained from the residue as a white foam by means of flash chromatography (SiO₂ 60F). Rf = 0.20 (EtOAc-heptane 1:1); Rt = 6.30 (gradient I).

### o) Methyl 4-[(2S,4R,5R)-5-[4-(3-methoxyproipyl)-3-oxo-3,4-dihydro-2H-benzo[1,4]oxazin-6-ylmethoxyl-1-(toluene-4-sulphonyl)-2-triisopropylsilanyloxymethylpiperidin-4-yl]benzoate

Analogously to method K (with addition of 110 mol% of tetrabutylammonium iodide), starting from methyl 4-[(2S,4R,5R)-5-hydroxy-1-(toluene-4-sulphonyl)-2-triisopropylsilanyloxymethylpiperidin-4-yl]benzoate and 6-bromomethyl-4-(3-methoxypropyl)-4H-benzo[1,4]oxazin-3-one (Example 36t2) the title compound is obtained as a yellow oil. Rf = 0.50 (EtOAc-heptane 1:1); Rt = 6.74 (gradient I).

### p) Methyl 4-[(2S,4R,5R)-5-hydroxy-1-(toluene-4-sulphonyl)-2-triisopropylsilanyloxymethyl-piperidin-4-yl]benzoate

Analogously to Example 36m2, starting from methyl 4-[(2S,4R,5R)-5-hydroxy-2-hydroxymethyl-1-(toluene-4-sulphonyl)piperidin-4-yl]benzoate the title compound is obtained as a yellow oil. Rf = 0.45 (EtOAc-heptane 1:1); Rt = 6.46 (gradient I).

### q) Methyl 4-[(2S,4R,5R)-5-hydroxy-2-hydroxymethyl-1-(toluene-4-sulphonyl)piperidin-4-yl]benzoate

Analogously to Example 9j, starting from 4-[(2S,4R,5R)-5-hydroxy-2-hydroxymethyl-1-(toluene-4-sulphonyl)-piperidin-4-yl]phenyl trifluoromethanesulphonate the title compound is obtained as a dark yellow oil. Rf = 0.38 (EtOAc-heptane 1:1); Rt = 3.75 (gradient I).

### r) 4-[(2S,4R,5R)-5-hydroxy-2-hydroxymethyl-1-(toluene-4-sulphonyl)piperidin-4-yl]phenyl trifluoromethanesulphonate

Analogously to Example 9k, starting from (3R,4R,6S)-6-hydroxymethyl-4-(4-hydroxyphenyl)-1-(toluene-4-sulphonyl)piperidin-3-ol the title compound is obtained as a white solid. Rf = 0.27 (EtOAc-heptane 1:1); Rt = 4.46 (gradient I).

### s) (3R,4R,6S)-6-Hydroxymethyl-4-(4-hydroxyphenyl)-1-(toluene-4-sulphonyl)piperidin-3-ol

Analogously to Example 20c, starting from (3R,4R,6S)-6-hydroxymethyl-4-(4-methoxyphenyl)-1-(toluene-4-sulphonyl)piperidin-3-ol (Example 36n2) the title compound is obtained as a white solid. Rf = 0.20 (EtOAc-heptane 2:1); Rt = 3.22 (gradient I).

According to the process described in Example 37, the following compounds are prepared in an analogous manner:

### Examples

### 38 6-[(3R,4R,6R)-4-[4-(2-Methoxyethoxymethyl)phenyl]-6-((S)-2-nitrooxypropyl)piperidin-3-yloxymethyl]-4-(3-methoxypropyl)-3,4-dihydro-2H-benzo[1,4]oxazine

starting from (R)-1-[(2R,4R,5R)-4-[4-(2-methoxyethoxy-methyl)phenyl]-5-[4-(3-methoxypropyl)-3,4-dihydro-2H-benzo[1,4]oxazin-6-ylmethoxy]-1-(toluene-4-sulphonyl)-piperidin-2-yl]propan-2-ol (Example 37e).

### 39 6-[(3R,4R,6R)-4-[4-(3-methoxypropoxy)phenyl]-6-((R)-2-nitrooxypropyl)piperidin-3-yloxymethyl]-4-(3-methoxypropyl)-3,4-dihydro-2H-benzo[1,4]oxazine

starting from (3R,4R,6S)-6-hydroxymethyl-4-[4-(3-methoxypropoxy)phenyl]-1-(toluenesulphonyl)piperidin-3-ol.

The starting material is prepared as follows:

### a) (3R,4R,6S)-6-Hydroxymethyl-4-[4-(3-methoxy-propoxy)phenyl]-1-(toluenesulphonyl)piperidin-3-ol

Analogously to method F, starting from (3R,4R,6S)-6-hydroxymethyl-4-(4-hydroxyphenyl)-1-(toluene-4-sulphonyl)piperidin-3-ol [911707-37-4] and 3-methoxypropyl chloride the title compound is identified by means of the Rf value.

### 40 6-[(3R,4R,6R)-4-[4-(3-Methoxypropoxy)phenyl]-6-((S)-2-nitrooxypropyl)piperidin-3-yloxymethyl]-4-(3-methoxypropyl-3,4-dihydro-2H-benzo[1,4]oxazine

starting from (3R,4R,6S)-6-hydroxymethyl-4-[4-(3-methoxypropoxy)phenyl]-1-(toluenesulphonyl)piperidin-3-ol (Example 39a).

### 42 6-[(3R,4R,6S)-4-[4-(2-methoxyethoxymethyl)phenyl]-6-((R)-2-nitrooxypropyl)piperidin-3-yloxymethyl]-4-(3-methoxypropyl)-3,4-dihydro-2H-benzo[1,4]-oxazine

starting from (R)-1-[(2S,4R,5R)-4-[4-(2-methoxyethoxy-methyl)phenyl]-5-[4-(3-methoxypropyl)-3,4-dihydro-2H-benzo[1,4]oxazin-6-ylmethoxy]-1-(toluene-4-sulphonyl)-piperidin-2-ylpropan-2-ol.

The starting materials are prepared as follows:

### a) (R)-1-[(2S,4R,5R)-4-[4-(2-Methoxyethoxymethyl)-phenyl]-5-[4-(3-methoxypropyl)-3,4-dihydro-2H-benzo[1,4]oxazin-6-ylmethoxyl-1-(toluene-4-sulphonyl)piperidin-2-yl]propan-2-ol and (S)-1-[(2S,4R,5R)-4-[4-(2-methoxyethoxymethyl)-phenyl]-5-[4-(3-methoxypropyl)-3,4-dihydro-2H-benzo[1,4]oxazin-6-ylmethoxy]-1-(toluene-4-sulphonyl)piperidin-2-yl]propan-2-ol

Analogously to Example 37f, the title compounds are obtained from [(2S,4R,5R)-4-[4-(2-methoxyethoxy-methyl)phenyl]-5-[4-(3-methoxypropyl)-3,4-dihydro-2H-benzo[1,4]oxazin-6-ylmethoxy]-1-(toluene-4-sulphonyl)-piperidin-2-yl]acetaldehyde. The diastereomers are separated by means of flash chromatography (SiO₂ 60F) and identified by means of their Rf values.

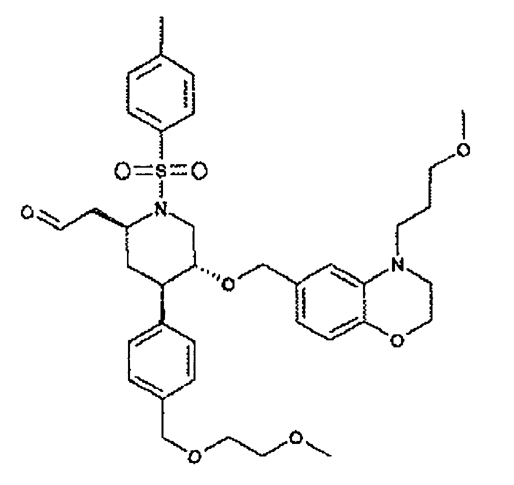

### b) [(2S,4R,5R)-4-[4-(2-Methoxyethoxymethyl)phenyl]-5-[4-(3-methoxypropyl)-3,4-dihydro-2H-benzo[1,4]-oxazin-6-ylmethoxy]-1-(toluene-4-sulphonyl)-piperidin-2-yl]acetaldehyde

A solution of 1.342 mmol of [(2S,4R,5R)-4-[4-(2-methoxyethoxymethyl)phenyl]-5-[4-(3-methoxypropyl)-3,4-dihydro-2H-benzo[1,4]oxazin-6-ylmethoxy]-1-(toluene-4-sulphonyl)piperidin-2-yl]acetonitrile in 10 ml of dichloromethane are treated at -30°C with 2.684 mmol of diisobutylaluminium hydride (1.0M solution in dichloromethane). The reaction mixture is warmed to -10°C over 3 hours. It is quenched with 1 M HCl and warmed to 0°C. It is extracted with dichloromethane (3X). The combined organic phases are washed with brine, dried using sodium sulphate and evaporated. The title compound is identified by means of the Rf value from the residue by means of flash chromatography (SiO₂ 60F).

### c) [(2S,4R,5R)-4-[4-(2-Methoxyethoxymethyl)phenyl]-5-[4-(3-methoxypropyl)-3,4-dihydro-2H-benzo[1,4]-oxazin-6-ylmethoxyl-1-(toluene-4-sulphonyl)-piperidin-2-yl]acetonitrile

A solution of 46.750 mmol of (2S,4R,5R)-4-[4-(2-methoxyethoxymethyl)phenyl]-5-[4-(3-methoxypropyl)-3,4-dihydro-2H-benzo[1,4]oxazin-6-ylmethoxy]-1-(toluene-4-sulphonyl)piperidin-2-ylmethyl methanesulphonate [911707-28-3] in 300 ml of dimethyl sulphoxide is treated with 189.00 mmol of sodium cyanide and the reaction mixture is heated at 55°C for 4 hours. The reaction mixture is diluted with water and extracted with ethyl acetate (3X). The combined organic phases are dried with sodium sulphate and evaporated. The title compound is obtained from the residue as a brown oil by means of flash chromatography (SiO₂ 60F). Rf = 0.24 (EtOAc-heptane 2:1); Rt = 5.02 (gradient I).

### 44 6-[(3R,4R,6R)-4-(4-Methoxyphenyl)-6-((R)-2-nitrooxypropyl)piperidin-3-yloxymethyl-4-(3-methoxypropyl)-3,4-dihydro-2H-benzo[1,4]oxazine

Starting from (2S,4R,5R)-4-(4-methoxyphenyl)-5-[4-(3-methoxypropyl)-3,4-dihydro-2H-benzo[1,4]oxazin-6-yl-methoxy]-1-(toluene-4-sulphonyl)piperidin-2-ylmethyl methanesulphonate [911705-65-2]

### Example 41

### N-{(2R,4R,5R)-4-[4-(2-Methoxyethoxymethyl)phenyl]-5-[4-(3-methoxypropyl)-3,4-dihydro-2H-benzo[1,4]oxazin-6-yl-methoxylpiperidin-2-ylmethyl}-2-nitrooxyacetamide

Analogously to the processes described in Example 10, 10a, 10b and 10c, starting from 2-hydroxy-N-{(2R,4R,5R)-4-[4-(2-methoxyethoxymethyl)phenyl]-5-[4-(3-methoxypropyl)-3,4-dihydro-2H-benzo[1,4]oxazin-6-ylmethoxy]piperidin-2-ylmethyl}acetamide the title compound is identified by means of the Rf value.

The starting materials are prepared as follows:

### a) 2-Hydroxy-N-{(2R,4R,5R)-4-[4-(2-methoxyethoxy-methyl)phenyl]-5-[4-13-methoxypropyl)-3,4-dihydro-2H-benzo[1,4]oxazin-6-ylmethoxylpiperidin-2-ylmethyl}acetamide

Analogously to method L, starting from 2-hydroxy-N-[(2R,4R,5R)-4-[4-(2-methoxyethoxymethyl)phenyl]-5-[4-(3-methoxypropyl)-3,4-dihydro-2H-benzo[1,4]oxazin-6-ylmethoxy]-1-(toluene-4-sulphonyl)piperidin-2-ylmethyl]-acetamide the title compound is identified by means of the Rf value.

### b) 2-Hydroxy-N-[(2R,4R,5R)-4-[4-(2-methoxyethoxy-methyl)phenyl]-5-[4-(3-methoxypropyl)-3,4-dihydro-2H-benzo[1,4]oxazin-6-ylmethoxyl-1-(toluene-4-sulphonyl)piperidin-2-ylmethyl]acetamide

Analogously to method J, starting from 2-(tert-butyl-dimethylsilanyloxy)-N-[(2R,4R,5R)-4-[4-(2-methoxy-ethoxymethyl)phenyl]-5-[4-(3-methoxypropyl)-3,4-dihydro-2H-benzo[1,4]oxazin-6-ylmethoxy]-1-(toluene-4-sulphonyl)piperidin-2-ylmethyl]acetamide the title compound is identified by means of the Rf value.

### c) 2-(tert-Butyldimethylsilanyloxy)-N-[(2R,4R,5R)-4-[4-(2-methoxyethoxymethyl)phenyl]-5-[4-(3-methoxy-propyl)-3,4-dihydro-2H-benzo[1,4]oxazin-6-ylmethoxy]-1-(toluene-4-sulphonyl)piperidin-2-ylmethyl]acetamide

Analogously to Example 10f, starting from C-[(2R,4R,5R)-4-[4-(2-methoxyethoxymethyl)phenyl]-5-[4-(3-methoxypropyl)-3,4-dihydro-2H-benzo[1,4]oxazin-6-ylmethoxy]-1-(toluene-4-sulphonyl)piperidin-2-yl]-methylamine the title compound is identified by means of the Rf value.

### d) C-[(2R,4R,5R)-4-[4-(2-Methoxyethoxymethyl)phenyl]-5-[4-(3-methoxypropyl)-3,4-dihydro-2H-benzo[1,4]-oxazin-6-ylmethoxy]-1-(toluene-4-sulphonyl)-piperidin-2-yl]methylamine

Analogously to method B (solvent: tetrahydrofuran-methanol 1:10), starting from benzyl [(2R,4R,5R)-4-[4-(2-methoxyethoxymethyl)phenyl]-5-[4-(3-methoxypropyl)-3,4-dihydro-2H-benzo[1,4]oxazin-6-ylmethoxy]-1-(toluene-4-sulphonyl)piperidin-2-ylmethyl]carbamate the title compound is identified by means of the Rf value.

### e) Benzyl [(2R,4R,5R)-4-[4-(2-methoxyethoxymethyl)-phenyl]-5-[4-(3-methoxypropyl)-3,4-dihydro-2H-benzo[1,4]oxazin-6-ylmethoxy]-1-(toluene-4-sulphonyl)piperidin-2-ylmethyl]carbamate

A solution of 1 mmol of 6-[(3R,4R,6R)-6-isocyanato-methyl-4-[4-(2-methoxyethoxymethyl)phenyl]-1-(toluene-4-sulphonyl)piperidin-3-yloxymethyl]-4-(3-methoxy-propyl)-3,4-dihydro-2H-benzo[1,4]oxazine in 50 mmol of benzyl alcohol is heated at 115°C overnight. The title compound is identified by means of the Rf value from the reaction mixture by means of flash chromatography (SiO₂ 60F).

### f) 6-[(3R,4R,6R)-6-isocyanatomethyl-4-[4-(2-methoxy-ethoxymethyl)phenyl]-1-(toluene-4-sulphonyl)-piperidin-3-yloxymethyl]-4-(3-methoxypropyl)-3,4-dihydro-2H-benzo[1,4]oxazine

A solution of 1 mmol of [(2R,4R,5R)-4-[4-(2-methoxy-ethoxymethyl)phenyl]-5-[4-(3-methoxypropyl)-3,4-di-hydro-2H-benzo[1,4]oxazin-6-ylmethoxy]-1-(toluene-4-sulphonyl)piperidin-2-yl]acetic acid [911707-54-5] and 2.5 mmol of triethylamine in 10 ml of tetrahydrofuran is treated at 0°C with 2 mmol of ethyl chloroacetate. The reaction mixture is stirred at 0°C for 1 hour. A solution of 20 mmol of sodium azide in 5 ml of water is added dropwise at 0°C and the mixture is subsequently stirred at 0°C for 1 hour. The reaction mixture is diluted with ethyl acetate and a little water and the aqueous phase is extracted with ethyl acetate (2X). The combined organic phases are washed with brine, dried with sodium sulphate and evaporated. The residue is dissolved in 10 ml of toluene, heated at 115°C for 1.5 hours and evaporated. The title compound is identified from the residue by means of the Rf value. The title compound is used in the next stage without further purification.

According to the process described in Example 41, the following compound is prepared in an analogous manner:

### Example

### 45 N-{(2R,4R,5R)-4-(4-Methoxyphenyl)-5-[4-(3-methoxy-propyl)-3,4-dihydro-2H-benzo[1,4]oxazin-6-yl-methoxylpiperidin-2-ylmethyl}-2-nitrooxyacetamide

Starting from [(2R,4R,5R)-4-(4-methoxyphenyl)-5-[4-(3-methoxypropyl)-3,4-dihydro-2H-benzo[1,4]oxazin-6-yl-methoxy]-1-(toluene-4-sulphonyl)piperidin-2-yl]acetic acid [911705-63-0].

### Example 46

### 3-{(35,45,5R)-4-(4-Methoxyphenyl)-5-[4-(3-methoxy-propyl)-3,4-dihydro-2H-benzo[1,4]oxazin-6-ylmethoxy]-piperidin-3-yloxy}propyl 6-nitrooxyhexanoate

Analogously to method Q, starting from tert-butyl (3R,4S,5S)-4-(4-methoxyphenyl)-3-[4-(3-methoxypropyl)-3,4-dihydro-2H-benzo[1,4]oxazin-6-ylmethoxy]-5-[3-(6-nitrooxyhexanoyloxy)propoxy]piperidine-1-carboxylate the title compound is identified by means of the Rf value.

The starting materials are prepared as follows:

### a) tert-Butyl(3R,4S,5S)-4-(4-methoxyphenyl)-3-[4-(3-methoxypropyl)-3,4-dihydro-2H-benzo[1,4]oxazin-6-ylmethoxyl-5-[3-(6-nitrooxyhexanoyloxy)propoxy]-piperidine-1-carboxylate

Analogously to method X, starting from tert-butyl (3S,4S,5R)-3-(3-hydroxypropoxy)-4-(4-methoxyphenyl)-5-[4-(3-methoxypropyl)-3,4-dihydro-2H-benzo[1,4]oxazin-6-ylmethoxy]piperidine-1-carboxylate (Example 4c) and pentafluorophenyl 6-nitrooxyhexanoate the title compound is identified by means of the Rf value.

### b) Pentafluorophenyl 6-nitrooxyhexanoate

Analogously to method Y, starting from pentafluoro-phenyl 6-bromohexanoate [816464-83-2] the title compound is identified by means of the Rf value.

According to the process described in Example 46, the following compounds are prepared in an analogous manner:

### Examples

### 47 (S)-2-{(3S,4R,5R)-[4-(2-Methoxyethoxymethyl-phenyl]-5-[4-(3-methoxypropyl)-3,4-dihydro-2H-benzo[1,4]oxazin-6-ylmethoxylpiperidin-3-yloxy}-1-methylethyl 5-nitrooxypentanoate

starting from tert-butyl (3S,4R,5R)-3-((S)-2-hydroxy-propoxy)-4-[4-(2-methoxyethoxymethyl)phenyl]-5-[4-(3-methoxypropyl)-3,4-dihydro-2H-benzo[1,4]oxazin-6-ylmethoxy]piperidine-1*-carboxylate and pentafluorophenyl 6-nitrooxypentanoate [874446-94-3].

The starting material is prepared as follows:

### a) tert-Butyl (3S,4R,5R)-3-((S)-2-hydroxylpropoxy)-4-[4-(2-methoxyethoxymethyl)phenyl]-5-[4-(3-methoxy-propyl)-3,4-dihydro-2H-benzo[1,4]oxazin-6-ylmethoxy]piperidine-1-carboxylate

Analogously to method R, starting from (S)-1-{(3S,4R,5R)-4-[4-(2-methoxyethoxymethyl)phenyl]-5-[4-(3-methoxypropyl)-3,4-dihydro-2H-benzo[1,4]oxazin-6-ylmethoxy]piperidin-3-yloxy}propan-2-ol [911854-73-4] the title compound is identified by means of the Rf value.

### 48 ({(3S,4R,5R)-4-(4-Methoxyphenyl)-5-[4-(3-methoxy-propyl)-3,4-dihydro-2H-benzo[1,4]oxazin-6-ylmethoxy]piperidin-3-ylmethyl}carbamoyl)methyl 4-nitrooxybutanoate

starting from tert-butyl (3R,4R,5R)-3-[(2-hydroxy-acetylaminomethyl]-4-(4-methoxyphenyl)-5-[4-(3-methoxy-propyl)-3,4-dihydro-2H-benzo[1,4]oxazin-6-ylmethoxy]-piperidine-1-carboxylate (Example 10c) and pentafluoro-phenyl 6-nitrooxybutanoate [838878-70-9].

### 52 (R)-2-{(3S,4R,5R)-4-[4-(4-Methoxybutoxy)-phenyl]-5-[4-(3-methoxypropyl)-3,4-dihydro-2H-benzo[1,4]oxazin-6-ylmethoxylpiperidin-3-yloxy}-1-methylethyl 4-nitrooxymethylbenzoate

starting from tert-butyl (3S,4R,5R)-3-((R)-2-hydroxy-propoxy)-4-[4-(4-methoxybutoxy)phenyl]-5-[4-(3-methoxy-propyl)-3,4-dihydro-2H-benzo[1,4]oxazin-6-ylmethoxy]-piperidine-1-carboxylate and pentafluorophenyl 4-nitrooxymethylbenzoate [874446-96-5].

The starting material is prepared as follows:

### a) tert-Butyl (3S,4R,5R)-3-((R)-2-hydroxypropoxy)-4-[4-(4-methoxybutoxy)phenyl]-5-[4-(3-methoxy-propyl)-3,4-dihydro-2H-benzo[1,4]oxazin-6-ylmethoxy]piperidine-1-carboxylate

Analogously to the processes described in Examples 2a, 2b, 2c, 2d and 2e, starting from benzyl (3S,4S,5R)-3-hydroxy-4-[4-(4-methoxybutoxy)phenyl]-5-[4-(3-methoxypropyl)-3,4-dihydro-2H-benzo[1,4]oxazin-6-ylmethoxy]-piperidine-1-carboxylate (Example 11 a) the title compound is identified by means of the Rf value.

### 60 (3S,4R,SR)-4-{4-[(S)-1-(3-Fluorophenyl)-pyrrolidin-3-yloxylphenyl}-5-[4-(3-methoxypropyl)-3,4-dihydro-2H-benzo[1,4]oxazin-6-ylmethoxyl-piperidin-3-yl 4-nitrooxymethylbenzoate

starting from tert-butyl (3S,4S,5R)-4-{4-[(S)-1-(3-fluorophenyl)pyrrolidin-3-yloxy]phenyl}-3-hydroxy-5-[4-(3-methoxypropyl)-3,4-dihydro-2H-benzo[1,4]oxazin-6-yl-methoxy]piperidine-1-carboxylate and pentafluorophenyl 4-nitrooxymethylbenzoate [874446-96-5].

The starting material is prepared as follows:

### a) tert-Butyl (3S,4S,5R)-4-{4-[(S)-1-(3-fluoro-phenyl)pyrrolidin-3-yloxy]phenyl}-3-hydroxy-5-[4-(3-methoxypropyl)-3,4-dihydro-2H-benzo[1,4]oxazin-6-ylmethoxylpiperidine-1-carboxylate

Analogously to method R, starting from (3S,4S,5R)-4-{4-[(S)-1-(3-fluorophenyl)pyrrolidin-3-yloxy]phenyl}-5-[4-(3-methoxypropyl)-3,4-dihydro-2H-benzo[1,4]oxazin-6-ylmethoxy]piperidin-3-ol [873945-18-7] the title compound is identified by means of the Rf value.

### Example 49

### (3S,4R,5R)-4-[4-(2-Methoxyethoxymethyl)phenyl]-5-[4-(3-methoxypropyl)-3,4-dihydro-2H-benzo[1,4]oxazin-6-ylmethoxylpiperidin-3-ylmethyl 4-nitrooxybutyl carbonate

Analogously to method Q, starting from tert-butyl (3R,4R,5S)-4-[4-(2-methoxyethoxymethyl)phenyl]-3-[4-(3-methoxypropyl)-3,4-dihydro-2H-benzo[1,4]oxazin-6-ylmethoxy]-5-(4-nitrooxybutoxycarbonyloxymethyl)-piperidine-1-carboxylate the title compound is identified by means of the Rf value.

The starting materials are prepared as follows:

### a) tert-Butyl (3R,4R,5S)-4-[4-(2-methoxyethoxy-methyl)phenyl]-3-[4-(3-methoxypropyl)-3,4-dihydro-2H-benzo[1,4]-oxazin-6-ylmethoxyl-5-(4-nitrooxybutoxycarbonyloxymethyl)piperidine-1-carboxylate

Analogously to method Z, starting from tert-butyl (3S,4R,5R)-3-hydroxymethyl-4-[4-(2-methoxyethoxy-methyl)phenyl]-5-[4-(3-methoxypropyl)-3,4-dihydro-2H-benzo[1,4]oxazin-6-ylmethoxy]piperidine-1-carboxylate and 4-nitrooxybutyl 4-nitrophenyl carbonate [935472-60-9] the title compound is identified by means of the Rf value.

### b) tert-Butyl (3S,4R,5R)-3-hydroxymethyl-4-[4-(2-methoxyethoxymethyl)phenyl]-5-[4-(3-methoxy-propyl)-3,4-dihydro-2H-benzo[1,4]oxazin-6-yl-methoxypiperidine-1-carboxylate

Analogously to the processes described in Examples 3c, 3d, 3e, 3f and 3g, starting from (3R,4R,5S)-1-benzyl-4-[4-(2-methoxyethoxymethyl)phenyl]-5-trityloxymethyl-piperidin-3-ol (Example 22a) the title compound is identified by means of the Rf value.

According to the process described in Example 49, the following compounds are prepared in an analogous manner:

### Examples

### 50 ({(3S,4R,5R)-4-[4-((S)-3-Methoxy-2-methylpropoxy-methyl)phenyl]-5-[4-(3-methoxypropyl)-3,4-dihydro-2H-benzo[1,4]oxazin-6-ylmethoxy]piperidin-3-ylmethyl}carbamoyl)methyl 3-nitrooxypropyl carbonate

starting from tert-butyl (3R,4R,5R)-3-[(2-hydroxy-acetylamino)methyl]-4-[4-((S)-3-methoxy-2-methyl-propoxymethyl)phenyl]-5-[4-(3-methoxypropyl)-3,4-dihydro-2H-benzo[1,4]oxazin-6-ylmethoxy]piperidine-1-carboxylate and 4-nitrooxypropyl 4-nitrophenyl carbonate

The starting materials are prepared as follows:

### a) tert-Butyl (3R,4R,5R)-3-[(2-hydroxyacetylamino)-methyl]-4-14-((S)-3-methoxy-2-methylpropoxymethyl)-phenyl]-5-14-(3-methoxypropyl)-3,4-dihydro-2H-benzo[1,4]oxazin-6-ylmethoxy]piperidine-1-carboxylate

Analogously to the processes described in Examples 10c, 10d, 10e, 10f, 10g, 10h and 10i, starting from benzyl (3S,4R,5R)-3-hydroxymethyl-4-[4-((S)-3-methoxy-2-methylpropoxymethyl)phenyl]-5-[4-(3-methoxypropyl)-3,4-dihydro-2H-benzo[1,4]oxazin-6-ylmethoxy]piperidine-1-carboxylate (Example 28a) the title compound is identified by means of the Rf value.

### b) 4-Nitrooxypropyl 4-nitrophenyl carbonate

Analogously to method AA, starting from 3-nitrooxy-propan-l-ol [100502-66-7] the title compound is identified by means of the Rf value.

### 51 (35,4R,5R)-4-{4-[(S)-1-(3-Fluorophenyl)-pyrrolidin-3-yloxylphenyl}-5-[4-(3-methoxypropyl)-3,4-dihydro-2H-benzo[1,4]oxazin-6-ylmethoxy]-piperidin-3-yl2-(2-nitrooxyethoxy)ethyl carbonate

starting from tert-butyl (3S,4S,5R)-4-{4-[(S)-1-(3-fluorophenyl)pyrrolidin-3-yloxy]phenyl}-3-hydroxy-5-[4-(3-methoxypropyl)-3,4-dihydro-2H-benzo[1,4]oxazin-6-yl-methoxy]piperidine-1-carboxylate (Example 60a) and 2-(2-nitrooxyethoxy)ethyl 4-nitrophenyl carbonate

The starting material is prepared as follows:

### a) 2-(2-Nitrooxyethoxy)ethyl 4-nitrophenyl carbonate

Analogously to method AA, starting from 2-(2-nitrooxy-ethoxy)ethanol [20633-16-3] the title compound is identified by means of the Rf value.

### 54 ({(2R,4R,5R)-4-[4-(2-Methoxyethoxymethyl)phenyl]-5-[4-(3-methoxypropyl)-3,4-dihydro-2H-benzo[1,4]-oxazin-6-ylmethoxylpiperidin-2-ylmethyl}methylcarbamoyl)methyl 3-nitrooxymethylphenyl carbonate

starting from tert-butyl (2R,4R,5R)-2-{[(2-hydroxy-acetyl)methylamino]methyl}-4-[4-(2-methoxyethoxy-methyl)phenyl]-5-[4-(3-methoxypropyl)-3,4-dihydro-2H-benzo[1,4]oxazin-6-ylmethoxy]piperidine-1-carboxylate and 3-nitrooxymethylphenyl 4-nitrophenyl carbonate [874447-03-7].

The starting material is prepared as follows:

### a) tert-Butyl (2R,4R,5R)-2-{[(2-hydroxyacetyl)methyl-amino]methyl}-4-[4-(2-methoxyethoxymethyl)phenyl]-5-[4-(3-methoxypropyl)-3,4-dihydro-2H-benzo[1,4]-oxazin-6-ylmethoxylpiperidine-1-carboxylate

Analogously to method R, starting from 2-hydroxy-N-{(2R,4R,5R)-4-[4-(2-methoxyethoxymethyl)phenyl]-5-[4-(3-methoxypropyl)-3,4-dihydro-2H-benzo[1,4]oxazin-6-ylmethoxy]piperidin-2-ylmethyl}acetamide (Example 41 a) the title compound is identified by means of the Rf value.

### 56 (R)-1-Methoxymethyl-2-{(3S,4R,5R)-4-[4-((S)-3-methoxy-2-methylpropoxymethyl)phenyl]-5-[4-(3-methoxyoropyl)-3,4-dihydro-2H-benzo[1,4]oxazin-6-ylmethoxy]piperidin-3-yloxylethyl1-nitrooxyethyl carbonate

starting from tert-butyl (3S,4R,5R)-3-((R)-2-hydroxy-3-methoxypropoxy)-4-[4-((S)-3-methoxy-2-methylpropoxy-methyl)phenyl]-5-(4-(3-methoxypropyl)-3,4-dihydro-2H-benzo[1,4]oxazin-6-ylmethoxy]piperidine-1-carboxylate and 1-nitrooxyethyl 4-nitrophenyl carbonate.

The starting materials are prepared as follows:

### a) tert-Butyl (3S,4R,5R)-3-((R)-2-hydroxy-3-methoxy-propoxy)-4-[4-((S)-3-methoxy-2-methylpropoxy-methyl)phenyl]-5-[4-(3-methoxypropyl)-3,4-dihydro-2H-benzo[1,4]oxazin-6-ylmethoxylpiperidine-1-carboxylate

Analogously to method R, starting from (R)-1-methoxy-3-{(3S,4R,5R)-4-[4-((S)-3-methoxy-2-methylpropoxymethyl)-phenyl]-5-[4-(3-methoxypropyl)-3,4-dihydro-2H-benzo- [1,4]oxazin-6-ylmethoxy]piperidin-3-yloxy}propan-2-ol [911854-52-9] the title compound is identified by means of the Rf value.

### b) 1-Nitrooxyethyl 4-nitrophenyl carbonate

Analogously to method Y, starting from 1-chloroethyl 4-nitrophenyl carbonate [101623-69-2] the title compound is identified by means of the Rf value.

### 61 (3S,4R,5R)-4-{4-[(S)-1-(3-Fluorophenyl)-pyrrolidin-3-yloxy]phenyl}-5-[4-(3-methoxypropyl)-3,4-dihydro-2H-benzo[1,4]oxazin-6-ylmethoxy]-piperidin-3-yl 1-nitrooxyethyl carbonate

starting from tert-butyl (3S,4S,5R)-4-{4-[(S)-1-(3-fluorophenyl)pyrrolidin-3-yloxy]phenyl}-3-hydroxy-5-[4-(3-methoxypropyl)-3,4-dihydro-2H-benzo[1,4]oxazin-6-ylmethoxy]piperidine-1-carboxylate (Example 60a) and 1-nitrooxyethyl 4-nitrophenyl carbonate (Example 56b).

### Example 53

### 1-Nitrooxyethyl (3-{(2R,4R,5R)-4-[4-(2-methoxyethoxy-methyl)phenyl]-5-[4-(3-methoxypropyl)-3,4-dihydro-2H-benzo[1,41oxazin-6-ylmethoxy]piperidin-2-yl}-2,2-dimethylpropionyl)methylcarbamate

Analogously to method Q, starting from tert-butyl (2R,4R,5R)-2-[2,2-dimethyl-3-[methyl-(1-nitrooxyethoxy-carbonyl)amino]-3-oxopropyl]-4-[4-(2-methoxyethoxy-methyl)phenyl]-5-[4-(3-methoxypropyl)-3,4-dihydro-2H-benzo-[1,4]oxazin-6-ylmethoxy]piperidine-1-carboxylate the title compound is identified by means of the Rf value.

The starting materials are prepared as follows:

### a) tert-Butyl (2R,4R,5R)-2-[2,2-dimethyl-3-[methyl-(1-nitrooxyethoxycarbonyl)amino]-3-oxopropyl]-4-[4-(2-methoxyethoxymethyl)phenyl]-5-[4-(3-methoxy-propyl)-3,4-dihydro-2H-benzo[1,4]oxazin-6-yl-methoxylpiperidine-1-carboxylate

Analogously to method Y, starting from tert-butyl (2R,4R,5R)-2-{3-[(1-chloroethoxycarbonyl)methylamino]-2,2-dimethyl-3-oxopropyl}-4-[4-(2-methoxyethoxymethyl)-phenyl]-5-[4-(3-methoxypropyl)-3,4-dihydro-2H-benzo-[1,4]oxazin-6-ylmethoxy] piperidine-1-carboxylate the title compound is identified by means of the Rf value.

### b) tert-Butyl (2R,4R,5R)-2-{3-[(1-chloroethoxy-carbonyl)methylaminol-2,2-dimethyl-3-oxopropyl}-4-[4-(2-methoxyethoxymethyl)phenyl]-5-[4-(3-methoxypropyl)-3,4-dihydro-2H-benzo[1,4]oxazin-6-ylmethoxylpiperidine-1-carboxylate

A solution of 1 mmol of tert-butyl (2R,4R,5R)-4-[4-(2-methoxyethoxymethyl)phenyl]-5-[4-(3-methoxypropyl)-3,4-dihydro-2H-benzo[1,4]oxazin-6-ylmethoxy]-2-(2-methyl-2-methylcarbamoylpropyl)piperidine-1-carboxylate in 2.5 ml of dichloromethane and 2.5 ml of acetonitrile at 0°C is treated with stirring with 1.1 mmol of lithium tert-butoxide. The reaction mixture is stirred at 0°C for 15 minutes and at room temperature for 10 minutes and then treated at 0°C with 1.2 mmol of 1-chloroethyl chloroformate [50893-53-3]. It is stirred at 0°C for 10 minutes and at room temperature overnight. The reaction mixture is diluted with tert-butyl methyl ether and washed with saturated aqueous sodium hydrogencarbonate solution. The aqueous phase is extracted with tert-butyl methyl ether. The combined organic phases are dried with sodium sulphate and evaporated. The title compound is identified by means of the Rf value from the residue by means of flash chromatography (SiO₂ 60F).

### c) tert-Butyl(2R,4R,5R)-4-[4-(2-methoxyethoxy-methyl)phenyl]-5-[4-(3-methoxypropyl)-3,4-dihydro-2H-benzo[1,4]oxazin-6-ylmethoxy]-2-(2-methyl-2-methylcarbamoylpropyl)piperidine-1-carboxylate

Analogously to method R, starting from 3-{(2R,4R,5R)-4-[4-(2-methoxyethoxymethyl)phenyl]-5-[4-(3-methoxy-propyl)-3,4-dihydro-2H-benzo[1,4]oxazin-6-ylmethoxy]-piperidin-2-yl}-2,2,N-trimethylpropionamide [911704-78-4] the title compound is identified by means of the Rf value.

According to the process described in Example 53 the following compound is prepared in an analogous manner:

### Example

### 55 1-Nitrooxyethyl (3-{(2S,4R,5R)-4-[4-(2-methoxy-ethoxymethyl)phenyl]-5-[4-(3-methoxypropyl)-3,4-di-hydro-2H-benzo[1,4]oxazin-6-ylmethoxy]piperidin-2-yl}-2,2-dimethylpropionylmethylcarbamate

starting from tert-butyl (2S,4R,5R)-4-[4-(2-methoxy-ethoxymethyl)phenyl]-5-[4-(3-methoxypropyl)-3,4-di-hydro-2H-benzo[1,4]oxazin-6-ylmethoxy]-2-(2-methyl-2-methylcarbamoylpropyl)piperidine-1-carboxylate [911704-57-9].

### Example 57

### N-(3-{(2R,4R,5R)-4-[4-(2-Methoxyethoxymethyl)phenyl]-5-[4-(3-methoxypropyl)-3,4-dihydro-2H-benzo[1,4]oxazin-6-ylmethoxylpiperidin-2-yl}-2,2-dimethylpropionyl)-N-methyl-4-nitrooxymethylbenzamide

Analogously to method Q, starting from tert-butyl (2R,4R,5R)-2-{2,2-dimethyl-3-[methyl-(4-nitrooxymethylbenzoyl)amino]-3-oxopropyl}-4-[4-(2-methoxyethoxymethyl)phenyl]-5-[4-(3-methoxypropyl)-3,4-dihydro-2H-benzo[1,4]oxazin-6-ylmethoxy]piperidine-1-carboxylate the title compound is identified by means of the Rf value.

The starting materials are prepared as follows:

### a) tert-Butyl (2R,4R,5R)-2-{2,2-dimethyl-3-[methyl-(4-nitrooxymethylbenzoyl)amino]-3-oxopropyl}-4-[4-(2-methoxyethoxymethyl)phenyl]-5-[4-(3-methoxy-propyl)-3,4-dihydro-2H-benzo[1,4]oxazin-6-yl-methoxylpiperidine-1-carboxylate

A solution of 1 mmol of tert-butyl (2R,4R,5R)-2-[2,2-dimethyl-3-(4-nitrooxymethylbenzoylamino)-3-oxopropyl]-4-[4-(2-methoxyethoxymethyl)phenyl]-5-[4-(3-methoxy-propyl)-3,4-dihydro-2H-benzo[1,4]oxazin-6-ylmethoxy]-piperidine-1-carboxylate in 10 ml of tetrahydrofuran is treated with stirring with 1.3 mmol of sodium hydride (60% dispersion in oil). The reaction mixture is stirred at room temperature for 1 hour and then treated at -20°C with 1.3 mmol of methyl iodide. After 1 hour at -20°C and 3 hours at room temperature the reaction mixture is diluted with tert-butyl methyl ether and washed with saturated aqueous sodium hydrogencarbonate solution. The aqueous phase is extracted with tert-butyl methyl ether. The combined organic phases are dried with sodium sulphate and evaporated. The title compound is identified by means of the Rf value from the residue by means of flash chromatography (SiO₂ 60F).

### b) tert-Butyl (2R,4R,5R)-2-[2,2-dimethyl-3-(4-nitro-oxymethylbenzoylamino)-3-oxopropyl]-4-[4-(2-methoxyethoxymethyl)phenyl]-5-[4-(3-methoxy-propyl)-3,4-dihydro-2H-benzo[1,4]oxazin-6-yl-methoxylpiperidine-1-carboxylate

A solution of 1 mmol of tert-butyl (2R,4R,5R)-2-(2-carbamoyl-2-methylpropyl)-4-[4-(2-methoxyethoxymethyl)-phenyl]-5-[4-(3-methoxypropyl)-3,4-dihydro-2H-benzo-[1,4]oxazin-6-ylmethoxy]piperidine-1-carboxylate in 10 ml of tetrahydrofuran at 0°C is treated with stirring with 2.5 mmol of potassium tert-butoxide. The reaction mixture is stirred at 0°C for 10 minutes and then treated at 0°C with 1 mmol of pentafluorophenyl 4-nitrooxymethylbenzoate [874446-96-5]. The reaction mixture is slowly warmed to room temperature and stirred at room temperature for 3 hours. It is poured into pH 3 buffer solution, brought to pH 1 using 1 M HCl and extracted with dichloromethane (2X). The organic phase is dried with sodium sulphate and evaporated. The title compound is obtained from the residue by means of flash chromatography (SiO₂ F60).

### c) tert-Butyl (2R,4R,5R)-2-(2-carbamoyl-2-methyl-propyl)-4-[4-(2-methoxyethoxymethyl)phenyl]-5-[4-(3-methoxypropyl)-3,4-dihydro-2H-benzo[1,4]oxazin-6-ylmethoxy]piperidine-1-carboxylate

A solution of 1 mmol of tert-butyl (2R,4R,5R)-2-(2-carboxy-2-methylpropyl)-4-[4-(2-methoxyethoxymethyl)-phenyl]-5-[4-(3-methoxypropyl)-3,4-dihydro-2H-benzo[1,4]oxazin-6-ylmethoxy]piperidine-1-carboxylate and 1.1 mmol of triethylamine in 10 ml of dichloromethane is cooled to 0°C and treated with 1 mmol of ethyl chloroformate. It is subsequently stirred for 5 minutes. Subsequently, up to the complete conversion of the anhydride in thin-layer chromatography, ammonia is allowed to bubble through the reaction mixture. It is diluted with dichloromethane and washed with 0.5 N HCl. The combined organic phases are dried with sodium sulphate and evaporated. The title compound is identified by means of the Rf value from the residue by means of flash chromatography (SiO₂ F60).

### d) tert-Butyl (2R,4R,5R)-2-(2-carboxy-2-methyl-propyl)-4-[4-(2-methoxyethoxymethyl)phenyl]-5-[4-(3-methoxypropyl)-3,4-dihydro-2H-benzo[1,4]oxazin-6-ylmethoxylpiperidine-1-carboxylate

A solution of 1 mmol of tert-butyl (2R,4R,5R)-2-(2-methoxycarbonyl-2-methylpropyl)-4-[4-(2-methoxyethoxy-methyl)phenyl]-5-[4-(3-methoxypropyl)-3,4-dihydro-2H-benzo[1,4]oxazin-6-ylmethoxy]piperidine-1-carboxylate in 2 ml of tetrahydrofuran and 5 ml of ethanol is treated with 6 ml of 20% NaOH. The reaction mixture is stirred at 65°C for 4 hours. The organic solvents are evaporated and the resulting solution is brought to pH 2 with 6M HCl. The mixture is extracted with ethyl acetate (3X). The combined organic phases are dried with sodium sulphate and evaporated. The title compound is identified by means of the Rf value from the residue by means of flash chromatography (SiO₂ F60).

### e) tert-Butyl (2R,4R,5R)-2-(2-methoxycarbonyl-2-methylpropyl)-4-[4-(2-methoxyethoxymethyl)phenyl-5-(4-(3-methoxypropyl)-3,4-dihydro-2H-benzo[1,4]-oxazin-6-ylmethoxylpiperidine-1-carboxylate

Analogously to method R, starting from methyl 3-{(2R,4R,5R)-4-[4-(2-methoxyethoxymethyl)phenyl]-5-[4-(3-methoxypropyl)-3,4-dihydro-2H-benzo[1,4]oxazin-6-ylmethoxy]piperidin-2-yl}-2,2-dimethylpropionate the title compound is identified by means of the Rf value.

### f) Methyl 3-{(2R,4R,5R)-4-[4-(2-methoxyethoxymethyl)-phenyl]-5-[4-(3-methoxypropyl)-3,4-dihydro-2H-benzo[1,4]oxazin-6-ylmethoxylpiperidin-2-yl}-2,2-dimethylpropionate

Analogously to method N, starting from methyl 3-[(2R,4R,5R)-4-[4-(2-methoxyethoxymethyl)phenyl]-5-[4-(3-methoxypropyl)-3,4-dihydro-2H-benzo[1,4]oxazin-6-ylmethoxy]-1-(toluene-4-sulphonyl)piperidin-2-yl]-2,2-dimethylpropionate [911707-59-0] the title compound is identified by means of the Rf value.

According to the process described in Example 57, the following compounds are prepared in an analogous manner:

### Examples

### 58 N-(3-{(2R,4R,5R)-4-[4-(2-Methoxyethoxymethyl)-phenyl]-5-[4-(3-methoxypropyl)-3,4-dihydro-2H-benzo[1,4]oxazin-6-ylmethoxylpiperidin-2-yl}-2,2-dimethylpropionyl)-N-methyl-4-nitrooxybutyramide

starting from methyl 3-[(2R,4R,5R)-4-[4-(2-methoxy-ethoxymethyl)phenyl]-5-[4-(3-methoxypropyl)-3,4-di-hydro-2H-benzo[1,4]oxazin-6-ylmethoxy]-1-(toluene-4-sulphonyl)piperidin-2-yl]-2,2-dimethylpropionate [911707-59-0] and pentafluorophenyl 6-nitrooxybutanoate [838878-70-9].

### 59 N-(3-{(2S,4R,5R)-4-[4-(2-Methoxyethoxymethyl)-phenyl]-5-[4-(3-methoxypropyl)-3,4-dihydro-2H-benzo[1,4]oxazin-6-ylmethoxylpiperidin-2-yl}-2,2-dimethylpropionyl)-N-methyl-4-nitrooxybutyramide

starting from tert-butyl (2S,4R,5R)-2-(2-carbamoyl-2-methylpropyl)-4-[4-(2-methoxyethoxymethyl)phenyl]-5-[4-(3-methoxypropyl)-3,4-dihydro-2H-benzo[1,4]oxazin-6-ylmethoxy]piperidine-1-carboxylate and pentafluorophenyl 6-nitrooxybutanoate [838878-70-9].

The starting materials are prepared as follows:

### a) tert-Butyl (25,4R,5R)-2-(2-carbamoyl-2-methyl-propyl)-4-[4-(2-methoxyethoxymethyl)phenyl]-5-[4-(3-methoxypropyl)-3,4-dihydro-2H-benzo[1,4]oxazin-6-ylmethoxylpiperidine-1-carboxylate

Analogously to the processes described in Examples 57c, 57d, 57e and 57f, starting from methyl 3-[(2S,4R,5R)-4-[4-(2-methoxyethoxymethyl)phenyl]-5-[4-(3-methoxypropyl)-3,4-dihydro-2H-benzo[1,4]oxazin-6-ylmethoxy]-1-(toluene-4-sulphonyl)piperidin-2-yl]-2,2-dimethyl-propionate the title compound is identified by means of the Rf value.

### b) Methyl 3-[(2S,4R,5R)-4-[4-(2-methoxyethoxymethyl)-phenyl]-5-[4-(3-methoxypropyl)-3,4-dihydro-2H-benzo[1,4]oxazin-6-ylmethoxyl-1-(toluene-4-sulphonyl)piperidin-2-yl]-2,2-dimethylpropionate

About 15 ml of a solution of diazomethane (about 0.2M in diethyl ether) are added dropwise at 0°C to a solution of 1 mmol of 3-[(2S,4R,5R)-4-[4-(2-methoxy-ethoxymethyl)phenyl]-5-[4-(3-methoxypropyl)-3,4-dihydro-2H-benzo[1,4]oxazin-6-ylmethoxy]-1-(toluene-4-sulphonyl)piperidin-2-yl]-2,2-dimethylpropionic acid [911707-26-1] in 10 ml of methanol. After stirring at room temperature for 3.5 hours, the reaction mixture is treated with solid magnesium sulphate in order to destroy the excess of diazomethane. The reaction mixture is filtered and evaporated. The title compound is identified by means of the Rf value from the residue by means of flash chromatography (SiO₂ F60).

### 62 N-(3-{(25,4R,5R)-4-[4-(2-Methoxyethoxymethyl)-phenyl]-5-[4-(3-methoxypropyl)-3,4-dihydro-2H-benzo[1,4]oxazin-6-ylmethoxy]piperidin-2-yl}-2,2-dimethylipropionyl)-N-methyl-4-nitrooxymethyl-benzamide

starting from tert-butyl (2S,4R,5R)-2-(2-carbamoyl-2-methylpropyl)-4-[4-(2-methoxyethoxymethyl)phenyl]-5-[4-(3-methoxypropyl)-3,4-dihydro-2H-benzo[1,4]oxazin-6-ylmethoxy]piperidine-1-carboxylate (Example 59a) and pentafluorophenyl 4-nitrooxymethylbenzoate [874446-96-5].

## Claims

1. Compound of the general formula (I) 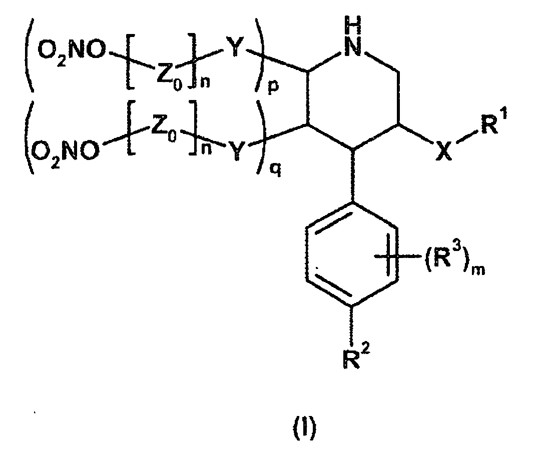 wherein
R¹ is aryl or heterocyclyl, in particular benzimidazolyl, benzo[1,3]dioxolyl, benzofuranyl, benzooxazolyl, benzothiazolyl, benzo[b]thienyl, quinazolinyl, quinolyl, quinoxalinyl, 2H-chromenyl, 3,4-dihydro-2H-benzo[1,4]oxazinyl, dihydro-3H-benzo-[1,4]oxazinyl, dihydro-2H-benzo[1,4]thiazinyl, 1 a,7b-dihydro-1 H-cyclopropa[c]chromenyl, 2,3-dihydro-1H-indolyl, dihydro-1H-pyrido[2,3-b][1,4]oxazinyl, imidazo[1,2-a]pyridyl, imidazo[1,5-a]pyridyl, indazolyl, indolyl, isobenzofuranyl, isoquinolyl, [1,5]naphthyridyl, phenyl, phthalazinyl, pyridyl, pyrimidinyl, 1 H-pyrrolo[2,3-b]pyridyl, 1 H-pyrrolo[2,3-c]pyridyl, 1 H-pyrrolo[3,2-b]pyridyl, tetrahydro-quinolyl, tetrahydroquinoxalinyl, 1,1a,2,7b-tetrahydro-cyclopropa[c]chromenyl, tetrahydroimidazo[1,2-a]-pyridyl, tetra-hydroimidazo[1,5-a]pyridyl, tetrahydro-isoquinolyl, [1,2,3]triazolo[1,5-a]pyridyl or
[1,2,4]triazolo [4,3-a]pyridyl, which are substituted by 1-4 acyl-C₁₋₈-alkoxy-C₁₋₈-alkoxy, acyl-C₁₋₈-alkoxy-C₁₋₈-alkyl, (N-acyl)-C₁₋₈-alkoxy-C₁₋₈-alkylamino, C₁₋₈-alkanoyl, C₁₋₈-alkoxy, C₁₋₈-alkoxy-C₁₋₈-alkanoyl, C₁₋₈-alkoxy-C₁₋₈-alkoxy, C₁₋₈-alkoxy-C₁₋₈-alkoxy-C₁₋₈-alkyl, C₁₋₈-alkoxy-C₁₋₈-alkyl , (N-C₁₋₈-alkoxy)-C₁₋₈-alkylamino-carbonyl-C₁₋₈-alkoxy, (N-C₁₋₈-alkoxy)-C₁₋₈-alkylamino-carbonyl-C₁₋₈-alkyl, C₁₋₈-alkoxy-C₁₋₈-alkylcarbamoyl, C₁₋₈-alkoxy-C₁₋₈-alkylcarbonyl, C₁₋₈-alkoxy-C₁₋₈-alkyl-carbonylamino, 1-C₁₋₈-alkoxy-C₁₋₈-alkylheterocyclyl, C₁₋₈-alkoxyaminocarbonyl-C₁₋₈-alkoxy, C₁₋₈-alkoxyamino-carbonyl-C₁₋₈-alkyl, C-₁₋₈-alkoxycarbonyl, C₁₋₈-alkoxy-carbonyl-C₁₋₈-alkoxy, C₁₋₈-alkoxycarbonyl-C₁-₈-alkyl, C₁₋₈-alkoxycarbonylamino-C-₁-₈-alkoxy, C₁₋₈-alkoxycarbonyl-amino-C₁₋₈-alkyl, C₁₋₈-alkyl, (N-C₁₋₈-alkyl)-C₁₋₈-alkoxy-C₁₋₈-alkylcarbamoyl, (N-C₁₋₈-alkyl)-C₁₋₈-alkoxy-C₁₋₈-alkyl-carbonylamino, (N-C₁₋₈-alkyl)-C₁₋₈-alkoxycarbonylamino, (N-C₁₋₈-alkyl)-C₁₋₈-alkylcarbonylamino-C₁₋₈-alkoxy, (N-C₁₋₈-alkyl-C₁₋₈-alkylcarbonylamino-C₁₋₈-alkyl, (N-C₁₋₈-alkyl)-C₁₋₈-alkylsulphonylamino-C₁₋₈-alkoxy, (N-C₁₋₈-alkyl)-C₁₋₈-alkylsulphonylamino-C₁₋₈-alkyl, C₁₋₈-alkylamidinyl, C₁₋₈-alkylamino-C₁₋₈-alkoxy, di-C₁₋₈-alkylamino-C₁₋₈-alkoxy, C₁₋₈-alkylamino-C₁₋₈-alkyl, di-C₁₋₈-alkylamino-C₁₋₈-alkyl, C₁₋₈-alkylaminocarbonyl-C₁₋₈-alkoxy, di-C₁₋₈-alkylamino-carbonyl-C₁₋₈-alkoxy, C₁₋₈-alkylaminocarbonyl-C₁₋₈-alkoxy-C₁₋₈-alkyl, C₁₋₈-alkylaminocarbonyl-C₁₋₈-alkyl, di-C₁₋₈-alkylaminocarbonyl-C₁₋₈-alkyl , C₁₋₈-alkylamino-carbonylamino-C₁₋₈-alkoxy, C₁₋₈-alkylaminocarbonylamino-C₁₋₈-alkyl, C₁₋₈-alkylcarbonylamino, C₁₋₈-alkylcarbonyl-amino-C₁₋₈-alkoxy, C₁₋₈-alkylcarbonylamino-C₁₋₈-alkyl, C₁₋₈-alkylcarbonyloxy-C₁₋₈-alkoxy, C₁₋₈-alkylcarbonyloxy-C₁₋₈-alkyl, C₁₋₈-alkylsulphonyl, C₁₋₈-alkylsulphonyl-C₁₋₈-alkoxy, C₁₋₈-alkylsulphonyl-C₁₋₈-alkyl, C₁₋₈-alkyl-sulphonylamino-C₁₋₈-alkoxy, C₁₋₈-alkylsulphonylamino-C₁₋₈-alkyl, optionally N-mono- or N,N-di-C₁₋₈-alkylated amino, aryl-C₀₋₈-alkoxy, aryl-C₀₋₈-alkyl, optionally N-mono- or N,N-di-C₁₋₈-alkylated carbamoyi-C₀₋₈-alkoxy, optionally N-mono- or N,N-di-C₁₋₈-alkylated carbamoyl-C₀₋₈-alkyl, carboxy-C₁₋₈-alkoxy, carboxy-C₁₋₈-alkoxy-C₁₋₈-alkyl, carboxy-C₁₋₈-alkyl, cyano, cyano-C₁₋₈-alkoxy, cyano-C₁₋₈-alkyl, C₃₋₈-cycloalkyl-C₁₋₈-alkoxy, C₃₋₈-cycloalkyl-C₁₋₈-alkyl, C₃₋₈-cycloalkylcarbonylamino-C₁₋₈-alkoxy, C₃₋₈-cycloalkylcarbonylamino-C₁₋₈-alkyl, O,N-dimethylhydroxyamino-C₁₋₈-alkyl, halogen, halogeno-C₁₋₈-alkoxy, halogeno-C₁₋₈-alkyl, heterocyclyl-C₀₋₈-alkoxy, heterocyclyl-C₀₋₈-alkyl, heterocyclylcarbonyl, hydroxy-C₁₋₈-alkoxy-C₁₋₈-alkoxy, hydroxy-C₁₋₈-alkoxy-C₁₋₈-alkyl, hydroxy-C₁₋₈-alkyl, O-methyloximyl-C₁₋₈-alkyl, oxide or oxo;
R² is C₂₋₈-alkenyloxy-C₁₋₈-alkoxy, C₂₋₈-alkenyloxy-C₁₋₈-alkyl, C₁₋₈-alkoxy, C₁₋₈-alkoxy-C₁₋₈-alkoxy, optionally halogen-substituted C₁₋₈-alkoxy-C₁₋₈-alkoxy-C₁₋₈-alkyl, C₁₋₈-alkoxy-C₁₋₈-alkyl, C₁₋₈-alkoxy-C₁₋₈-alkylamino-C₁₋₈-alkoxy, C₁₋₈-alkoxy-C₁₋₈-alkylamino-C₁₋₈-alkyl , C₁₋₈-alkoxy-C₁₋₈-alkylsulphanyl-C₁₋₈-alkoxy, C₁₋₈-alkoxy-C₁₋₈-alkylsulphanyl-C₁₋₈-alkyl, C₁₋₈-alkoxy-C₀₋₈-alkyl-C₃₋₈-cycloalkyl-C₀₋₈-alkoxy-C₁₋₈-alkyl , C₁₋₈-alkyl, C₁₋₈-alkyl-sulphanyl-C₁₋₈-alkoxy, C₁₋₈-alkylsulphanyl-C₁₋₈-alkoxy-C₁₋₈-alkoxy, C₁₋₈-alkylsulphanyl-C₁₋₈-alkoxy-C₁₋₈-alkyl, C₁₋₈-alkylsulphanyl-C₁₋₈-alkyl, C₁₋₈-alkylsulphonyl-C₁₋₈-alkoxy-C₁₋₈-alkoxy, C₁₋₈-alkylsulphonyl-C₁₋₈-alkoxy-C₁₋₈-alkyl, aryl-C₀₋₈-alkoxy-C₁₋₈-alkoxy, aryl-C₀₋₈-alkoxy-C₁₋₈-alkyl, aryloxy, C₃₋₈-cycloalkyl-C₀₋₈-alkoxy-C₁₋₈-alkoxy, C₃-₈-cydoalkyl-C₀₋₈-alkoxy-C₁₋₈-alkoxy-C₁₋₈-alkyl, C₃-₈-cycloalkyl-C₀₋₈-alkoxy-C₁₋₈-alkyl, heterocydyl-C₀₋₈-alkoxy-C₁₋₈-alkoxy, heterocyclyl-C₀₋₈-alkoxy-C₁₋₈-alkyl or heterocyclyloxy;
R³ is halogen,
R⁴ is acyl, C₂₋₈-alkenyl, C₁₋₈-alkyl, aryl-C₁₋₈-alkyl or hydrogen;
X is -Alk-, -O-Alk-, -Alk-O-, -O-Alk-O-, -S-Alk-, -Alk-S-, -Alk-NR⁴-, -NR⁴-Alk-, -C(O)-NR⁴-, -Alk-C(O)-NR⁴-, -O-Alk-C(O)-NR⁴-, -C(O)-NR⁴-Alk-, -Alk-C(O)-NR⁴-Alk-, -NR⁴-C(O)-, -Alk-NR⁴-C(O)-, -NR⁴-C(O)-Alk-, -Alk-NR⁴-C(O)-Alk-, -O-Alk-C(O)-NR⁴-, -O-Alk-NR⁴-C(O)-, -S(O)₂-NR⁴-, -Alk-S(O)₂-NR⁴-, -S(O)₂-NR⁴-Alk-, -Alk-S(O)₂-NR⁴-Alk-, -NR⁴-S(O)₂-, -Alk-NR⁴-S(O)₂-, -NR⁴-S(O)₂-Alk- or -Alk-NR⁴-S(O)₂-Alk-, where Alk designates C₁₋₈-alkylene which can optionally be substituted by halogen;
Y is a) unsubstituted or heterocyclyl- and/or halogen- and/or hydroxy- and/or C₁₋₈-alkoxy-substituted
- Alk-,
- Alk-O-Alk-,
- Alk-NR⁴-Alk-,
- Alk-C(O)-Alk-,
- Alk-C(O)-NR⁴-Alk-,
- Alk-C(O)-Alk-NR⁴-Alk-,
- Alk-NR⁴-C(O)-Alk- or
- Alk-NR⁴-Alk-C(O)-Alk-,
where Alk designates linear or branched C₁₋₈-alkylene; or
b) if p = 0, is additionally
i) a bond
ii) unsubstituted or heterocyclyl- and/or halogen- and/or hydroxy- and/or C₁₋₈-alkoxy-substituted
-Alk-O-,
-Alk-NR⁴-,
-Alk-C(O)- or
-Alk-NR⁴-C(O)-Alk-O-,
where Alk designates linear or branched C₁₋₈-alkylene;
Z₀ is equal to -U-Z₁-
wherein Z₁ is -C(O)-O-, -O-C(O)O-, -C(O)-NR⁷-C(O) or -O-C(O)-NR⁷-C(O)-, wherein R⁷is as defined below;
U is a bivalent radical having the following meaning:
a)
- C₁₋₈-alkylene, preferably C₁₋₈-alkylene, being optionally substituted with one or more of the substituents selected from the group consisting of: halogen, hydroxy, -ONO₂ or Tₒ, wherein Tₒ is -OC(O)-(C₁₋₈-alkyl)-ONO₂ or -O-(C₁₋₈-alkyl)-ONO₂;
- C₃₋₈-cycloalkylene, the ring being optionally substituted with side chains T, wherein T is C₁₋₈-alkyl;
b) 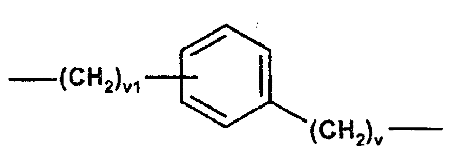 wherein v is an integer from 0 to 20, and v1 is an integer from 1 to 20;
c) 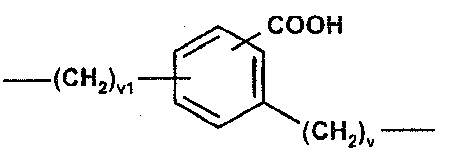 wherein v is an integer from 0 to 20, and v1 is an integer from 1 to 20;
d) 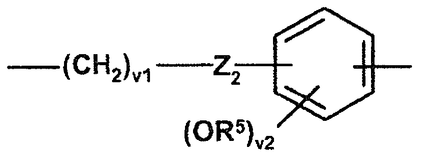 wherein:
v1 is as defined above and v2 is an integer from 0 to 2;
Z₂ = -O-C(O)- or -C(O)-O- and R⁵ is H or CH₃;
e) 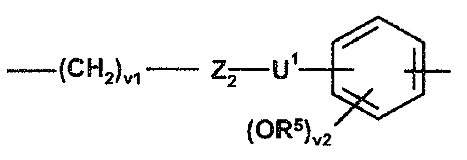 wherein:
v1, v2, R⁵ and Z₂ are as defined above;
U¹ is -CH₂-CH₂- or -CH=CH-(CH₂)ᵥ₂-;
f) 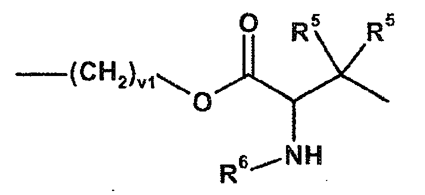 wherein:
v1 and R⁵ are as defined above, R⁶ is H or -C(O)CH₃;
g) 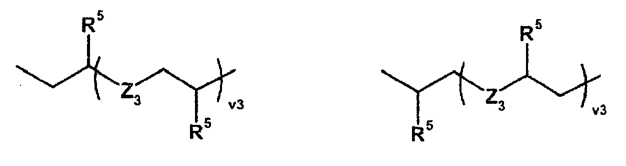 wherein Z₃ is -O-, or -S-, v3 is an integer from 1 to 6, preferably from 1 to 4, R⁵ is as defined above; or
h) 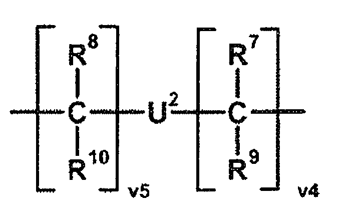 wherein:
v4 is an integer from 0 to 10;
v5 is an integer from 1 to 10;
R⁷, R⁸, R⁹, R¹⁰ are the same or different, and are H or C₁₋₄ alkyl;
U² is a heterocyclic saturated, unsaturated or aromatic 5 or 6 membered ring, containing one or more heteroatoms selected from nitrogen, oxygen and sulphur, and is preferably selected from 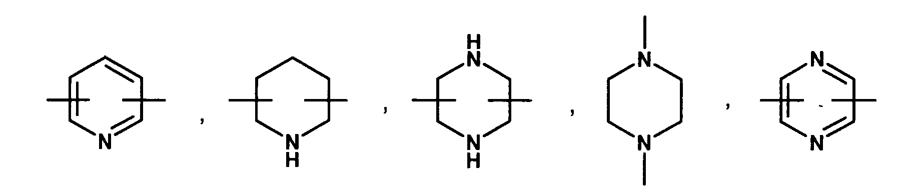 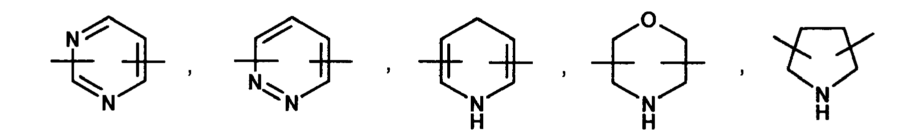 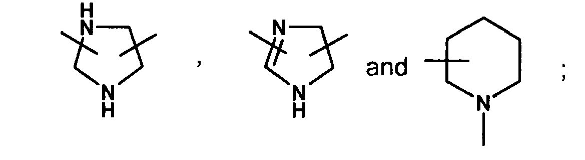 m is 0, 1 or 2;
n is 0 or 1;
p is 0 or 1;
q is 0 or 1;
where if p is = 0, q is = 1 and if p is = 1, q is = 0,
and their salts, preferably their pharmaceutically usable salts.

2. Compound according to Claim 1, **characterized in that** it has the general formula 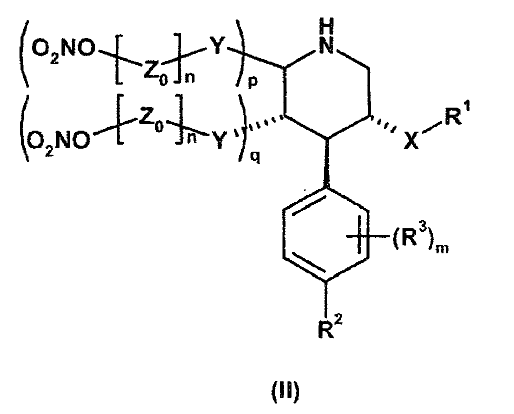 wherein R¹, R², R³, X, Y and Z₀, and m, n, p and q have the meaning indicated for the compounds of the formula (I) in Claim 1.

3. Compound according to Claim 1 or 2, **characterized in that**
R¹ is benzimidazolyl, 2H-chromenyl, 3,4-dihydro-2H-benzo[1,4]oxazinyl, 1 a,7b-dihydro-1 H-cyclopropa[c]-chromenyl, indazolyl, indolyl, 2,3-dihydro-1 H-indolyl, phenyl or 1,1a,2,7b-tetrahydrocyclopropa[c]chromenyl, which are mono- or polysubstituted by C₁₋₈-alkoxy, C₁₋₈-alkoxy-C₁₋₈-alkoxy, C₁₋₈-alkoxy-C₁₋₈-alkoxy-C₁₋₈-alkyl, C-₁₋₈-alkoxy-C₁₋₈-alkyl, ₁₋₈-alkoxycarbonylamino-C₁₋₈-alkoxy, C₁₋₈-alkoxy-carbonylamino-C₁₋₈-alkyl, C₁₋₈-alkyl, (N-C₁₋₈-alkyl)-C₁₋₈-alkylcarbonylamino-C₁₋₈-alkoxy, (N-C₁₋₈-alkyl)-C₁₋₈-alkylcarbonylamino-C₁₋₈-alkyl, (N-C₁₋₈-alkyl)-C₁₋₈-alkylsulphonylamino-C₁₋₈-alkoxy, (N-C₁₋₈-alkyl)-C₁₋₈-alkylsulphonylamino-C₁₋₈-alkyl, C₁₋₈-alkylcarbonylamino-C₁₋₈-alkoxy, C₁₋₈-alkyl-carbonylamino-C₁₋₈-alkyl, C₁₋₈-alkylsulphonyl-C₁₋₈-alkoxy, C₁₋₈-alkyl-sulphonyl-C₁₋₈-alkyl, C₁₋₈-alkylsulphonylamino-C₁₋₈-alkoxy, C₁₋₈-alkylsulphonylamino-C₁₋₈-alkyl, C₃₋₈-cydoalkylcarbonylamino-C₁₋₈-alklxy, C₃₋₈-cycloalkylcarbonylamino-C₁₋₈-alkyl, halogen, halo-C₁₋₈-alkoxy, halo-C₁₋₈-alkyl or oxide.

4. Compound according to one of Claims 1-3, **characterized in that** m is = 0.

5. Use of a compound according to one of Claims 1-4 for the production of a medicament.

6. Use of a compound according to one of Claims 1-4 for the production of a medicament for the prevention, for the delay of the development or for the treatment of disease states in humans, which are caused by or associated with the activity the renin system and nitrogen monoxide deficiency.

7. Use of a compound according to one of Claims 1 to 4 for the production of a medicament for the prevention, for the delay of the development or for the treatment of high blood pressure, left-ventricular hypertrophy, heart failure, ischaemic heart disease, stable angina pectoris, unstable angina pectoris, acute coronary syndrome, vasospastic or Prinzmetal's angina, stroke, peripheral, myocardial or cerebral ischaemic disorders, cardiac infarction, ischaemic reperfusion damage, Raynaud's syndrome, thrombosis, cardiac arrhythmias, dyslipidaemia, atherosclerosis, prevention of stenoses after angioplasties, peripheral arterial occlusive diseases, erectile disorders, diabetes type 1 and type 2, diabetic complications, nephropathy, retinopathy, neuropathy, pulmonary arterial hypertension, digestive disorders, disorders of the digestive tract, portal hypertension and cirrhosis of the liver.

8. Pharmaceutical preparation comprising a compound according to Claims 1 to 4 and a common additive.

9. Pharmaceutical combination in the form of a preparation or of a kit of individual components consisting of a) a compound according to Claims 1 to 4 and b) at least one pharmaceutical form whose active agent has a blood pressure-lowering, inotropic, anti-diabetic, lipid-lowering or anti-oxidative effect.

## Patentansprüche

1. Verbindung der allgemeinen Formel (I) 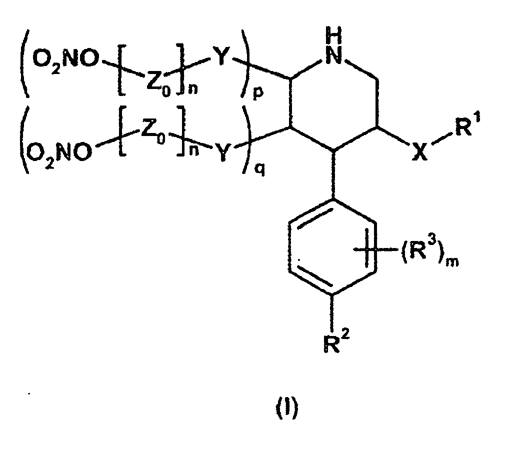 worin
R¹ für Aryl oder Heterocyclyl, insbesondere Benzimidazolyl, Benzo[1,3]dioxolyl, Benzofuranyl, Benzooxazolyl, Benzothiazolyl, Benzo[b]thienyl, Chinazolinyl, Chinolyl, Chinoxalinyl, 2H-Chromenyl, 3,4-Dihydro-2H-benzo[1,4]oxazinyl, Dihydro-3H-benzo-[1,4]oxazinyl, Dihydro-2H-benzo[1,4]thiazinyl, 1a,7b-Dihydro-1H-cyclopropa[c]chromenyl, 2,3-Dihydro-1H-indolyl, Dihydro-1H-pyrido[2,3-b][1,4]oxazinyl, Imidazo[1,2-a]pyridyl, Imidazo[1,5-a]pyridyl, Indazolyl, Indolyl, Isobenzofuranyl, Isochinolyl, [1,5]-Naphthyridyl, Phenyl, Phthalazinyl, Pyridyl, Pyrimidinyl, 1 H-Pyrrolo[2,3-b]pyridyl, 1H-Pyrrolo[2,3-c]pyridyl, 1H-Pyrrolo[3,2-b]pyridyl, Tetrahydro-chinolyl, Tetrahydrochinoxalinyl, 1,1a,2,7b-Tetrahydrocyclopropa[c]chromenyl, Tetrahydroimidazo[1,2-a]-pyridyl, Tetra-hydroimidazol[1,5-a]pyridyl, Tetrahydroisochinolyl, [1,2,3]Triazolo[1,5-a]pyridyl oder [1,2,4]-Triazolo-[4,3-a]pyridyl steht, die durch 1-4 Acyl-C₁₋₈-alkoxy-C₁₋₈-alkoxy, Acyl-C₁₋₈-alkoxy-C₁₋₈-alkyl, (N-Acyl)-C₁₋₈-alkoxy-C₁₋₈-alkylamino, C₁₋₈-Alkanoyl, C₁₋₈-Alkoxy,
C₁₋₈-Alkoxy-C₁₋₈-alkanoyl, C₁₋₈-Alkoxy-C₁₋₈-alkoxy, C₁₋₈-Alkoxy-C₁₋₈-alkoxy-C₁₋₈-alkyl, C₁₋₈-Alkoxy-C₁₋₈-alkyl, (N-C₁₋₈-Alkoxy)-C₁₋₈-alkylamino-carbonyl-C₁₋₈-alkoxy, (N-C₁₋₈-Alkoxy)-C₁₋₈-alkylamino-carbonyl-C₁₋₈-alkyl, C₁₋₈-Alkoxy-C₁₋₈-alkylcarbamoyl, C₁₋₈-Alkoxy-C₁₋₈-alkylcarbonyl, C₁₋₈-Alkoxy-C₁₋₈-alkyl-carbonylamino, 1-C₁₋₈-Alkoxy-C₁₋₈-alkylheterocyclyl, C₁₋₈-Alkoxyaminocarbonyl-C₁₋₈-alkoxy, C₁₋₈-Alkoxyamino-carbonyl-C₁₋₈-alkyl, C₁₋₈-Alkoxycarbonyl, C₁₋₈-Alkoxy-carbonyl-C₁₋₈-alkoxy, C₁₋₈-Alkoxycarbonyl-C₁₋₈-alkyl, C₁₋₈-Alkoxycarbonylamino- C₁₋₈-alkoxy, C₁₋₈-Alkoxycarbonyl-amino-C₁₋₈-alkyl, C₁₋₈-Alkyl, (N-C₁₋₈-Alkyl)-C₁₋₈-alkoxy-C₁₋₈-alkylcarbamoyl, (N-C₁₋₈-Alkyl)-C₁₋₈-alkoxy-C₁₋₈-alkyl-carbonylamino, (N-C₁₋₈-Alkyl)-C₁₋₈-alkoxycarbonylamino, (N-C₁₋₈-Alkyl)-C₁₋₈-alkyl-carbonylamino-C₁₋ₐ-alkoxy, (N-C₁₋ₐ-Alkyl-C₁₋₈-alkoxycarbonylamino-C₁₋₈-alkyl, (N-C₁₋₈-Alkyl)-C₁₋₈-alkylsulfonylamino-C₁₋₈-alkoxy, (N-C₁₋₈-Alkyl)-C₁₋₈-alkylsulfonylamino-C₁₋₈-alkyl, C₁₋₈-Alkylamidinyl, C₁₋₈-Alkylamino-C₁₋₈-alkoxy, Di-C₁₋₈-alkylamino-C₁₋₈-alkoxy, C₁₋₈-Alkylamino-C₁₋₈-alkyl, Di-C₁₋₈-alkylamino-C₁₋₈-alkyl, C₁₋₈-Alkylamino-carbonyl-C₁₋₈-alkoxy, Di-C₁₋₈-Alkylamino-carbonyl-C₁₋₈-alkoxy, C₁₋₈-Alkylaminocarbonyl-C₁₋₈-alkoxy-C₁₋₈-alkyl, C₁₋₈-Alkylaminocarbonyl-C₁₋₈-alkyl Di-C₁₋₈-alkylaminocarbonyl-C₁₋₈-alkyl, C₁₋₈-Alkylamino-carbonylamino-C₁₋₈-alkoxy, C₁₋₈-Alkylaminocarbonylamino-C₁₋₈-alkyl, C₁₋₈-Alkylaminocarbonylamino, C₁₋₈-Alkylcarbonyl-amino-C₁₋₈-alkoxy, C₁₋₈-Alkyl-carbonylamino-C₁₋₈-alkyl, C₁₋₈-Alkylcarbonyloxy-C₁₋₈-alkoxy, C₁₋₈-Alkylcarbonyloxy-C₁₋₈-alkyl, C₁₋₈-Alkylsulfonyl, C₁₋₈-Alkylsulfonyl-C₁₋₈-alkoxy, C₁₋₈-Alkylsulfonyl-C₁₋₈-alkyl, C₁₋₈-Alkylsulfonylamino-C₁₋₈-alkoxy, C₁₋₈-Alkylsulfonylamino-C₁₋₈-alkyl, optional N-mono oder N,N-di-C₁₋₈-alkyliertes Amino, Aryl-C₀₋₈-alkoxy, Aryl-C₀₋₈-alkyl, optional N-mono oder N,N-di-C₁₋₈-alkyliertes Carbamoyl-C₀₋₈-alkoxy, optional N-mono oder N,N-di-C₁₋₈-alkyliertes Carbamoyl-C₀₋₈-alkyl, Carboxy-C₁₋₈-alkoxy, Carboxy-C₁₋₈-alkoxy-C₁₋₈-alkyl, Carboxy-C₁₋₈-alkyl, Cyano, Cyano-C₁₋₈-alkoxy, Cyano-C₁₋₈-alkyl, C₃₋₈-Cycloalkyl-C₁-₈-alkoxy, C₃₋₈-Cycloalkyl-C₁₋₈-alkyl, C₃₋₈-Cycloalkylcarbonylamino-C₁₋₈-alkoxy, C₃₋₈-Cycloalkylcarbonylamino-C₁₋₈-alkyl, O,N-Dimethylhydroxyamino-C₁₋₈-alkyl, Halogen, Halogen-C₁₋₈-alkoxy, Halogen-C₁₋₈-alkyl, Heterocyclyl-C₀₋₈-alkoxy, Heterocyclyl-C₀₋₈-alkyl, Heterocyclylcarbonyl, Hydroxy-C₁₋₈-alkoxy-C₁₋₈-alkoxy, Hydroxy-C₁₋₈-alkoxy-C₁₋₈-alkyl, Hydroxy-C₁₋₈-alkyl, O-Methyloximyl-C₁₋₈-alkyl, Oxid oder Oxo substituiert sind;
R² für C₂₋₈-Alkenyloxy-C₁₋₈-alkoxy, C₂₋₈-Alkenyloxy-C₁₋₈-alkyl, C₁₋₈-Alkoxy, C₁₋₈-Alkoxy-C₁₋₈-alkoxy, optional halogensubstituiertes C₁₋₈-Alkoxy-C₁₋₈-alkoxy-C₁₋₈-alkyl, C₁₋₈-Alkoxy-C₁₋₈-alkyl, C₁₋₈-Alkoxy-C₁₋₈-alkylamino-C₁₋₈-alkoxy, C₁₋₈-Alkoxy-C₁₋₈-alkylamino-C₁₋₈-alkyl, C₁₋₈-Alkoxy-C₁₋₈-alkylsulfanyl-C₁₋₈-alkoxy, C₁₋₈-Alkoxy-C₁₋₈-alkylsulfanyl-C₁₋₈-alkyl, C₁₋₈-Alkoxy-C₁₋₈-alkyl-C₁₋₈-cycloalkyl-C₁₋₈-alkoxy-C₁₋₈-alkyl, C₁₋₈-Alkyl, C₁₋₈-Alkyl-sulfanyl-C₁₋₈-alkoxy, C₁₋₈-Alkylsulfanyl-C₁₋₈-alkoxy-C₁₋ₐ-alkoxy, C₁₋₈-Alkylsulfanyl-C₁₋₈-alkoxy-C₁₋₈-alkyl, C₁₋₈-Alkylsulfanyl-C₁₋₈-alkyl, C₁₋₈-Alkylsulfonyl-C₁₋₈-alkoxy-C₁₋₈-alkoxy, C₁₋₈-Alkylsulfonyl-C₁₋₈-al koxy-C₁₋₈-alkyl, Aryl-C₁₋₈-alkoxy-C₁₋₈-alkoxy, Aryl-C₀₋₈-alkoxy-C₁₋₈-alkyl, Aryloxy, C₃₋₈-Cycloalkyl-C₀₋₈ alkoxy-C₁₋₈-alkoxy, C₃₋₈-Cycloalkyl-C₀₋₈-alkoxy-C₁₋₈-alkoxy-C₁₋₈-alkyl, C₃₋₈-Cycloalkyl-C₀₋₈-alkoxy-Cᵢz-alkyl, Heterocydyl-C₀₋₈-alkoxy-C₁₋₈-alkoxy, Heterocyclyl-C₀₋₈-alkoxy-C₁₋₈alkyl oder Heterocyclyloxy steht;
R³ für Halogen steht;
R⁴ für Acyl, C₂₋₈-Alkenyl, C₁₋₈-Alkyl, Aryl-C₁₋₈-alkyl oder Wasserstoff steht;
X für -Alk-, -O-Alk-, -Alk-O-, -O-Alk-O-, -S-Alk-, -Alk-S-, -Alk-NR⁴-, -NR⁴-Alk-, -C(O)-NR⁴-, -Alk-C(O)-NR⁴-, -O-Alk-C(O)-NR⁴-, -C(O)-NR⁴-Alk-, -Alk-C(O)-NR⁴-Alk-, -NR⁴-C(O)-, -Alk-NR⁴-C(O)-, -NR⁴-C(O)-Alk-, -Alk-NR⁴-C(O)-Alk-, -O-Alk-C(O)-NR⁴-, -O-Alk-NR⁴-C(O)-, -S(O)₂-NR⁴-, -Alk-S(O)₂-NR⁴-, -S(O)₂-NR⁴-Alk-, -Alk-S(O)₂-NR⁴-Alk-, NR⁴-S(O)₂-, -Alk-NR⁴-S(O)₂-, -NR⁴-S(O)₂-Alk- oder -Alk-NR⁴-S(O)₂-Alk- steht, wobei Alk C₁₋₈-Alkylen bezeichnet, das optional durch Halogen substituiert sein kann;
Y a) für nicht substituiertes oder heterocyclyl- und/oder halogen- und/oder hydroxy- und/oder C₁₋₈-alkoxysubstituiertes
- Alk-,
- Alk-O-Alk-,
- Alk-NR ⁴-Alk-,
- Alk-C(O)-Alk-,
- Alk-C(O)-NR⁴-Alk-,
- Alk-C(O)-Alk-NR⁴-Alk-,
- Alk-NR⁴-C(O)-Alk- oder
- Alk-NR⁴-Alk-C(O)-Alk- steht,
worin Alk lineares oder verzweigtes C₁₋₈-Alkylen bedeutet; oder
b) wenn p = 0 bedeutet, zusätzlich für
i) eine Bindung,
ii) nicht substituiertes oder heterocyclyl- und/oder halogen- und/oder hydroxy- und/oder C₁₋₈-alkoxysubstituiertes
- Alk-O-,
- Alk-NR⁴-,
- Alk-C(O)- oder
- Alk-NR⁴-C(O)-Alk-O-,
worin Alk lineares oder verzweigtes C₁₋₈-Alkylen bezeichnet, steht;
Z₀-U-Z₁- entspricht,
worin Z₁ für -C(O)-O-, -O-C(O)-O-, -C(O)-NR⁷-C(O)- oder -O-C(O)-NR⁷-C(O)- steht, worin R⁷ die unten angegebene Bedeutung besitzt,
U für einen zweiwertigen Rest mit der folgenden Bedeutung steht:
a)
- C₁-₈-Alkylen, vorzugsweise C₁₋₈-Alkylen, das optional mit einem oder mehreren der Substituenten substituiert ist, die aus der Gruppe ausgewählt sind, die aus Halogen, Hydroxy, - ONO₂ oder -T₀ besteht, wobei T₀ für -OC(O)-(C₁₋₈-Alkyl)-ONO₂ oder -O-(C₁₋₈-Alkyl)-ONO₂ steht;
- C₃-₈-Cycloalkylen, wobei der Ring optional mit Seitenketten T substituiert ist, wobei T für C₁₋₈-Alkyl steht;
b) 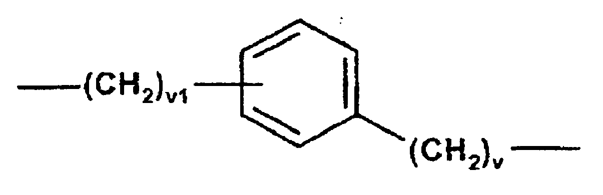 worin v für eine ganze Zahl von 0 bis 20 steht, und v1 für eine ganze Zahl von 1 bis 20 steht;
c) 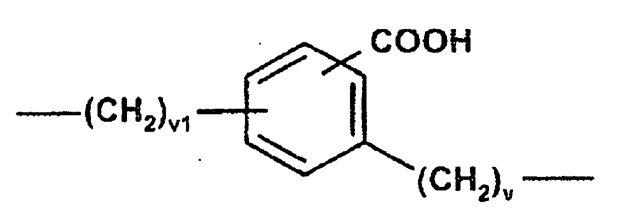 worin v für eine ganze Zahl von 0 bis 20 steht, und v1 für eine ganze Zahl von 1 bis 20 steht;
d) 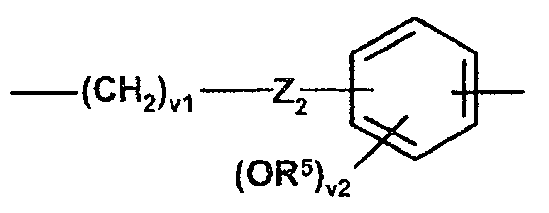 worin: v1 die oben angegebene Bedeutung besitzt und v2 für eine ganze Zahl von 0 bis 2 steht;
Z₂= -O-C(O)- oder -C(O)-O- steht und R⁵ für H oder CH₃ steht;
e) 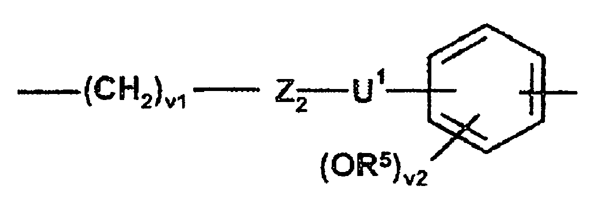 worin:
v1, v2, R⁵ und Z₂ die oben angegebenen Bedeutungen besitzen; U¹ für -CH₂-CH₂- oder -CH=CH-(CH₂)ᵥ₂- steht;
f) 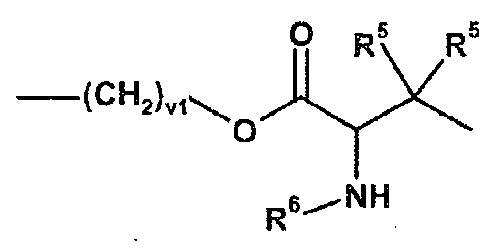 worin:
v1 und R⁵ die oben angegebenen Bedeutungen besitzen, R⁶ für H oder -C(O)CH₃ steht;
g) 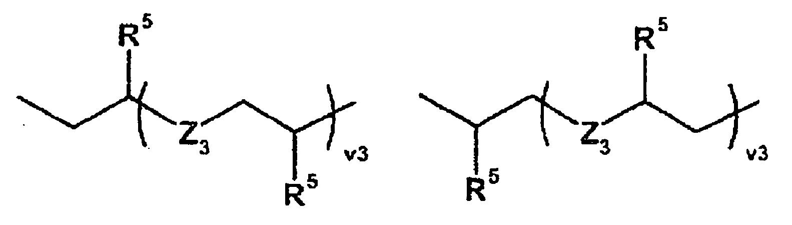 worin Z₃ für -O- oder -S- steht, v3 für eine ganze Zahl von 1 bis 6, vorzugsweise 1 bis 4 steht, und
R⁵ die oben angegebene Bedeutung besitzt; oder
h) 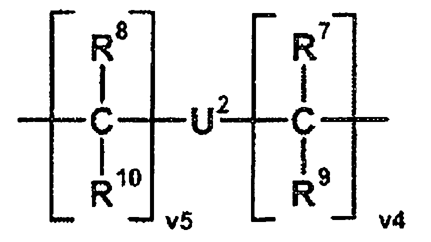 worin:
v4 für eine ganze Zahl von 0 bis 10 steht;
v5 für eine ganze Zahl von 1 bis 10 steht;
R⁷, R⁸, R⁹, R¹⁰ gleich oder verschieden sind und für H oder C₁₋₄-Alkyl stehen;
U² für einen heterozyklischen gesättigten, nicht gesättigten oder aromatischen 5- bis 6-gliedrigem Ring mit einem oder mehreren Heteroatomen, die aus Stickstoff, Sauerstoff und Schwefel ausgewählt sind, steht und vorzugsweise aus den folgenden ausgewählt ist: 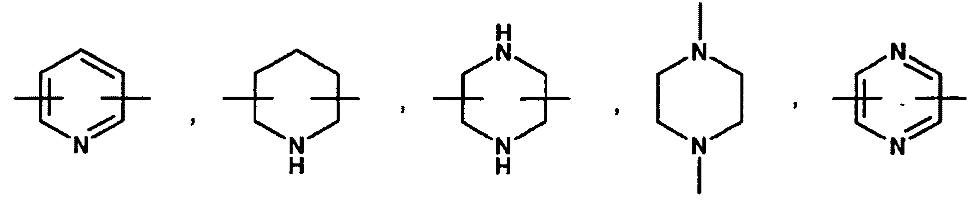 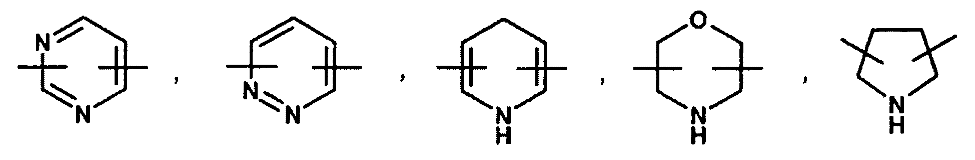 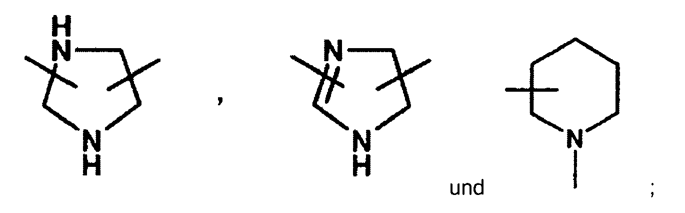 m für 0, 1 oder 2 steht;
n für 0 oder 1 steht;
p für 0 oder 1 steht;
q für 0 oder 1 steht;
wobei, wenn p für 0 steht, q für 1 steht und, wenn p für 1 steht, q für 0 steht,
und ihre Salze, vorzugsweise ihre pharmazeutisch geeigneten Salze.

2. Verbindung nach Anspruch 1, **dadurch gekennzeichnet, dass** sie die folgende allgemeine Formel besitzt: 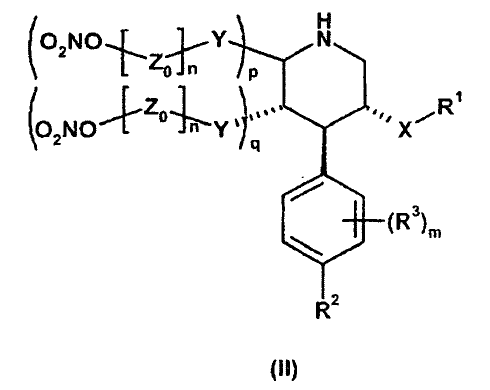 worin R¹, R², R³, X, Y und Z₀ sowie m, n, p und q die für die Verbindungen der Formel (1) in Anspruch 1 angegebenen Bedeutungen besitzen.

3. Verbindung nach Anspruch 1 oder 2, **dadurch gekennzeichnet, dass**
R¹ für Benzimidazolyl, 2H-Chromenyl, 3,4-Dihydro-2H-benzo[1,4]oxazinyl, 1 a,7b-Dihydro-1 H-cyclopropa[c]-chromenyl, Indazolyl, Indolyl, 2,3-Dihydro-1H-indolyl, Phenyl oder 1,1a,2,7b-Tetrahydrocyclopropa[c]chromenyl steht, die durch C₁₋₈-Alkoxy, C₁₋₈-Alkoxy-C₁₋₈-alkoxy, C₁₋₈-Alkoxy-C₁₋₈-alkoxy-C₁₋₈-alkyl, C₁₋₈-Alkoxy-C₁₋₈-alkyl, C₁₋₈-Alkoxycarbonylamino-C₁₋₈-alkoxy, C₁₋₈-Alkoxy-carbonylamino-C₁₋₈-alkyl, C₁₋₈-Alkyl, (N-C₁₋₈-Alkyl)-C₁₋₈-alkylcarbonylamino-C₁₋₈-Alkoxy, (N-C₁₋₈-Alkyl)-C₁₋₈-alkylcarbonylamino-C₁₋₈-alkyl, (N-C₁₋₈-Alkyl)-C₁₋₈-alkylsulfonylamino-C₁₋₈-alkoxy, (N-C₁₋₈-Alkyl)-C₁₋₈-alkylsulfonylamino-C₁₋₈-alkyl, C₁₋₈-Alkylcarbonylamino-C₁₋₈-alkoxy, C₁₋₈-Alkyl-carbonylamino-C₁₋₈-alkyl, C₁₋₈-Alkylsulfonyl-C₁₋₈-alkoxy, C₁₋₈-Alkyl-sulfonyl-C₁₋₈-alkyl, C₁₋₈-Alkylsulfonylamino-C₁₋₈-alkoxy, C₁₋₈-Alkyl-sulfonylamino-C₁₋₈-alkyl, C₃₋₈-Cycloalkylcarbonylamino-C₁₋₈-alkoxy, C₃₋₈-Cycloalkylcarbonylamino-C₁₋₈-alkyl, Halogen, Halogen-C₁₋₈-alkoxy, Halogen-C₁₋₈-alkyl oder Oxid mono- oder polysubstituiert sind.

4. Verbindung nach einem der Ansprüche 1-3, **dadurch gekennzeichnet, dass** m für 0 steht.

5. Verwendung einer Verbindung nach einem der Ansprüche 1-4 zur Herstellung eines Medikaments.

6. Verwendung einer Verbindung nach einem der Ansprüche 1-4 zur Herstellung eines Medikaments zur Verhinderung, zur Verzögerung der Entwicklung oder zur Behandlung von Erkrankungszuständen bei Menschen, die durch die Aktivität den Reninsystems und eine Stickstoff-monoxiddefizienz verursacht sind oder damit in Verbindung stehen.

7. Verwendung einer Verbindung nach einem der Ansprüche 1-4 zur Herstellung eines Medikaments zur Verhinderung, zur Verzögerung der Entwicklung oder zur Behandlung von hohem Blutdruck, links-ventrikulärer Hypertrophie, Herzinsuffizienz, ischämischer Herzerkrankung, stabiler Angina Pectoris, nicht stabiler Angina Pectoris, akutem Koronarsyndrom, vasospastischer oder Prinzmetall-Angina, Schlaganfall, periphären, myokardialen oder zerebralen ischämischen Störungen, Herzinfarkt, ischämischem Reperfusionsschäden, Raynaud-Syndrom, Thrombose, Herzarrhytmien, Dyslipidämie, Atherosklerose, Verhinderung von Stenosen nach Angioplastien, pheriphären arteriellen Verschlusskrankheiten, Erektionsstörungen, Diabetes Typ I und Typ II, diabetischen Komplikationen, Nephropathie, Retinopathie, Neuropathie, pulmonalem arteriellem Hochdruck, Verdauungsstörungen, Störungen des Verdauungstrakts, portalem Hochdruck und Leberzirrhose.

8. Pharmazeutische Zubereitung, die eine Verbindung nach den Ansprüchen 1 bis 4 und ein übliches Additiv umfasst.

9. Pharmazeutische Kombination in Form einer Zubereitung oder eines Kits individueller Komponenten aus a) einer Verbindung nach den Ansprüchen 1 bis 4 und b) mindestens einer pharmazeutischen Form, deren wirksamer Bestandteil eine blutdrucksenkende, inotrope, antidiabetische, lipidsenkende oder antioxidative Wirkung aufweist.

## Revendications

1. Composé de formule générale (I) : 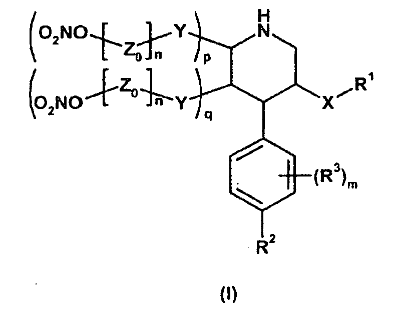 dans laquelle :
R¹ est un groupe aryle ou hétérocyclyle, en particulier benzimidazolyle, benzo[1,3]dioxolyle, benzofuranyle, benzooxazolyle, benzothiazolyle, benzo[b]thiényle, quinazolinyle, quinolyle, quinoxalinyle, 2H-chroményle, 3,4-dihydro-2H-benzo-[1,4]oxazinyle, dihydro-3H-benzo-[1,4]oxazinyle, dihydro-2H-benzo-[1,4]thiazinyle, 1a,7b-dihydro-1H-cyclopropa[c]-chroményle, 2,3-dihydro-1H-indolyle, dihydro-1H-pyrido-[2,3-b][1,4]oxazinyle, imidazo[1,2-a]pyridyle, imidazo[1,5-a]pyridyle, indazolyle, indolyle, isobenzofuranyle, isoquinolyle, [1,5]naphtyridyle, phényle, phtalazinyle, pyridyle, pyrimidinyle, 1H-pyrrolo-[2,3-b]pyridyle, 1H-pyrrolo-[2,3-c]pyridyle, 1H-pyrrolo-[3,2-b]pyridyle, tétrahydroquinolyle, tétrahydroquinoxalinyle, 1,1 a,2,7b-tétrahydrocyclopropa[c]chroményle, tétrahydroimidazo[ 1,2-a]-pyridyle, tétrahydroimidazo[1,5-a]pyridyle, tétrahydro-isoquinolyle, [1,2,3]triazolo[1,5-a]pyridyle ou [1,2,4]triazolo [4,3-a]pyridyle, lesquels sont substitués par 1 à 4 groupes acyl-alcoxy en C₁₋₈-alcoxy en C₁₋₈, acyl-alcoxy en C₁₋₈-alkyle en C₁₋₈, (N-acyl)-alcoxy en C₁₋₈-alkylamino en C₁₋₈, alcanoyle en C₁₋₈, alcoxy en C₁₋₈, alcoxy en C₁₋₈-alcanoyle en C₁₋₈, alcoxy en C₁₋₈-alcoxy en C₁₋₈, alcoxy en C₁₋₈-alcoxy en C₁₋₈- alkyle en C₁₋₈, alcoxy en C₁₋₈-alkyle en C₁₋₈, (N-alcoxy en C₁₋₈)-alkylamino en C₁₋₈-carbonyl-alcoxy en C₁₋₈, (N-alcoxy en C₁₋₈)-alkylamino en C₁₋₈-carbonyle-alkyle en C₁₋₈, alcoxy en C₁₋₈-alkylcarbamoyle en C₁₋₈, alcoxy en C₁₋₈-alkylcarbonyle en C₁₋₈, alcoxy en C₁₋₈-alkyl(en C₁₋₈)-carbonylamino, 1-alcoxy en C₁₋₈-alkylhétérocyclyle en C₁₋₈, alcoxyaminocarbonyl(en C₁₋₈)-alcoxy en C₁₋₈, alcoxyamino en C₁₋₈-carbonyl-alkyle en C₁₋₈, alcoxycarbonyle en C₁₋₈, alcoxy en C₁₋₈-carbonyl-alcoxy en C₁₋₈, alcoxycarbonyl(en C₁₋₈)-alkyle en C₁₋₈, alcoxycarbonylamino en C₁₋₈-alcoxy en C₁₋₈, alcoxycarbonyl(en C₁₋₈)-amino-alkyle en C₁₋₈, alkyle en C₁₋₈, (N-alkyl(en C₁₋₈))-alcoxy en C₁₋₈-alkylcarbamoyle en C₁₋₈, (N-alkyl(en C₁₋₈))-alcoxy en C₁₋₈-alkyl(en C₁₋₈)-carbonylamino, (N-alkyl(en C₁₋₈))-alcoxycarbonylamino en C₁₋₈, (N-alkyl(en C₁₋₈))-alkyl(en C₁₋₈)-carbonylamino-alcoxy en C₁₋₈, (N-alkyl(en C₁₋₈)-alkylcarbonylamino en C₁₋₈-alkyle en C₁₋₈, (N-alkyl(en C₁₋₈)) alkylsulfonylamino en C₁₋₈-alcoxy en C₁₋₈, (N-alkyl(en C₁₋₈))-alkylsulfonylamino en C₁₋₈-alkyle en C₁₋₈, alkylamidinyle en C₁₋₈, alkylamino en C₁₋₈-alcoxy en C₁₋₈, di-alkylamino en C₁₋₈-alcoxy en C₁₋₈, alkylamino en C₁₋₈-alkyle en C₁₋₈, di-alkylamino en C₁₋₈-alkyle en C₁₋₈, alkylamino en C₁₋₈-carbonyl-alcoxy en C₁₋₈, di-alkylamino en C₁₋₈-carbonyl-alcoxy en C₁₋₈, alkylaminocarbonyl(en C₁₋₈)-alcoxy en C₁₋₈-alkyle en C₁₋₈, alkylaminocarbonyl(en C₁₋₈)-alkyle en C₁₋₈, di-alkylaminocarbonyl(en C₁₋₈)-alkyle en C₁₋₈, alkylamino en C₁₋₈-carbonylamino-alcoxy en C₁₋₈, alkylaminocarbonylamino en C₁₋₈-alkyle en C₁₋₈, alkylcarbonylamino en C₁₋₈, alkylcarbonyl(en C₁₋₈)-amino-alcoxy en C₁₋₈, alkyl(en C₁₋₈)-carbonylamino-alkyle en C₁₋₈, alkylcarbonyloxy en C₁₋₈-alcoxy en C₁₋₈, alkylcarbonyloxy en C₁₋₈-alkyle en C₁₋₈, alkylsulfonyle en C₁₋₈, alkylsulfonyl(en C₁₋₈)-alcoxy en C₁₋₈, alkylsulfonyle en C₁₋₈-alkyle en C₁₋₈, alkyl(en C₁₋₈)-sulfonylamino-alcoxy en C₁₋₈, alkylsulfonylamino en C₁₋₈-alkyle en C₁₋₈, facultativement amino N-mono- ou N,N-di-alkyle en C₁₋₈, aryl-alcoxy en C₀₋₈, aryl-alkyle en C₀₋₈, facultativement carbamoyl-alkyle en C₀₋₈ N-mono- ou N,N-di-alkyle en C₁₋₈, facultativement carbamoyl-alkyle en C₀₋₈ N-mono- ou N,N-di-alkyle en C₁₋₈, carboxy-alcoxy en C₁₋₈, carboxy-alcoxy en C₁₋₈-alkyle en C₁₋₈, carboxy-alkyle en C₁₋₈, cyano, cyano-alcoxy en C₁₋₈, cyano-alkyle en C₁₋₈, cycloalkyl(en C₃₋₈)-alcoxy en C₁₋₈, cycloalkyl(en C₃₋₈)-alkyle en C₁₋₈, cycloalkylcarbonylamino en C₃₋₈-alcoxy en C₁₋₈, cycloalkylcarbonylamino en C₃₋₈-alkyle en C₁₋₈, O,N-diméthylhydroxyamino-alkyle en C₁₋₈, halogène, halogéno-alcoxy en C₁₋₈, halogéno-alkyle en C₁₋₈, hétérocyclyl-alcoxy en C₀₋₈, hétérocyclyl-alkyle en C₀₋₈, hétérocyclylcarbonyle, hydroxy-alcoxy en C₁₋₈-alcoxy en C₁₋₈, hydroxy-alcoxy en C₁₋₈-alkyle en C₁₋₈, hydroxy-alkyle en C₁₋₈, O-méthyloximyl-alkyle en C₁₋₈, oxyde ou oxo ;
R² est un groupe alcényloxy en C₂₋₈-alcoxy en C₁₋₈, alcényloxy en C₂₋₈-alkyle en C₁₋₈, alcoxy en C₁₋₈, alcoxy en C₁₋₈-alcoxy en C₁₋₈, facultativement alcoxy en C₁₋₈-alcoxy en C₁₋₈-alkyle en C₁₋₈ substitué par un halogène, alcoxy en C₁₋₈-alkyle en C₁₋₈, alcoxy en C₁₋₈-alkylamino en C₁₋₈-alcoxy en C₁₋₈, alcoxy en C₁₋₈-alkylamino en C₁₋₈-alkyle en C₁₋₈, alcoxy en C₁₋₈-alkylsulfanyl(en C₁₋₈)-alcoxy en C₁₋₈, alcoxy en C₁₋₈-alkylsulfanyle en C₁₋₈-alkyle en C₁₋₈, alcoxy en C₁₋₈-alkyl(en C₀₋₈)-cycloalkyl(en C₃₋₈)-alcoxy en C₀₋₈-alkyle en C₁₋₈, alkyle en C₁₋₈, alkyl(en C₁₋₈)-sulfanyl-alcoxy en C₁₋₈, alkylsulfanyl(en C₁₋₈)-alcoxy en C₁₋₈-alcoxy en C₁₋₈, alkylsulfanyl(en C₁₋₈)-alcoxy en C₁₋₈-alkyle en C₁₋₈, alkylsulfanyl(en C₁₋₈)-alkyle en C₁₋₈, alkylsulfonyl(en C₁₋₈)-alcoxy en C₁₋₈-alcoxy en C₁₋₈, alkylsulfonyl(en C₁₋₈)-alcoxy en C₁₋₈-alkyle en C₁₋₈, aryl-alcoxy en C₀₋₈-alcoxy en C₁₋₈, aryl-alcoxy en C₀₋₈-alkyle en C₁₋₈, aryloxy, cycloalkyl(en C₃₋₈)-alcoxy en C₀₋₈-alcoxy en C₁₋₈, cycloalkyl(en C₃₋₈)-alcoxy en C₀₋₈-alcoxy en C₁₋₈-alkyle en C₁₋₈, cycloalkyl(en C₃₋₈)-alcoxy en C₀₋₈-alkyle en C₁₋₈, hétérocyclyle-alcoxy en C₀₋₈-alcoxy en C₁₋₈, hétérocyclyl-alcoxy en C₀₋₈-alkyle en C₁₋₈ ou hétérocyclyloxy ;
R³ est un halogène,
R⁴ est un groupe acyle, alcényle en C₂₋₈, alkyle en C₁₋₈, aryl-alkyle en C₁₋₈ ou hydrogène ;
X est -Alk-, -O-Alk-, -Alk-O-, -O-Alk-O-, -S-Alk-, -Alk-S-, -Alk-NR⁴-, -NR⁴-Alk-, -C(O)-NR⁴-, -Alk-C(O)-NR⁴-, -O-Alk-C(O)-NR⁴, -C(O)-NR⁴-Alk-, -Alk-C(O)-NR⁴-Alk-, -NR⁴-C(O)-, -Alk-NR⁴-C(O)-, -NR⁴-C(O)-Alk-, -Alk-NR⁴-C(O)-Alk-, -O-Alk-C(O)-NR⁴-, -O-Alk-NR⁴-C(O)-, -S(O)₂-NR⁴-, -Alk-S(O)₂-NR⁴-, -S(O)₂-NR⁴-Alk-, -Alk-S(O)₂-NR⁴-Alk-, -NR⁴-S(O)₂, -Alk-NR⁴-S(O)₂-, -NR⁴-S(O)₂-Alk- ou -Alk-NR⁴-S(O)₂-Alk-, où Alk désigne un alkylène en C₁₋₈ pouvant facultativement être substitué par un halogène ;
Y est a)
- Alk-,
- Alk-O-Alk,
- Alk-NR⁴-Alk,
- Alk-C(O)-Alk,
- Alk-C(O)-NR⁴-Alk,
- Alk-C(O)-Alk-NR⁴-Alk,
- Alk-NR⁴-C(O)-Alk ou
- Alk-NR⁴-Alk-C(O)-Alk-
non substitué ou substitué par un hétérocyclyle et/ou un halogène et/ou un hydroxy et/ou un alcoxy en C₁₋₈
où Alk désigne un alkylène en C₁₋₈ linéaire ou ramifié, ou
b) si p = 0, est en outre
i) une liaison
ii) -Alk-O-,
-Alk-NR⁴-,
-Alk-C(O)- ou
-Alk-NR⁴-C(O)-Alk-O-,
non substitué ou substitué par un hétérocyclyle et/ou un halogène et/ou un hydroxy et/ou un alcoxy en C₁₋₈,
où Alk désigne un alkylène en C₁₋₈ linéaire ou ramifié ;
Z₀ est égal à -U-Z₁-
où Z₁ est -C(O)-O-, -O-C(O)O-, -C(O)-NR⁷-C(O) ou -O-C(O)-NR⁷-C(O)-,
où R⁷ est tel que défini ci-après ;
U est un radical bivalent ayant la signification suivante :
a)
- alkylène en C₁₋₈, de préférence alkylène en C₁₋₈ facultativement substitué par un ou plusieurs substituants choisis dans le groupe constitué par un groupe : halogène, hydroxy, -ONO₂ ou T₀, où T₀ est -OC(O)-(alkyl(en C₁₋₈))-ONO₂ ou -O-(alkyl(en C₁₋₈))-ONO₂ ;
- cycloalkylène en C₃₋₈, le cycle étant facultativement substitué par des chaînes latérales T, où T est un alkyle en C₁₋₈ ;
b) 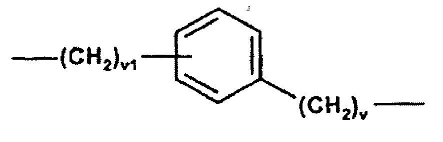 où v est un nombre entier de 0 à 20, et v1 un nombre entier de 1 à 20 ;
c) 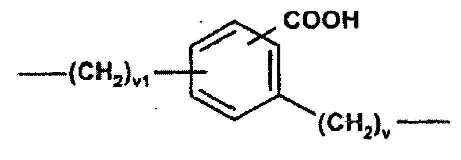 où v est un nombre entier de 0 à 20, et v1 un nombre entier de 1 à 20 ;
d) 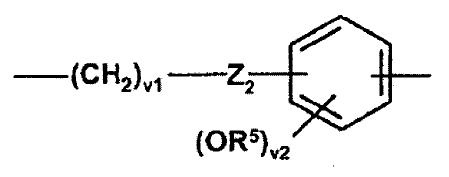 où :
v1 est tel que défini ci-avant et v2 est un nombre entier de 0 à 2 ; Z₂ = -O-C(O)- ou -C(O)-O- et R⁵ est H ou CH₃ ;
e) 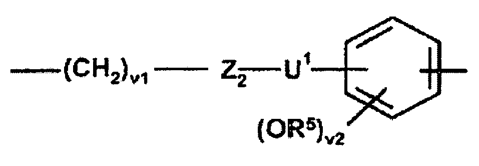 où :
v1, v2, R⁵ et Z₂ sont tels que définis ci-avant ;
U¹ est -CH₂-CH₂- ou -CH=CH-(CH₂)ᵥ₂- ;
f) 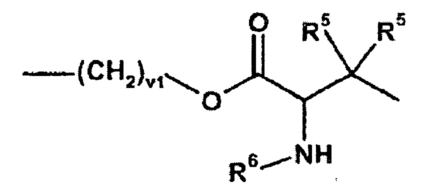 où :
v1 et R⁵ sont tels que définis ci-avant, R⁶ est H ou -C(O)CH₃ ;
g) 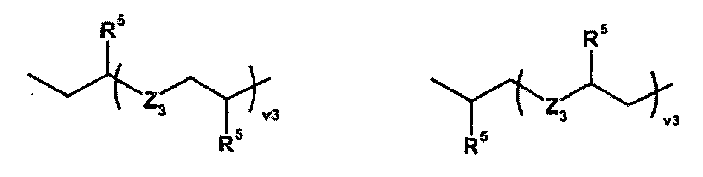 où Z₃ est -O-, ou -S-, v3 est un nombre entier de 1 à 6, de préférence de 1 à 4, R⁵ est tel que défini ci-avant ; ou
h) 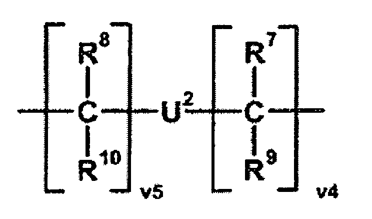 où :
v4 est un nombre entier de 0 à 10 ;
v5 est un nombre entier de 1 à 10 ;
R⁷, R⁸, R⁹, R¹⁰ sont identiques ou différents, et sont H ou un alkyle en C₁₋₄ ;
U² est un cycle hétérocyclique à 5 ou 6 chaînons saturé, insaturé ou aromatique, contenant un ou plusieurs hétéroatomes choisi parmi l'azote, l'oxygène et le souffre, et est de préférence choisi parmi 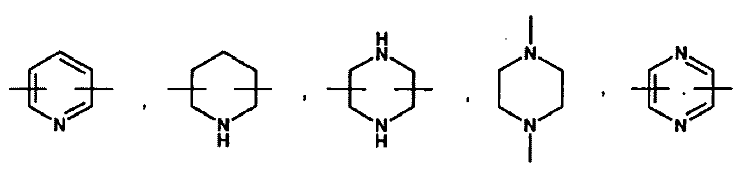 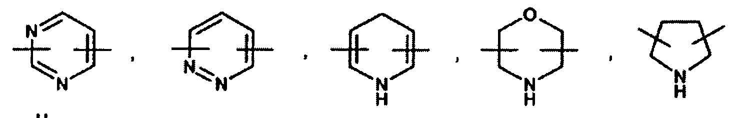 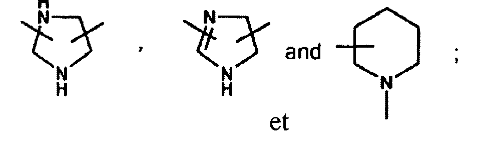 m est égal à 0, 1 ou 2 ;
n est égal à 0 ou 1 ;
p est égal à 0 ou 1 ;
q est égal à 0 ou 1 ;
où, si p est égal à 0, q est égal à 1, et si p est égal à 1, q est égal à 0, et leurs sels, de préférence leurs sels utilisables en pharmacie.

2. Composé selon la revendication 1, **caractérisé en ce qu'**il possède la formule générale 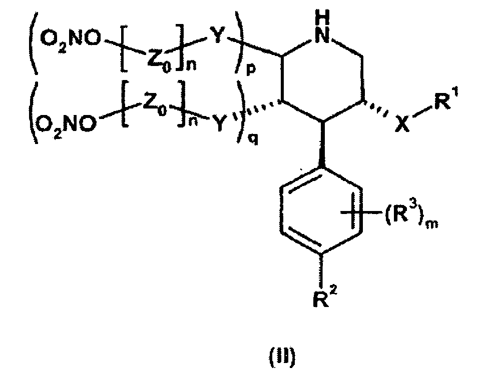 dans laquelle R¹, R², R³, X, Y et Z₀, et m, n, p et q ont la signification indiquée pour les composés de formule (I) dans la revendication 1.

3. Composé selon la revendication 1 ou 2, **caractérisé en ce que** R¹ est un groupe benzimidazolyle, 2H-chroményle, 3,4-dihydro-2H-benzo[1,4]oxazinyle, 1a,7b-dihydro-1H-cyclopropa[c]-chroményle, indazolyle, indolyle, 2,3-dihydro-1H-indolyle, phényle ou 1,1a,2,7b-tétrahydrocyclopropa[c]chroményle, lesquels sont mono- ou polysubstitués par un groupe alcoxy en C₁₋₈, alcoxy en C₁₋₈-alcoxy en C₁₋₈, alcoxy en C₁₋₈-alcoxy en C₁₋₈-alkyle en C₁₋₈, alcoxy en C₁₋₈-alkyle en C₁₋₈, alcoxycarbonylamino en C₁₋₈-alcoxy en C₁₋₈, alcoxy en C₁₋₈-carbonylamino-alkyle en C₁₋₈, alkyle en C₁₋₈, (N-alkyl(en C₁₋₈))-alkylcarbonylamino en C₁₋₈-alcoxy en C₁₋₈, (N-alkyl(en C₁₋₈))- alkylcarbonylamino en C₁₋₈- alkyle en C₁₋₈, (N-alkyl(en C₁₋₈))-alkylsulfonylamino en C₁₋₈-alcoxy en C₁₋₈, (N-alkyl(en C₁₋₈))-alkylsulfonylamino en C₁₋₈-alkyle en C₁₋₈, alkylcarbonylamino en C₁₋₈-alcoxy en C₁₋₈, alkyl(n C₁₋₈)-carbonylamino-alkyle en C₁₋₈, alkylsulfonyl(en C₁₋₈)-alcoxy en C₁₋₈, alkyl(en C₁₋₈)-sulfonyl-alkyle en C₁₋₈, alkylsulfonylamino en C₁₋₈-alcoxy en C₁₋₈, alkyl(C₁₋₈)-sulfonylamino-alkyle en C₁₋₈, cycloalkylcarbonylamino en C₃₋₈-alcoxy en C₁₋₈, cycloalkyl(en C₃₋₈)-carbonylamino-alkyle en C₁₋₈, halogène, halo-alcoxy en C₁₋₈, halo-alkyle en C₁₋₈ ou oxyde.

4. Composé selon l'une des revendications 1 à 3, **caractérisé en ce que** m est égal à 0.

5. Utilisation d'un composé selon l'une des revendications 1 à 4 pour la production d'un médicament.

6. Utilisation d'un composé selon l'une des revendications 1 à 4 pour la production d'un médicament destiné à la prévention, au retardement de l'évolution ou au traitement d'états maladifs chez l'être humain, causés par ou associés à l'activité du système de la rénine et à une carence en monoxyde d'azote.

7. Utilisation d'un composé selon l'une des revendications 1 à 4 pour la production d'un médicament destiné à la prévention, au retardement de l'évolution ou au traitement de l'hypertension, de l'hypertrophie du ventricule gauche, de l'insuffisance cardiaque, de la cardiopathie ischémique, de l'angor stable, de l'angor instable, du syndrome coronarien aigu, de l'angor vasospastique ou de Prinzmetal, de l'accident vasculaire cérébral, des troubles ischémiques périphériques, myocardiques ou cérébraux, de la crise cardiaque, des lésions de reperfusion ischémique, du syndrome de Raynaud, de la thrombose, de l'arythmie cardiaque, de la dyslipidémie, de l'athérosclérose, la prévention de sténoses après une angioplastie, des maladies occlusives des artères périphériques, des troubles de l'érection, du diabète de type 1 et de type 2, des complications liées au diabète, de la néphropathie, de la rétinopathie, de la neuropathie, de l'hypertension artérielle pulmonaire, des troubles digestifs, des troubles du tube digestif, de l'hypertension portale et de la cirrhose du foie.

8. Préparation pharmaceutique comprenant un composé selon les revendications 1 à 4 et un additif courant.

9. Combinaison pharmaceutique sous la forme d'une préparation ou d'un kit de composants individuels constitué de a) un composé selon les revendication 1 à 4 et b) au moins une forme pharmaceutique dont le principe actif a un effet d'abaissement de la tension artérielle, inotrope, antidiabétique, hypolipidémiant ou anti-oxydant.
